# EUROPEAN PATENT APPLICATION

(11) **EP 4 356 894 A1**
(43) Date of publication of application: **24.04.2024**
(21) Application number: 22201850.9
(22) Date of filing: 17.10.2022
(51) Int. Cl.: A61K 8/02, A61K 8/31, A61K 8/33, A61K 8/34, A61K 8/35, A61K 8/37, A61K 8/44, A61K 8/46, A61K 8/49, A61K 8/60, A61K 8/92, A61Q 5/02, A61Q 13/00, A61Q 19/10

(54) **SUSTAINABLE PRODUCT WITH MALODORS**

(71) Applicant: Beiersdorf AG, 22529 Hamburg (DE)
(72) Inventor: KRÖPKE, Petra, 22869 Schenefeld (DE); LATASTE, Noelie, Cedar Knols, NJ 07932 (US); PETERSEN, Miriam, 22297 Hamburg (DE); PRZYBILL, Adrian, 22765 Hamburg (DE); DUFLOT, Sabine, 20255 Hamburg (DE); D'INCECCO, Giulia, 20257 Hamburg (DE)

(57) **Abstract**

Cosmetic product comprising
a) a container comprising at least 80% by weight post-consumer recycled plastic, based on the total weight of the container, whereby the container has at least one opening,
b) a cosmetic cleansing composition situated in the container characterized in that the composition comprises
- a fragrance and
- at least one surfactant.

The fragrance can have a floral family, coconut, orange, jasmine, coconut, sandalwood, vanilla or amber scent. The plastic may comprise polyethylene or polypropylene.

## Description

The present invention belongs to the cosmetic field and relates to a cosmetic product including a cosmetic cleansing formulation

A beautiful and attractive appearance is a desire for many people. One typical sign of such an appearance is a healthy and smooth looking skin. In order to take care on the skin, it is for many people a daily routine to apply cosmetic products such as cleansing formulations, in particular body wash or shampoo products.

Products such as body washes or hair shampoos are conveniently sold in plastic container having a volume suitable for a filling of approximately 200 to 600 ml. The plastic container is formed using synthetic polymers. Most of the materials used to produce polymers for plastic container applications, such as polyethylene, polyethylene terephthalate, and polypropylene, are derived from monomers (e.g., ethylene, propylene, terephthalic acid, ethylene glycol), which are obtained from non-renewable, fossil-based resources, such as petroleum, natural gas, and coal. Thus, the price and availability of the petroleum, natural gas, and coal feedstock ultimately have a significant impact on the price of polymers used for plastic container materials. As the worldwide price of petroleum, natural gas, and/or coal escalates, so does the price of plastic container materials. Furthermore, many consumers display an aversion to purchasing products that are derived from petrochemicals. In some instances, consumers are hesitant to purchase products made from limited non-renewable resources (e.g., petroleum, natural gas and coal). Other consumers may have adverse perceptions about products derived from petrochemicals as being "unnatural" or not environmentally friendly.

Thus, it is generally desirable to provide plastic container for the cleansing products named above which are substantially free of virgin petroleum-based compounds, 100% sustainable, and 100% recyclable, while having a shelf life of at least two years.

However, current plastic container also can face difficulties during recycling. In the first few steps of a typical recycling procedure, a commonly used floatation process is used to separate polymers in a mixture based on density. Polymers that are more dense than water, such as polyethylene terephthalate, sink to the bottom of a solution, while polymers that are less dense than water, such as polyethylene and polypropylene, rise to the top of the solution. Contamination issues frequently occur during recycling because current plastic container that is highly filled or that is composed of some renewable materials often contains dense materials that sink during the floatation process and contaminate the polyethylene terephthalate stream (e.g., polylactic acid, highly filled high density polyethylene, or highly filled polypropylene). The polyethylene terephthalate stream is very sensitive to contamination, while the polyethylene stream is typically more robust.

Nonvirgin based material suitable to be used for container cosmetic products are usually post-consumer recycled polypropylene (PCR-PP) and post-consumer recycled polyethylene (PCR-PE). These materials can be obtained by recycling from post-consumer waste. Post-consumer waste is a waste type produced by the end consumer of a material stream; that is, where the waste-producing use did not involve the production of another product.

Post-consumer recycled polypropylene (PCR-PP) and post-consumer recycled polyethylene (PCR-PE) are generally available to be used for container cosmetic products, such as cleansing formulations. However, comparing container made of virgin polyethylene and polypropylene to those produced by using post-consumer recycled plastic, some differences can be noticed.

A particular problem with post-consumer recycled plastic is that it cannot be excluded that impurities in the recycled plastic outgas into the air-/headspace of a cosmetic container situated above the filling of the container. This effect may have considerable impact on the purchasing behavior of consumers. It is often observed that at the point of sale a consumer opens a bottle containing a cosmetic composition and starts to smell on the opening of the product to evaluate the fragrance of the composition. In this process the gas/air from the headspace of the bottle passes the opening of the bottle first. Thus, for the case that post-consumer recycled plastic is used, a mixture of the fragrance from the cosmetic composition itself and outgassed materials from the post-consumer recycled plastic is smelled by the consumer. The outgassed materials are often perceived as malodors. Hence, it is less likely that the consumer will buy the product.

In general, the intensity of the malodors caused by post-consumer recycled plastic in cosmetic products correlated with the shelf lifetime and the storage conditions of the products. Malodors may be extremely intensive if the products are stored at 40°C for a period of 4 months, thus that most common fragrance ingredients cannot compensate the smell.

The document WO 2012/102778 A1 discloses cosmetic container comprising recycled and bio-based plastic. However, there is disclosure how malodors caused by post-consumer recycled plastic can be addressed.

US 5350788A discloses a method of reducing malodors caused by recycled polymeric material such as plastic, which includes the step of incorporating a polyalkylene imine into the polymeric plastic material. However, the disadvantage of this method is that production cost of container made of post-consumer recycled plastic may be increased, due to a chemical modification of the plastic mixture.

An analysis of post-consumer recycled plastic revealed that potential malodors may be caused by various impurities. Difficulties arise especially from the fact the composition of the malodor causing impurities alternates depending on the source used for providing the post-consumer recycled plastic. Thus, if a cosmetic company tries to use post-consumer recycled plastic as container for consumer products it faces the problem that depending on the batch of the plastic different malodors may occur. Conventional cosmetic formulations are not capable of masking or preventing the formation of the large variety of malodors, such that malodors may be smelled by the consumer when opening a bottle which was stored for some time. Thus, it is generally desirable to provide cosmetic formulation which are capable of preventing or reducing the formation of malodors in the headspace of a post-consumer recycled plastic packing.

Typical malodor causing ingredients in post-consumer recycled plastic can be categorized in the group of alkanes and aldehydes. Examples of aldehydes, which can often be found in post-consumer recycled plastic are hexanal, octanal, nonanal, decanal, undecanal and dodecanal. Typically, dodecanal causes a soapy smell. Examples of alkanes are toluene and octane.

Fragrances can generally be technically distinguished by their scent family. In this way, molecules which may have a different chemical structure are categorized by their function. Categorizing chemical substances by their function is a standard procedure by a person skilled art. For example, the person skilled is well aware which chemicals are contained the group of rheology modifiers, even though the particular chemical structure of the individual chemicals is quite different. Typical examples of chemicals of different chemical structure but fulfilling the same function are carbomer and xanthan gum. As both are used to thicken cosmetic formulation both are contained int the group of rheology modifiers. Further examples of an analog categorization by the function and not by the molecular structure is known for further groups such as surfactants and emulsifier.

The person skilled in art categorizes fragrance ingredients by their scent family, which is done by applying the principle of the fragrance wheel provided by Michael Edwards. The fragrance wheel is described in the public domain e.g.in the internet accessible via the links https://www.fragrancex.com/blog/fragrance-wheel/ or https://en.wikipedia.org/wiki/Fragrance_wheel.

The fragrance wheel distinguishes the following group of scent families:

| **Families** | **Subfamilies** |
|---|---|
| **Floral** | Floral |
| | Soft Floral |
| | Floral Oriental |
| **Oriental** | Soft Oriental |
| | Oriental |
| | Woody Oriental |
| **Woody** | Woods |
| | Mossy Woods |
| | Dry Woods |
| | Aromatic Fougère |
| **Fresh** | Citrus |
| | Fruity |
| | Green |
| | Water |

It was now surprisingly found by the applicant that certain ingredients allow to prevent the formation and/or reduces the formation of malodors in the headspace of a cosmetic container. Prevent in terms of the present invention is understood, as the malodors are no more noticeable. Reduce means that the perceptibility of malodors is reduced. Both terms are related to perceptibility with a human nose.

A first object of the present invention is a cosmetic product comprising
a) a container comprising at least 80% by weight post-consumer recycled plastic, based on the total weight of the container, whereby the container has at least one opening,
b) a cosmetic cleansing composition situated in the container characterized in that the composition comprises
   - a fragrance having a floral family scent and
   - at least one surfactant.

A further object of the invention is the use of a cosmetic cleansing composition characterized in that the composition comprises
- a fragrance having a floral family scent and
- at least one surfactant,
to reduce malodors caused by post-consumer recycled plastic, whereby the cosmetic cleansing composition is situated in a container, whereby the container comprises at least 80% by weight post-consumer recycled plastic, based on the total weight of the container.

A second object of the present invention is a cosmetic product comprising
a) a container comprising at least 80% by weight post-consumer recycled plastic, based on the total weight of the container, whereby the container has at least one opening,
b) a cosmetic cleansing composition situated in the container characterized in that the composition comprises
   - a fragrance having a scent of coconut, orange, jasmine, sandalwood, vanilla and/or amber and
   - at least one surfactant.

A further object of the invention is the use of a cosmetic cleansing composition characterized in that the composition comprises
- a fragrance having a scent of coconut, orange, jasmine, sandalwood, vanilla and/or amber and
- at least one surfactant,
to reduce malodors caused by post-consumer recycled plastic, whereby the cosmetic cleansing composition is situated in a container, whereby the container comprises at least 80% by weight post-consumer recycled plastic, based on the total weight of the container.

The term "a container comprising at least 80% by weight of post-consumer recycled plastic" is understood as the material the container is composed of comprises at least 80% by weight of post-consumer recycled plastic. For the cases the container is described, the term "comprising" is always used to describe the material of which the container is made of.

According to the invention the container comprises at least 80% by weight post-consumer recycled plastic, based on the total weight of the container. It is preferred if the content of post-consumer recycled plastic is at least 85% by weight, more preferably 90% by weight, more preferably 95% by weight, more preferably 98% by weight, more preferably 99% by weight and most preferably 100% by weight, calculated to the total weight of the container.

According to the invention the container is preferably substantially free of virgin petroleum-based materials. In particular, it is preferred if virgin petroleum-based materials are contained in quantities of less than about 10 % by weight, preferably less than about 5% by weight, more preferably less than about 3% by weight, based on the total weight of the container. Further it is preferred if virgin petroleum-based materials are contained in quantities of less than about 2% by weight, preferably less than about 1% by weight, more preferably 0% by weight, based on the total weight of the container. Thus, it is most preferred if virgin petroleum-based materials are not contained in the container. As used herein, "virgin petroleum-based" refers to materials that are derived from a petroleum source, such as oil, natural gas, or coal, and that have not been recycled, either industrially or through the consumer waste stream.

Besides post-consumer recycled plastic it is in some embodiments of the invention preferred if the container comprises to some extent renewable polymeric materials. Examples of renewable polymeric materials include bio-polyethylene, bio-polyethylene terephthalate, and bio-polypropylene. As used herein, the prefix "bio-" is used to designate a material that has been derived from a renewable resource.

Bio-polyethylene is produced from the polymerization of bio-ethylene, which is formed from the dehydration of bio-ethanol. Bio-ethanol can be derived from, for example, (i) the fermentation of sugar from sugar cane, sugar beet, or sorghum; (ii) the saccharification of starch from maize, wheat, or manioc; and (iii) the hydrolysis of cellulosic materials. U.S. Patent Application Publication No. 2005/0272134, incorporated herein by reference, describes the fermentation of sugars to form alcohols and acids.

Suitable sugars used to form ethanol include monosaccharides, disaccharides, trisaccharides, and oligosaccharides. Sugars, such as sucrose, glucose, fructose, and maltose, are readily produced from renewable resources, such as sugar cane and sugar beets. As previously described, sugars also can be derived (e.g., via enzymatic cleavage) from other agricultural products (i.e., renewable resources resulting from the cultivation of land or the husbandry of animals). For example, glucose can be prepared on a commercial scale by enzymatic hydrolysis of corn starch. Other common agricultural crops that can be used as the base starch for conversion into glucose include wheat, buckwheat, arracaha, potato, barley, kudzu, cassava, sorghum, sweet potato, yam, arrowroot, sago, and other like starchy fruit, seeds, or tubers. The sugars produced by these renewable resources (e.g., corn starch from corn) can be used to produce alcohols, such as propanol, ethanol, and methanol. For example, corn starch can be enzymatically hydrolyzed to yield glucose and/or other sugars. The resultant sugars can be converted into ethanol by fermentation.

Monofunctional alcohols, such as ethanol and propanol can also be produced from fatty acids, fats (e.g., animal fat), and oils (e.g., monoglycerides, diglycerides, triglycerides, and mixtures thereof). These fatty acids, fats, and oils can be derived from renewable resources, such as animals or plants. "Fatty acid" refers to a straight chain monocarboxylic acid having a chain length of 12 to 30 carbon atoms. "Monoglycerides," "diglycerides," and "triglycerides" refer to containing multiple mono-, di- and tri- esters, respectively, of (i) glycerol and (ii) the same or mixed fatty acids unsaturated double bonds. Nonlimiting examples of fatty acids include oleic acid, myristoleic acid, palmitoleic acid, sapienic acid, linoleic acid, linolenic acid, arachidonic acid, eicosapentaenoic acid, and docosahexaenoic acid. Nonlimiting examples of monoglycerides include monoglycerides of any of the fatty acids described herein. Nonlimiting examples of diglycerides include diglycerides of any of the fatty acids described herein. Nonlimiting examples of the triglycerides include triglycerides of any of the fatty acids described herein, such as, for example, tall oil, corn oil, soybean oil, sunflower oil, safflower oil, linseed oil, perilla oil, cotton seed oil, tung oil, peanut oil, oiticica oil, hempseed oil, marine oil (e.g. alkali-refined fish oil), dehydrated castor oil, and mixtures thereof. Alcohols can be produced from fatty acids through reduction of the fatty acids by any method known in the art. Alcohols can be produced from fats and oils by first hydrolyzing the fats and oils to produce glycerol and fatty acids, and then subsequently reducing the fatty acids.

In a preferred embodiment, the bio-ethylene is produced from sugar cane. The life cycle stages of ethylene production from sugar cane include (i) sugar cane farming, (ii) fermentation of sugar cane to form bio-ethanol, and (iii) dehydration of bio-ethanol to form ethylene. Specifically, sugar cane is washed and transported to mills where sugar cane juice is extracted, leaving filter cake, which is used as fertilizer, and bagasse (residual woody fiber of the cane obtained after crushing). The bagasse is burned to generate steam and the electricity used to power the sugar cane mills, thereby reducing the use of petroleum-derived fuels. The sugar cane juice is fermented using yeast to form a solution of ethanol and water. The ethanol is distilled from the water to yield about 95% pure bio-ethanol. The bio-ethanol is subjected to catalytic dehydration (e.g., with an alumina catalyst) to produce ethylene, which is subsequently polymerized to form bio-polyethylene.

BRASKEM has demonstrated the production of high density polyethylene (HDPE) and linear, low density polyethylene (LLDPE) from sugar cane using a Hostalen/Basell technology for the HDPE production and a Spherilene/Basell technology for the LLDPE production. These catalysts allow (in some cases), superior processability of the bio-polyethylene and results in products with superior consistency to incumbent resins made by other processes.

As used herein and unless otherwise noted, "polyethylene" encompasses high density polyethylene (HDPE), low density polyethylene (LDPE), linear low density polyethylene (LLDPE), and ultra low density polyethylene (ULDPE). As used herein and unless otherwise noted, "polypropylene" encompasses homopolymer polypropylene, random copolymer polypropylene, and block copolymer polypropylene

The container is preferably composed of at least about 25% by weight, preferably at least about 35% by weight, more preferably at least about 50% by weight, even more preferably at least about 75% by weight, for example, at least about 95% by weight or about 100% by weight of polypropylene (PP), based on the total weight of the container. According to the invention it is preferred if the polypropylene is characterized in that it is composed of post-consumer recycled polypropylene (PCR-PP), post-industrial recycled polypropylene (PIR-PP), regrind polypropylene, and a mixture thereof. Most preferred is post-consumer recycled polypropylene (PCR-PP).

Further it is preferred if the container is composed of at least about 30% by weight, preferably at least about 50% by weight, more preferably at least about 65% by weight, even more preferably at least about 85% by weight, for example, at least about 95% by weight or about 100% by weight of polyethylene (PE), based on the total weight of the container. According to the invention it is preferred if the polyethylene is characterized in that it is composed of post-consumer recycled polyethylene (PCR-PE), post-industrial recycled polyethylene (PIR-PE), regrind polyethylene, and a mixture thereof. Most preferred is post-consumer recycled polyethylene (PCR-PE).

According to the invention it is preferred if the container is composed of both, polyethylene and polypropylene. Further it is preferred if the container is composed of a mixture comprising post-consumer recycled polyethylene (PCR-PE) and post-consumer recycled polypropylene (PCR-PP).

It is preferred if the ratio by weight between polyethylene and polypropylene is in the range from 1000:1 to 1:1000, preferably from 500:1 to 1:500 and most preferably from 100:1 to 1:100. In particular, it is preferred if the ratio by weight between post-consumer recycled polyethylene (PCR-PE) and post-consumer recycled polypropylene (PCR-PP) is in the range from 1000:1 to 1:1000, preferably from 500:1 to 1:500 and most preferably from 100:1 to 1:100. In one embodiment it is particular preferred if the ratio by weight between post-consumer recycled polyethylene (PCR-PE) and post-consumer recycled polypropylene (PCR-PP) is in the range from 150:1 to 1:1, preferably from 120:1 to 50:1 and most preferably from 100:1 to 90:1. In one embodiment it is particular preferred if the ratio by weight between post-consumer recycled polypropylene (PCR-PP) and post-consumer recycled polyethylene (PCR-PE) is in the range from 150:1 to 1:1, preferably from 120:1 to 50:1 and most preferably from 100:1 to 90:1.

According to the invention it is preferred is the polyethylene and/or polypropylene used is characterized in that it individually contains impurities, in particular aldehydes, alcohols, ester and/o hydrocarbon compounds. These compounds count to the total weight of the designated polyethylene or polypropylene as these are residues from the post-consumer use recycling process and these impurities are inevitably and irremovably linked to the polypropylene and polyethylene.

Further it is preferred if the container is build-up of a single layer of post-consumer recycled plastic, in particular post-consumer recycled polyethylene (PCR-PE) and/or post-consumer recycled polypropylene (PCR-PP). Thus, it is preferred if the cosmetic cleansing composition is in direct contact with the post-consumer recycled plastic, in particular post-consumer recycled polyethylene (PCR-PE) and/or post-consumer recycled polypropylene (PCR-PP). Thus, it is preferred if the container has no separation layer between the post-consumer recycled plastic, in particular post-consumer recycled polyethylene (PCR-PE) and/or post-consumer recycled polypropylene (PCR-PP), and the contact surfaced inside the container. The contact surface inside the container is in contact with the cosmetic cleansing formulation.

In other embodiments of the present invention the container is characterized in that the wall of the container it comprises at least two layers of plastic, whereby at least one layer, preferably all layer, comprises post-consumer recycled polyethylene (PCR-PE) and/or post-consumer recycled polypropylene (PCR-PP). Conveniently, it is preferred if the most outer layer of the container comprises at least one colorant. In this way it is possible to have a coherent outer appearance on the container on the shelf of a store.

It is preferred if the cosmetic product further comprises a cap which is removably attached to the container and which can be moved between a closed position, in which the cap closes the opening of the container and an open position, which opens the opening of the container, such that the cosmetic cleansing composition can be taken out of the container.

In agreement with the container, it is preferred if the cap is composed of post-consumer recycled plastic, which is in particular polyethylene and/or polypropylene. Accordingly, it is preferred if the cap is composed of at least about 25% by weight, preferably at least about 35% by weight, more preferably at least about 50% by weight, even more preferably at least about 75% by weight, for example, at least about 95% by weight or about 100% by weight of polypropylene (PP), based on the total weight of the cap. According to the invention it is preferred if the polypropylene is characterized in that it is composed of post-consumer recycled polypropylene (PCR-PP), post-industrial recycled polypropylene (PIR-PP), regrind polypropylene, and a mixture thereof. Most preferred is post-consumer recycled polypropylene (PCR-PP).

Further it is preferred if the cap is composed of at least about 25% by weight, preferably at least about 35% by weight, more preferably at least about 50% by weight, even more preferably at least about 75% by weight, for example, at least about 95% by weight or about 100% by weight of polyethylene (PE), based on the total weight of the cap. According to the invention it is preferred if the polyethylene is characterized in that it is composed of post-consumer recycled polyethylene (PCR-PE), post-industrial recycled polyethylene (PIR-PE), regrind polyethylene, and a mixture thereof. Most preferred is post-consumer recycled polyethylene (PCR-PE).

According to the invention it is preferred if the cap is composed of both, polyethylene and polypropylene. Further it is preferred if the cap is composed of a mixture comprising post-consumer recycled polyethylene (PCR-PE) and post-consumer recycled polypropylene (PCR-PP).

Regarding the cap, it is preferred if the ratio by weight between polyethylene and polypropylene is in the range from 1000:1 to 1:1000, preferably from 500:1 to 1:500 and most preferably from 100:1 to 1:100. In particular, it is preferred if the ratio by weight between post-consumer recycled polyethylene (PCR-PE) and post-consumer recycled polypropylene (PCR-PP) is in the range from 1000:1 to 1:1000, preferably from 500:1 to 1:500 and most preferably from 100:1 to 1:100. In one embodiment it is particular preferred if the ratio by weight between post-consumer recycled polyethylene (PCR-PE) and post-consumer recycled polypropylene (PCR-PP) is in the range from 150:1 to 1:1, preferably from 120:1 to 50:1 and most preferably from 100:1 to 90:1. In one embodiment it is particular preferred if the ratio by weight between post-consumer recycled polypropylene (PCR-PP) and post-consumer recycled polyethylene (PCR-PE) is in the range from 150:1 to 1:1, preferably from 120:1 to 50:1 and most preferably from 100:1 to 90:1.

According to the invention it is preferred if a label is attached to the container. The label is preferably composed of a substrate that includes a polymer selected from the group consisting of polyethylene, polyethylenterephthalate and polypropylene. In some embodiements of the invention it is preferred if the polyethylene, polyethylenterephthalate and/or polypropylene, which are used for the label have a biobased content of at least about 90%, preferably at least about 93%, more preferably at least about 95%, for example about 100%. Alternatively, or in addition, it is preferred if the polyethylene, polyethylenterephthalate and/or polypropylene, which is used for the label is made of post-consumer recycled plastic. In this context post-consumer recycled polyethylene, post-consumer recycled polyethylenterephthalate and/or post-consumer recycled polypropylene are used for the label.

The label further includes ink, which can be solvent-based or water-based. In some embodiments, the ink is derived from a renewable resource, such as soy, a plant, or a mixture thereof. The ink can be cured using heat or ultraviolet radiation (UV). In some preferred embodiments, the ink is cured by UV, which results in a reduction of curing time and energy output. Nonlimiting examples of inks include ECO-SURE!^{™} from Gans Ink & Supply Co. and the solvent-based VUTEk^{®} and BioVu^{™} inks from EFI, which are derived completely from renewable resources (e.g., corn).

The label can be fixed to the container using adhesive. In some preferred embodiments, the adhesive is a renewable adhesive, such as BioTAK^{®} by Berkshire Labels, which is fully biodegradable and compostable, conforms to European standard EN 13432, and is approved by the FDA, a shrink sleeve, or by melting the label onto the container during manufacturing. Alternatively, the label can be molded directly into the plastic of the container.

The label can optionally comprise layers. For example, a metallization effect results when a layer composed of ink/metallization is flanked by outer layers composed of polyethylene in a trilayer label.

As used herein, "biobased content" refers to the amount of bio-carbon in a material as a percent of the weight (mass) of the total organic carbon in the product. For example, polyethylene contains two carbon atoms in its structural unit. If ethylene is derived from a renewable resource, then a homopolymer of polyethylene theoretically has a biobased content of 100% because all of the carbon atoms are derived from a renewable resource. A copolymer of polyethylene could also theoretically have a biobased content of 100% if both the ethylene and the co-monomer are each derived from a renewable resource. In embodiments where the co-monomer is not derived from a renewable resource, the HDPE will typically include only about 1% by weight to about 2% by weight of the non-renewable co-monomer, resulting in HDPE having a theoretical biobased content that is slightly less than 100% by weight. As another example, polyethylene terephthalate contains ten carbon atoms in its structural unit (i.e., two from the ethylene glycol monomer and eight from the terephthalic acid monomer). If the ethylene glycol portion is derived from a renewable resource, but the terephthalic acid is derived from a petroleum-based resource, the theoretical biobased content of the polyethylene terephthalate is 20% by weight.

A suitable method to assess materials derived from renewable resources is through ASTM D6866, which allows the determination of the biobased content of materials using radiocarbon analysis by accelerator mass spectrometry, liquid scintillation counting, and isotope mass spectrometry. When nitrogen in the atmosphere is struck by an ultraviolet light produced neutron, it loses a proton and forms carbon that has a molecular weight of 14, which is radioactive. This 14C is immediately oxidized into carbon dioxide, which represents a small, but measurable fraction of atmospheric carbon. Atmospheric carbon dioxide is cycled by green plants to make organic molecules during the process known as photosynthesis. The cycle is completed when the green plants or other forms of life metabolize the organic molecules producing carbon dioxide, which causes the release of carbon dioxide back to the atmosphere. Virtually all forms of life on Earth depend on this green plant production of organic molecules to produce the chemical energy that facilitates growth and reproduction. Therefore, the 14C that exists in the atmosphere becomes part of all life forms and their biological products. These renewably based organic molecules that biodegrade to carbon dioxide do not contribute to global warming because no net increase of carbon is emitted to the atmosphere. In contrast, fossil fuel-based carbon does not have the signature radiocarbon ratio of atmospheric carbon dioxide. See WO 2009/155086, incorporated herein by reference.

The application of ASTM D6866 to derive a "biobased content" is built on the same concepts as radiocarbon dating, but without use of the age equations. The analysis is performed by deriving a ratio of the amount of radiocarbon (14C) in an unknown sample to that of a modern reference standard. The ratio is reported as a percentage with the units "pMC" (percent modern carbon). If the material being analyzed is a mixture of present day radiocarbon and fossil carbon (containing no radiocarbon), then the pMC value obtained correlates directly to the amount of biomass material present in the sample.

The modern reference standard used in radiocarbon dating is a NIST (National Institute of Standards and Technology) standard with a known radiocarbon content equivalent approximately to the year AD 1950. The year AD 1950 was chosen because it represented a time prior to thermo-nuclear weapons testing, which introduced large amounts of excess radiocarbon into the atmosphere with each explosion (termed "bomb carbon"). The AD 1950 reference represents 100 pMC.

"Bomb carbon" in the atmosphere reached almost twice normal levels in 1963 at the peak of testing and prior to the treaty halting the testing. Its distribution within the atmosphere has been approximated since its appearance, showing values that are greater than 100 pMC for plants and animals living since AD 1950. The distribution of bomb carbon has gradually decreased over time, with today's value being near 107.5 pMC. As a result, a fresh biomass material, such as corn, could result in a radiocarbon signature near 107.5 pMC. Petroleum-based carbon does not have the signature radiocarbon ratio of atmospheric carbon dioxide. Research has noted that fossil fuels and petrochemicals have less than about 1 pMC, and typically less than about 0.1 pMC, for example, less than about 0.03 pMC. However, compounds derived entirely from renewable resources have at least about 95 percent modern carbon (pMC), preferably at least about 99 pMC, for example, about 100 pMC.

Combining fossil carbon with present day carbon into a material will result in a dilution of the present day pMC content. By presuming that 107.5 pMC represents present day biomass materials and 0 pMC represents petroleum derivatives, the measured pMC value for that material will reflect the proportions of the two component types. A material derived 100% by weight from present day soybeans would give a radiocarbon signature near 107.5 pMC. If that material was diluted with 50% by weight petroleum derivatives, it would give a radiocarbon signature near 54 pMC.

A biobased content result is derived by assigning 100% by weight equal to 107.5 pMC and 0% by weight equal to 0 pMC. In this regard, a sample measuring 99 pMC will give an equivalent biobased content result of 93% by weight.

At least one of the container, cap, or label may optionally include a colorant masterbatch. As used herein, a "colorant masterbatch" refers to a mixture in which pigments are dispersed at high concentration in a carrier material. The colorant masterbatch is used to impart color to the final product. In some embodiments, the carrier is a biobased plastic or a petroleum-based plastic, while in alternative embodiments, the carrier is a biobased oil or a petroleum-based oil. The colorant masterbatch can be derived wholly or partly from a petroleum resource, wholly or partly from a renewable resource, or wholly or partly from a recycled resource. Nonlimiting examples of the carrier include bio-derived or post-consumer recycled polyethylene (e.g,. LLDPE, LDPE, HDPE), post-consumer recycled terephthalate, post-consumer recycled polypropylene, and a mixture thereof. The pigment of the carrier, which can be derived from either a renewable resource or a nonrenewable resource, can include, for example, an inorganic pigment, an organic pigment, a polymeric resin, or a mixture thereof. Nonlimiting examples of pigments include titanium dioxide (e.g., rutile, anatase), copper phthalocyanine, antimony oxide, zinc oxide, calcium carbonate, fumed silica, phthalocyamine (e.g., phthalocyamine blue), ultramarine blue, cobalt blue, monoazo pigments, diazo pigments, acid dye, base dye, quinacridone, and a mixture thereof. In some embodiments, the colorant masterbatch can further include one or more additives, which can either be derived from a renewable resource or a non-renewable resource. Nonlimiting examples of additives include slip agents, UV absorbers, nucleating agents, UV stabilizers, heat stabilizers, clarifying agents, fillers, brighteners, process aids, perfumes, flavors, and a mixture thereof.

In some embodiments, color can be imparted to the container, cap, or label in any of the aspects by using direct compounding (i.e., in-line compounding).

The container of the invention can be produced using blow molding. Blow molding is a manufacturing process by which hollow plastic parts are formed from thermoplastic materials.

The blow molding process begins with melting down thermoplastic and forming it into a parison or preform. The parison is a tube-like piece of plastic with a hole in one end through which compressed air can pass. Pressurized gas, usually air, is used to expand the parison or the hot preform and press it against a mold cavity. The pressure is held until the plastic cools. After the plastic has cooled and hardened the mold opens up and the part is ejected. There are three main types of blow molding: extrusion blow molding, injection blow molding, and injection stretch blow molding. In extrusion blow molding, a molten tube of plastic is extruded into a mold cavity and inflated with compressed air. One end of the cylinder is pinched closed. After the plastic part has cooled, it is removed from the mold. Extrusion blow molding can be used to produce the HDPE and PP containers of the invention. These containers can be single layer or multilayer.

Injection blow molding (IBM) involves three steps: injection, blowing and ejection. First, molten polymer is fed into a manifold where it is injected through nozzles into a hollow, heated preform mold. The preform mold forms the external shape of the resulting container and is clamped around a mandrel (the core rod) which forms the internal shape of the preform. The preform consists of a fully formed bottle/jar neck with a thick tube of polymer attached, which will form the body. The preform mold opens and the core rod is rotated and clamped into the hollow, chilled blow mold. The core rod opens and allows compressed air into the preform, which inflates it to the finished article shape. After a cooling period the blow mold opens and the core rod is rotated to the ejection position. The finished article is stripped off the core rod and leak-tested. Injection blow molding, as well as the other blow molding methods described herein, is useful for the formation of article components that have embedded biodegradable polymer. Injection blow molding can be used to produce containers that include blends of biodegradable or post-consumer recycled polymers.

Injection stretch blow molding (ISBM) is a method for producing a plastic container from a preform or parison that is stretched in both the hoop direction and the axial direction when the preform is blown into its desired container shape. In the ISBM process, a plastic is first molded into a "preform" using the injection molding process. These preforms are produced with the necks of the containers, including threads. The preforms are packaged, and after cooling, fed into a reheat stretch blow molding machine. The preforms are heated above their glass transition temperature, then blown using high pressure air into containers using metal blow molds. Typically, the preform is stretched with a core rod as part of the process. Injection stretch blow molding can be used to produce the HDPE, PET, and PP containers of the invention.

The caps of the invention can be formed using injection molding. Injection molding is a manufacturing process for producing parts from thermoplastic materials, thermosetting plastic materials, or a mixture thereof. During injection molding, polymeric material is fed into a barrel, mixed, formed into a melt, and forced into a three-dimensional mold cavity where it solidifies into the configuration of the mold cavity via cooling, heating, and/or chemical reaction. Injection molding can be used to make single layer caps or multilayer caps.

The labels of the invention can be formed using film extrusion. In film extrusion, thermoplastic material is melted and formed into a continuous profile. In some embodiments, multilayer films are coextruded. Film extrusion and coextrusion can be performed by any method known to one skilled in the art.

Regarding the composition it should be noted that fragrances leading to a floral scent can preferably be created using one or more ingredients taken from the following list (List A): (-)-alpha-terpineol; (+)-alpha-terpineol; (+)-nerolidol; (±)-2,3-dihydrofarnesol; (±)-2,4,8-trimethyl-7-nonen-2-ol; (±)-3-((2-methyl-3-furyl)thio)-2-butanone; (±)-3-methyl-gamma-decalactone; (±)-4-ethyl octanal; (±)-8,9-dehydrotheaspirone; (±)-lilac aldehyde; (2-methoxy-1-methyl butyl) benzene; (2-methoxy-1-methyl propyl) benzene; (2S)-1-isopropoxy-1-oxopropan-2-yl pivalate; (3-methoxy-2-methyl propyl) benzene; (3S,6S)-(Z)-linalool oxide (pyranoid); (4E,9Z)-13-methyloxacyclopentadeca-4,9-dien-2-one; (E)-2,5,9-trimethyl-4,9-decadien-1-al; (E)-2-hexen-1-yl salicylate; (E)-2-undecen-1-ol; (E)-3-hexen-1-ol; (E)-4-undecenal; (E)-5-decen-1-ol; (E)-7-methyl-3-octen-2-one; (E)-alpha-amyl cinnamaldehyde; (E)-alpha-damascone; (E)-alpha-methyl ionone; (E)-beta-damascone; (E)-beta-ionone; (E)-beta-methyl ionone; (E)-cinnamophenone; (E)-cinnamyl acetate; (E)-citronellyl tiglate; (E)-ethyl tiglate; (E)-geranyl linalool; (E)-isoeugenol; (E)-linalool oxide (furanoid); (E)-nerolidol; (E)-para-methoxycinnamaldehyde; (E)-squalene; (E)-trimethyl phenyl butenone; (E,E)-2,4-hexadienal; (E,E)-2,6-alloocimene; (E,E)-digeranyl ether; (E,E)-farnesol; (E+Z)-4,8-dimethyl-3,7-nonadien-2-one; (R)-(+)-dihydro-alpha-ionone; (R)-1-phenyl propyl alcohol; (R)-2-phenyl propionaldehyde; (R)-2-phenyl propyl alcohol; (R)-citronellyl nitrile; (R)-dihydro-beta-ionol; (R)-dihydro-gamma-ionone; (R)-hydroxycitronellal; (R)-linden ether; (R)-styralyl acetate; (R)-styralyl alcohol; (S)-(-)-dihydro-alpha-ionone; (S)-1-phenyl propyl alcohol; (S)-2,5,6-trimethyl-2-heptanol; (S)-2-phenyl propionaldehyde; (S)-2-phenyl propyl alcohol; (S)-campholene acetate; (S)-dihydro-beta-ionol; (S)-dihydro-gamma-ionone; (S)-hydroxycitronellal; (S)-ocean propanal; (S)-rhodinol; (Z)-2-octen-1-ol; (Z)-3-hexen-1-yl acetoacetate; (Z)-3-hexen-1-yl angelate; (Z)-3-hexen-1-yl benzoate; (Z)-3-hexen-1-yl methyl carbonate; (Z)-3-hexen-1-yl salicylate; (Z)-4-decen-1-yl acetate; (Z)-4-hepten-2-yl salicylate; (Z)-8-tetradecenal; (Z)-8-undecenal; (Z)-9-undecene nitrile; (Z)-alpha-amyl cinnamaldehyde; (Z)-alpha-damascone; (Z)-beta-damascone; (Z)-beta-ocimene; (Z)-cinnamyl acetate; (Z)-jasmone; (Z)-linalool oxide (furanoid); (Z)-methyl epi-jasmonate; (Z)-nerolidol; (Z)-woody amylene; (Z,E)-phytol; [(4E,4Z)-5-methoxy-3-methyl-4-penten-1-yl] benzene; 1-(2-methyl allyl oxy)-2-methyl butane; 1-(2-methyl allyl oxy)-3-methoxy-3-methyl-1-butane; 1-(octahydro-4,7-methanoinden-5-yl)-propan-2-ol; 1-(octahydro-4,7-methanoinden-5-yl)-propan-2-one + 1-(6-methyloctahydro-4,7-methanoinden-5-yl)-ethanone; 1,3,3,5-tetramethyl-7 and 8-cyanobicyclo(2.2.2)oct-5-ene; 1,4-diisopropyl-6,8-dioxabicyclo(3.2.1)octane; 1,8-octane dinitrile; 1-[(3aS,7aR)-3a,4,7,7a-tetrahydro-1H-inden-6-yl]ethanone; 10-epi-gamma-eudesmol; 10-undecen-1-ol; 1-acetyl cyclohexyl acetate; 1-acetyl indole; 1-isobutyl-2-methyl butyl 2-butenoate; 1-octen-3-yl butyrate; 1-phenethyl mercaptan; 1-phenyl propyl alcohol; 1-phenyl propyl butyrate; 1-undecanol; 2-(2-cyanoethylidene)-3-methyl bicyclo(2.2.1)hept-5-ene; 2,2'-oxamidobis[ethyl-3-(3,5-di-tert-butyl-4-hydroxyphenyl)-propionate; 2,4,6-trimethyl-6-(3-methyl-2-buten-1-yl)-2-cyclohexen-1-one; 2,4-difurfuryl furan; 2',4'-dimethyl acetophenone; 2,4-dimethyl cyclohexyl methyl acetate; 2,4-dimethyl-3-cyclohexene-1-methanol; 2,4-dimethyl-3-cyclohexene-1-methanyl acetate; 2,4-dimethyl-alpha-allyl-3-cyclohexene methanol; 2,4-ivy carbaldehyde; 2,5,6-trimethyl-2-heptanol; 2,5-dimethyl-2-indan methanol; 2,6-dimethyl-2,18-nonadecadien-8-one; 2-acetyl-3-methyl thiophene; 2-butyl thiophene; 2-decalinyl acetate; 2-decalinyl formate; 2-decanone; 2-decenal; 2-dodecanone; 2-ethyl butyl 2-butenoate; 2-ethyl octine carbonate; 2-ethyl-1-hexanol; 2-ethyl-4-methyl-1,3-oxathiane; 2-ethylidene-6-methyl-cis-3-heptenal; 2-heptanol; 2-heptyl cyclopropane carboxylic acid; 2-heptyl pyridine; 2-heptyl tetrahydrofuran; 2-hexyl thiophene; 2-hexyl-4-acetoxytetrahydrofuran; 2-isopropoxyethyl salicylate; 2-methyl butyl angelate; 2-methyl butyl salicylate; 2-methyl naphthalene; 2-methyl octanal; 2-methyl-4-phenyl butanal; 2-methyl-4-phenyl-1,3-dioxolane; 2-naphthyl phenyl ketone; 2-nonanone oxime; 2-nonanone propylene glycol acetal; 2-octanone oxime; 2-para-cresyl ethanol; 2-para-cresyl oxyethyl acetate; 2-pentyl cyclopentan-1-ol; 2-phenyl propionaldehyde dimethyl acetal; 2-phenyl propionaldehyde ethylene glycol acetal; 2-phenyl propyl isobutyrate; 2-phenyl-2-pentenal; 2-phenyl-4-pentenal; 2-tetradecenal; 2-undecanone; 2-undecen-1-ol; 3-(3-propen-2-yl phenyl) butanal; 3-(3-tert-butyl phenyl) propanal; 3-(4-hydroxy-4-methyl cyclohexyl) butanal; 3-(4-isobutyl-2-methylphenyl)propanal; 3-(4-propyl phenyl) propionaldehyde; 3-(5-methyl-2-furyl) butanal; 3-(benzyl oxy)-2,2-dimethyl propanal; 3',4'-dimethoxyacetophenone; 3,4-dimethoxystyrene; 3,5,5-trimethyl hexanol; 3,6-dimethyl-3-octyl acetate; 3,6-dimethylheptan-2-ol; 3,7-dimethyl-6-octenoic acid; 3-[(4R)-4-(propan-2-yl)cyclohex-1-en-1-yl]propanal; 3-allylthio-2,6-dimethyl-4-heptanone; 3-crotylthio-2,6-dimethyl-4-heptanone; 3-cyclohexyl propyl acetate; 3-decanol; 3-decanone; 3-heptyl acetate; 3-isopropenyl-6-oxoheptanoic acid; 3-mercaptohexyl acetate; 3-methyl pentyl angelate; 3-methyl-2-hexen-1-ol 1-acetate; 3-methyl-4-(2-methyl butyl oxy) butyraldehyde; 3-methyl-4-hexyl oxybutyraldehyde; 3-methyl-6-ethyl-5-octen-1-ol; 3-nonanon-1-yl acetate; 3-pentyl bicyclo[3.2.1]octan-2-one; 3-phenyl propyl formate; 3-phenyl propyl propionate; 3-propyl bicyclo(3.2.1)-6-octen-2-one; 4-(1,1,2,3,3-pentamethyl indan-5-yl) pyrimidine; 4-(2,3-dihydro-1,1,2,3,3-pentamethyl-1H-inden-5-yl)pyrimidine; 4-(para-tolyl)-2-butanone; 4,6,11-trimethyl-5-oxatricyclo(6.2.2.0*4,9*)dodec-6-ene; 4,6-dimethyl-alpha-allyl-3-cyclohexene methanol; 4,8-dimethyl-7-nonen-2-ol; 4-cyclohexyl cyclohexanone; 4-cyclopentyl pentane nitrile; 4-dimethyl ionone; 4-ethoxy-3-anisaldehyde; 4-ethyl cyclohexane propanal; 4-hydroxyphenethyl alcohol; 4-isobutyl cyclohexane propanal; 4-mercapto-4-methyl-2-hexanone; 4-mercapto-4-methyl-2-pentanol; 4-methoxy-alpha-toluene thiol; 4-methyl biphenyl; 4-methyl cyclohexane propanal; 4-methyl-2-(2-methyl-1-propenyl)-1,3-dioxane; 4-methyl-3-pentyl-2(5H)-furanone; 4-methyl-4-phenyl pentanone; 4-methyl-4-phenyl-2-pentanol; 4-methyl-8-vinyl-4,7-nonadienal; 4-methyl-8-vinyl-4,8-nonadienal; 4-phenyl-2-butanol; 4-phenyl-3-buten-2-ol; 4-propyl cyclohexane propanal; 4-tert-butyl cyclohexane carboxaldehyde; 5-tricyclodecenyl acetate; 6,7,8-decen-1-ol; 6,8-dimethyl-2-nonanol; 6-methyl coumarin; 7-allyl benzo[b][1,4]dioxepin-3-one; 8,9-dehydrotheaspirone; 8-methylnonanal; 9-[9,12-epoxy-ethyl]-5-methyl-tricyclo[6.2.1.0.sup.2,7 ]undec-4-ene; 9-decenal; abrialis lavandin oil; acetal 318; acetaldehyde dibutyl acetal; acetaldehyde ethyl phenethyl acetal; acetyl tetralin; achillea millefolium herb oil CO2 extract; adoxal (Givaudan); alloocimene; allyl acetone; allyl anthranilate; allyl benzoate; alpha-acorenol; alpha-allyl-4,6-dimethyl-3-cyclohexene-1-methanyl acetate; alpha-allyl-4-methyl-3-cyclohexene-1-methanyl acetate; alpha-amyl cinnamaldehyde; alpha-amyl cinnamaldehyde / methyl anthranilate schiffs base; alpha-amyl cinnamaldehyde diethyl acetal; alpha-amyl cinnamyl acetate; alpha-amyl cinnamyl alcohol; alpha-bisabolene; alpha-bisabolol; alpha-butyl cinnamaldehyde; alpha-damascone; alpha-decalactone; alpha-hexyl cinnamaldehyde; alpha-ionol; alpha-ionone; alpha-ionone; alpha-ionyl acetate; alpha-irone; alpha-isomethyl ionone; alpha-methyl cinnamyl alcohol; alpha-methyl ionone; alpha-ocimene; alpha-terpineol; amber dioxane; amyl angelate; amyl benzoate; amyl cyclopentanone propanone; amyl isoeugenol; amyl nonanoate; amyl salicylate; aniba rosaeodora var. amazonica wood extract; anise indene; anisyl propanal / methyl anthranilate schiff's base; anthocephalus cadamba oil; atlas cedarwood oil fractions; autumn carboxylate; benzaldehyde / methyl anthranilate schiffs base; benzyl acetate; benzyl acetoacetate; benzyl acetone; benzyl alcohol; benzyl anthranilate; benzyl cinnamate; benzyl eugenol; benzyl formate; benzyl lactate; benzyl methyl ether; benzyl phenyl acetate; benzyl phenyl carbinol; benzyl pivalate; benzyl propionate; benzyl valerate; benzylidene acetone; bergamot acetoacetate; bergamot oil; berry hexanoate; beta-bisabolol; beta-bourbonene; beta-damascone; beta-ionol; beta-ionone; beta-isomethyl ionone; beta-methyl ionone; beta-naphthyl anthranilate; beta-naphthyl ethyl ether; beta-naphthyl methyl ether; beta-phenoxyethyl acetate; bitter orange peel oil ; bois de rose oil ;brachyleana hutchinsii wood oil; butyl 2-naphthyl ether; butyl anthranilate; butyl benzyl ether; butyl para-cresol ether; butyl tiglate; butyl undecylenate; cabreuva wood oil; calendula officinalis flower oil ;callitris intratropica wood oil ;callitropsis araucarioides wood oil; camellia oleifera leaf extract; cananga fruit oil; cananga leaf oil; cananga odorata flower extract; cananga oil; cardamom seed oil; carrot seed oil ;cedarwood oil ;cedarwood oil terpenes; cerastium candidissimum corr. oil; cestrum nocturnum flower oil; chamomile isobutyrate; chamomile propionate; chamomile valerate; chrysanthemum ketone; chrysanthemum oxide; cinnamyl acetate; cinnamyl cinnamate; cinnamyl formate; cis-muguet shiseol; cis-woody acetate; citral dimethyl acetal; citral ethylene glycol acetal; citronellal; citronellol; citronellone; citronellyl (R)-lactate; citronellyl acetate; citronellyl acetone; citronellyl butyrate; citronellyl ethyl ether; citronellyl hexanoate; citronellyl isobutyrate; citronellyl nitrile; citronellyl oxyacetaldehyde; citronellyl propionate; citronitrile; citrus ocimenol; citrus reticulata blanco var. mandarin oil ;clary propionate; clary propyl acetate; convolvulus scoparius wood oil; coranol; coriander oleoresin; coriander seed oil; corps popinal; cumin carbinol; cuminyl acetaldehyde; cyclamen aldehyde; cyclamen homoaldehyde; cyclamen propanal; cyclohexyl benzoate; cyclohexyl butyrate; cyclohexyl crotonate; cyclohexyl ethyl alcohol; cyclohexyl propanol; cyclohexyl salicylate; cymbopogon validus leaf oil; damascate; daniellia oliveri bark oil ;decanal ; decanal methyl enol ether; decanal propylene glycol acetal; decanol; decyl anthranilate; dehydrodihydroionone; dextro-linalool; dianthus ethone; diethyl acetonyl diisopropyl acetonyl sulfide; dihexyl (E)-fumarate; dihydro-alpha-ionone; dihydro-beta-ionol; dihydrocarvyl acetate; dihydrodamascone; dihydrofarnesol; dihydrogeranyl linalool; dihydroisojasmonate methyl ester; dihydromyrcenol; dihydroterpineol; diisobutyl carbinyl acetate; dimethyl alpha-ionone; dimethyl benzyl carbinol; dimethyl benzyl carbinyl acetate; dimethyl benzyl carbinyl butyrate; dimethyl glutarate; dimethyl succinate; diphenyl amine; dodecanal (aldehyde C-12 lauric); dryopteris filix-mas oleoresin; earthy acetal; earthy indane; epoxylinalyl acetate; eriocephalus punctulatus flower oil; eschweilera coriacea mori flower oil ; ethyl (E)-cinnamate; ethyl 2-benzyl butyrate; ethyl 2-furoate; ethyl 3,5,5-trimethyl hexanoate; ethyl 3-hydroxyoctanoate; ethyl 4-methyl salicylate; ethyl anthranilate; ethyl cyclohexyl acetate; ethyl laurate; ethyl levulinate; ethyl linalyl acetal; ethyl linalyl acetate; ethyl linalyl ether; ethyl methyl-para-tolyl glycidate; ethyl ortho-anisate; ethyl para-methyl-beta-phenyl glycidate; ethyl phenoxyacetate; ethyl phenyl acetate; ethyl salicylate; ethylene brassylate; ethylene glycol diacetate; eucalyptus citriodora oil; eugenyl acetate; exo-isocitral; farnesal; farnesol; farnesyl acetate; farnesyl acetone; farnesyl palmitoleate; fokienol; freesia heptanol; fruity carboxylate; furfuryl isopropyl sulfide; gamma-damascone; gamma-ionone; gardenia concrete; georgywood; geraniol; geranium dihydropyran; geranium leaf oil; geranium oil; geranyl acetate; geranyl acetone; geranyl heptanoate; geranyl isobutyrate; geranyl linalool; geranyl phenyl acetate; geranyl propionate; geranyl undecylenate; ginsene; globulol; glycoacetal; grapefruit pentanol; guaiacyl phenyl acetate; hawthorn acetate; hawthorn carbinol; hawthorn ethanol; helichrysum bracteatum flower oil; heliotropyl acetate; heliotropyl diethyl acetal; heptyl butyrate; heptyl formate; heptyl isobutyrate; heptyl nonanoate; herbal pyran; herniaria incana lam. oil ;hexadecanol; hexanal diethyl acetal; hexen-1-yl oxypropane nitrile; hexenyl cyclopentanone; hexoxyacetaldehyde dimethyl acetal; hexyl (Z)-tiglate; hexyl 2-furoate; hexyl lactate; hexyl oxyacetonitrile; hexyl pivalate; hibiscus sabdariffa flower extract; ho leaf oil; ho wood oil; homalomena rubescens root oil; homoambrinol; homoanisaldehyde; homomenthyl acetate; homovanillin; honey extract; hyacinth acetals; hyacinth ether; hydroxycitronellal; hydroxycitronellal / methyl anthranilate schiffs base; hydroxycitronellal diethyl acetal; hydroxycitronellal propylene glycol acetal; hydroxycitronellal residue; hydroxycitronellol; ilex paraguariensis oleoresin; immortelle flower oil; immortelle flower resinoid; indocolore; indole; intreleven aldehyde; inula root oil; ionone mixed isomers; iris germanica florentina root extract; isoamyl 3-(2-furan) propionate; isoamyl angelate; isoamyl cinnamate; isoamyl nonanoate; isoamyl salicylate; isoamyl undecylenate; isobutyl anthranilate; isobutyl benzyl carbinol; isobutyl citral; isobutyl furyl propionate; isobutyl octanoate; isobutyl phenyl acetate; isobutyl salicylate; isobutyraldehyde; isocyclodimethyl octanol; isocyclogeraniol; isodecanal; isoeugenol; isoeugenyl acetate; isoeugenyl ethyl acetal; isoeugenyl phenyl acetate; isogreen methanoindene; isojasmone; isojasmone; isophytol; isopropyl anthranilate; isopropyl benzoate; ivy carbaldehyde / methyl anthranilate schiffs base; ivy dioxolane; jambu flower oil brazil; jasimia; jasmin acetate; jasmin cyclopentanol; jasmin lactone ; jasmin oil; jasmin pyranol; jasminum grandiflorum extract; jasminum grandiflorum flower extract; jasmopyrane ; ketaki flower oil; kunzea ericoides leaf oil; laevo-citronellol; laevo-linalool; laevo-menthyl acetate; laevo-menthyl propionate; laevo-rose oxide; lavandin concrete; lavandin flower water; lavandin oil; lavandula angustifolia flower oil; lavender concrete; lavender oil; lavender oil bulgaria; lavender oil france; lavender oil greece; lavender oil terpeneless; lavender stem oil lithuania; lawsonia inermis flower oil; lecythis usitata miers. var. paraensis (ducke) r. kunth. flower oil; leerall; leerall / methyl anthranilate schiffs base; lepidine; leptospermum scoparium branch/leaf oil; lily propanol; lilyall; lime octadienal; lime oil ;linaloe wood oil; linalool; linalool oxide; linalyl acetate; linalyl acetate terpenes; linalyl benzoate; lonicera japonica flower extract; mace oleoresin; magnolia cyclohexanol; magnolia decadienal; magnolia flower oil ;magnolia indene; malpighia glabra fruit oil; mandarin oil; mandarin oil; marigold pot flower oil; marine decadienal; matricaria chamomilla flower oil ;melaleuca ericifolia leaf oil; melozol acetate; menthadienyl formate; meta-cresyl acetate; meta-cresyl phenyl acetate; meta-cresyl phenyl ether; methoxycitronellal; methoxymelonal; methyl (Z)-3-hexenoate; methyl (Z)-jasmonate; methyl 10-undecenoate; methyl 2-methyl benzoate; methyl 2-undecynoate; methyl 4-methyl benzoate; methyl 4-phenyl butyrate; methyl benzoate; methyl benzyl acetate; methyl bicycloheptenyl methyloxirane carboxylate; methyl citronellate; methyl cyclocitrone; methyl cyclogeranate; methyl decanoate; methyl dihydrojasmonate; methyl heptanoate; methyl hydrocinnamate; methyl ionyl acetate; methyl isobutyrate; methyl nerate; methyl octine carbonate; methyl ortho-anisate; methyl phenoxyethanol; methyl phenyl acetate; methyl trans-3-oxo-2-pentyl cyclopentane carboxylate; methyl undecylenate; mimosa heptanal; mimosa tenuiflora leaf extract; muguet butanal; muguet butanol; muguet carbaldehyde; muguet carboxaldehyde; muguet dienal; muguet ethanol; muguet nitrile; muguet octadienol; muguet propanol; muguet shiseol; muguet undecadienal; musk acetate; musk dec-8-enone; myrascone; myrcenol; myrcenyl acetate; myristica fragrans fruit extract; myrtle oil; myrtle oil morocco; narcissus acetate; narcissus jonquilla extract; neroli extract; neroli ketone; neroli oil; neroli oil bigarde; nerolidol; nerolidol oxide; nerolidyl propionate; nerolione; neryl acetate; neryl benzoate; neryl isovalerate; neryl propionate; nonanal / methyl anthranilate schiff's base; nonanal diethyl acetal; nonanal dimethyl acetal; nonanol; nonisyl propionate; nonyl octanoate; ocean carboxaldehyde; ocean propanal / methyl anthranilate schiffs base; ocimen-1-yl acetate; octahydro-4,7-methano-1H-indene-5-acetaldehyde; octahydro-4,7-methano-1H-indene-5-acetaldehyde + 6-methyl-octahydro-4,7-methano-indene-5-carbaldehyde; octanal / methyl anthranilate schiff'sbase; octanal dimethyl acetal; octyl isovalerate; octyl oxyacetaldehyde; octyl propionate; O-ethyl S-(2-furyl methyl) thiocarbonate; origanum majorana leaf oil; orris butenone; orris pyridine 25% IPM; orris rhizome concrete butter (iris pallida); orris rhizome oil (iris germanica); orris rhizome oil; orris rhizome resinoid (iris pallida); ortho-acetyl-para-cresol; ortho-cresyl acetate; ortho-methoxybenzyl ethyl ether; ortho-methyl anisole; palmarosa oil; papaya isobutyrate; para-anisaldehyde; para-anisaldehyde dimethyl acetal; para-anisyl alcohol; para-anisyl butyrate; para-anisyl formate; para-anisyl nitrile; para-cresyl alcohol; para-cresyl isobutyrate; para-cresyl laurate; para-cresyl phenyl ether; para-ethyl acetophenone; para-jasmone; para-methoxy-alpha-methyl cinnamaldehyde; para-methoxycinnamaldehyde; para-methyl benzyl acetate; para-methyl hydratropaldehyde; para-methyl phenoxyacetaldehyde; para-tolualdehyde propylene glycol acetal; peach nitrile; peach pivalate; pentenyl cyclopentanone; peony acetonitrile; peony alcohol; perilla alcohol; perillyl acetate; persea americana fruit oil; petal pyranone; petitgrain bigarade oil; petitgrain cedrat oil; petitgrain lemon oil; petitgrain mandarin oil; petitgrain oil; petitgrain oil fractions; petitgrain oil ;petitgrain oil terpeneless; petitgrain oil terpenes; petitgrain tangerine oil; phenethyl acetate; phenethyl alcohol; phenethyl anthranilate; phenethyl benzoate; phenethyl butyrate; phenethyl heptanoate; phenethyl hexanoate; phenethyl isobutyrate; phenethyl isovalerate; phenethyl phenyl acetate; phenethyl propionate; phenethyl salicylate; phenoxyacetaldehyde ; phenoxyethyl isobutyrate; phenyl acetaldehyde; phenyl acetaldehyde / methyl anthranilate schiff's base; phenyl acetaldehyde 2,3-butylene glycol acetal; phenyl acetaldehyde dicitronellyl acetal; phenyl acetaldehyde digeranyl acetal; phenyl acetaldehyde diisoamyl acetal; phenyl acetaldehyde diisobutyl acetal; phenyl acetaldehyde dimethyl acetal; phenyl acetaldehyde; phenyl acetic acid; phenyl amyl alcohol; phenyl butyrate; phenyl ethylidene acetone; phenyl propyl valerate; phytol; phytyl acetate; piperidine; plum crotonate; plum damascone; prenyl salicylate; propiophenone; propyl phenyl acetate; propyl pyruvate; pseudoionone; raspberry ketone; rhodinol; rhodinyl acetate substitutes; rhodinyl benzoate; rhodinyl formate; rhodinyl phenyl acetate; rhubarb oxirane; rhubarb pyran; rosa alba flower oil; rosa damascena flower oil; rosa odorata flower oil; rose acetate; rose butanoate; rose oil (rosa centifolia) ; rose undecene; S-(2,5-dimethyl-3-furyl) ethane thioate; S-(methyl thio) hexanoate; safrole; salicynile; sambucus nigra flower extract; sambucus nigra flower oil ;sandal cyclopropane; sandal octanol; santalyl acetate; santalyl phenyl acetate; santolina oil; sec-butyl ethyl ether; siam wood oil; spicy acetoacetate; spicy carbonate; spike lavender oil; star fruit oil; styralyl butyrate; styralyl formate; styralyl propionate; styralyl valerate; sunflower oil; surfleurs d'oranger oil; sweet petitgrain oil; tagete oil madagascar; tangerine oil terpeneless; tarchonanthus camphoratus oil; terpineol acetate; terpineols; terpinyl benzoate; terpinyl butyrate; terpinyl formate; terpinyl isobutyrate; terpinyl valerate; tetrahydrofurfuryl phenyl acetate; tetrahydroionol; tetrahydroionyl acetate; tetrahydrolinalool; tetrahydrolinalyl acetate; tetrahydromyrcenol; tilia cordata flower oil; tonka ketone; tropical ionone; tuberose oil; undecanal; undecanal dimethyl acetal; undecanal propylene glycol acetal; vanilla tahitensis fruit oil; vassoura oil; verdyl acetate; vetiver pentanone; violet dienyne; violet methyl carbonate; violet nitrile; violet propanol; vitispirane; white frangipani concrete; white nelumbo nucifera flower concrete; white sassafras oil; witch hazel leaf oil; yarrow oil; ylang ylang flower concrete; ylang ylang flower oil; yuzu leaf oil; yuzunone and zdravetz oil.

All scent ingredient names used in this disclosure can be allocated to a CAS number or a molecular structure via the website http://www.thegoodscentscompany.com/search2.html.

Accordingly, it is preferred if the composition comprises one or more of the ingredients of list A.

Thus, a preferred embodiment of the invention is a cosmetic product comprising
a) a container comprising at least 80% by weight post-consumer recycled plastic, based on the total weight of the container, whereby the container has at least one opening,
b) a cosmetic cleansing composition situated in the container characterized in that the composition comprises
   - at least one ingredient selected from the group consisting of (-)-alpha-terpineol; (+)-alpha-terpineol; (+)-nerolidol; (±)-2,3-dihydrofarnesol; (±)-2,4,8-trimethyl-7-nonen-2-ol; (±)-3-((2-methyl-3-furyl)thio)-2-butanone; (±)-3-methyl-gamma-decalactone; (±)-4-ethyl octanal; (±)-8,9-dehydrotheaspirone; (±)-lilac aldehyde; (2-methoxy-1-methyl butyl) benzene; (2-methoxy-1-methyl propyl) benzene; (2S)-1-isopropoxy-1-oxopropan-2-yl pivalate; (3-methoxy-2-methyl propyl) benzene; (3S,6S)-(Z)-linalool oxide (pyranoid); (4E,9Z)-13-methyloxacyclopentadeca-4,9-dien-2-one; (E)-2,5,9-trimethyl-4,9-decadien-1-al; (E)-2-hexen-1-yl salicylate; (E)-2-undecen-1-ol; (E)-3-hexen-1-ol; (E)-4-undecenal; (E)-5-decen-1-ol; (E)-7-methyl-3-octen-2-one; (E)-alpha-amyl cinnamaldehyde; (E)-alpha-damascone; (E)-alpha-methyl ionone; (E)-beta-damascone; (E)-beta-ionone; (E)-beta-methyl ionone; (E)-cinnamophenone; (E)-cinnamyl acetate; (E)-citronellyl tiglate; (E)-ethyl tiglate; (E)-geranyl linalool; (E)-isoeugenol; (E)-linalool oxide (furanoid); (E)-nerolidol; (E)-para-methoxycinnamaldehyde; (E)-squalene; (E)-trimethyl phenyl butenone; (E,E)-2,4-hexadienal; (E,E)-2,6-alloocimene; (E,E)-digeranyl ether; (E,E)-farnesol; (E+Z)-4,8-dimethyl-3,7-nonadien-2-one; (R)-(+)-dihydro-alpha-ionone; (R)-1-phenyl propyl alcohol; (R)-2-phenyl propionaldehyde; (R)-2-phenyl propyl alcohol; (R)-citronellyl nitrile; (R)-dihydro-beta-ionol; (R)-dihydro-gamma-ionone; (R)-hydroxycitronellal; (R)-linden ether; (R)-styralyl acetate; (R)-styralyl alcohol; (S)-(-)-dihydro-alpha-ionone; (S)-1-phenyl propyl alcohol; (S)-2,5,6-trimethyl-2-heptanol; (S)-2-phenyl propionaldehyde; (S)-2-phenyl propyl alcohol; (S)-campholene acetate; (S)-dihydro-beta-ionol; (S)-dihydro-gamma-ionone; (S)-hydroxycitronellal; (S)-ocean propanal; (S)-rhodinol; (Z)-2-octen-1-ol; (Z)-3-hexen-1-yl acetoacetate; (Z)-3-hexen-1-yl angelate; (Z)-3-hexen-1-yl benzoate; (Z)-3-hexen-1-yl methyl carbonate; (Z)-3-hexen-1-yl salicylate; (Z)-4-decen-1-yl acetate; (Z)-4-hepten-2-yl salicylate; (Z)-8-tetradecenal; (Z)-8-undecenal; (Z)-9-undecene nitrile; (Z)-alpha-amyl cinnamaldehyde; (Z)-alpha-damascone; (Z)-beta-damascone; (Z)-beta-ocimene; (Z)-cinnamyl acetate; (Z)-jasmone; (Z)-linalool oxide (furanoid); (Z)-methyl epi-jasmonate; (Z)-nerolidol; (Z)-woody amylene; (Z,E)-phytol; [(4E,4Z)-5-methoxy-3-methyl-4-penten-1-yl] benzene; 1-(2-methyl allyl oxy)-2-methyl butane; 1-(2-methyl allyl oxy)-3-methoxy-3-methyl-1-butane; 1-(octahydro-4,7-methanoinden-5-yl)-propan-2-ol; 1-(octahydro-4,7-methanoinden-5-yl)-propan-2-one + 1-(6-methyloctahydro-4,7-methanoinden-5-yl)-ethanone; 1,3,3,5-tetramethyl-7 and 8-cyanobicyclo(2.2.2)oct-5-ene; 1,4-diisopropyl-6,8-dioxabicyclo(3.2.1)octane; 1,8-octane dinitrile; 1-[(3aS,7aR)-3a,4,7,7a-tetrahydro-1H-inden-6-yl]ethanone; 10-epi-gamma-eudesmol; 10-undecen-1-ol; 1-acetyl cyclohexyl acetate; 1-acetyl indole; 1-isobutyl-2-methyl butyl 2-butenoate; 1-octen-3-yl butyrate; 1-phenethyl mercaptan; 1-phenyl propyl alcohol; 1-phenyl propyl butyrate; 1-undecanol; 2-(2-cyanoethylidene)-3-methyl bicyclo(2.2.1)hept-5-ene; 2,2'-oxamidobis[ethyl-3-(3,5-di-tert-butyl-4-hydroxyphenyl)-propionate; 2,4,6-trimethyl-6-(3-methyl-2-buten-1-yl)-2-cyclohexen-1-one; 2,4-difurfuryl furan; 2',4'-dimethyl acetophenone; 2,4-dimethyl cyclohexyl methyl acetate; 2,4-dimethyl-3-cyclohexene-1-methanol; 2,4-dimethyl-3-cyclohexene-1-methanyl acetate; 2,4-dimethyl-alpha-allyl-3-cyclohexene methanol; 2,4-ivy carbaldehyde; 2,5,6-trimethyl-2-heptanol; 2,5-dimethyl-2-indan methanol; 2,6-dimethyl-2,18-nonadecadien-8-one; 2-acetyl-3-methyl thiophene; 2-butyl thiophene; 2-decalinyl acetate; 2-decalinyl formate; 2-decanone; 2-decenal; 2-dodecanone; 2-ethyl butyl 2-butenoate; 2-ethyl octine carbonate; 2-ethyl-1-hexanol; 2-ethyl-4-methyl-1,3-oxathiane; 2-ethylidene-6-methyl-cis-3-heptenal; 2-heptanol; 2-heptyl cyclopropane carboxylic acid; 2-heptyl pyridine; 2-heptyl tetrahydrofuran; 2-hexyl thiophene; 2-hexyl-4-acetoxytetrahydrofuran; 2-isopropoxyethyl salicylate; 2-methyl butyl angelate; 2-methyl butyl salicylate; 2-methyl naphthalene; 2-methyl octanal; 2-methyl-4-phenyl butanal; 2-methyl-4-phenyl-1,3-dioxolane; 2-naphthyl phenyl ketone; 2-nonanone oxime; 2-nonanone propylene glycol acetal; 2-octanone oxime; 2-para-cresyl ethanol; 2-para-cresyl oxyethyl acetate; 2-pentyl cyclopentan-1-ol; 2-phenyl propionaldehyde dimethyl acetal; 2-phenyl propionaldehyde ethylene glycol acetal; 2-phenyl propyl isobutyrate; 2-phenyl-2-pentenal; 2-phenyl-4-pentenal; 2-tetradecenal; 2-undecanone; 2-undecen-1-ol; 3-(3-propen-2-yl phenyl) butanal; 3-(3-tert-butyl phenyl) propanal; 3-(4-hydroxy-4-methyl cyclohexyl) butanal; 3-(4-isobutyl-2-methylphenyl)propanal; 3-(4-propyl phenyl) propionaldehyde; 3-(5-methyl-2-furyl) butanal; 3-(benzyl oxy)-2,2-dimethyl propanal; 3',4'-dimethoxyacetophenone; 3,4-dimethoxystyrene; 3,5,5-trimethyl hexanol; 3,6-dimethyl-3-octyl acetate; 3,6-dimethylheptan-2-ol; 3,7-dimethyl-6-octenoic acid; 3-[(4R)-4-(propan-2-yl)cyclohex-1-en-1-yl]propanal; 3-allylthio-2,6-dimethyl-4-heptanone; 3-crotylthio-2,6-dimethyl-4-heptanone; 3-cyclohexyl propyl acetate; 3-decanol; 3-decanone; 3-heptyl acetate; 3-isopropenyl-6-oxoheptanoic acid; 3-mercaptohexyl acetate; 3-methyl pentyl angelate; 3-methyl-2-hexen-1-ol 1-acetate; 3-methyl-4-(2-methyl butyl oxy) butyraldehyde; 3-methyl-4-hexyl oxybutyraldehyde; 3-methyl-6-ethyl-5-octen-1-ol; 3-nonanon-1-yl acetate; 3-pentyl bicyclo[3.2.1]octan-2-one; 3-phenyl propyl formate; 3-phenyl propyl propionate; 3-propyl bicyclo(3.2.1)-6-octen-2-one; 4-(1,1,2,3,3-pentamethyl indan-5-yl) pyrimidine; 4-(2,3-dihydro-1,1,2,3,3-pentamethyl-1H-inden-5-yl)pyrimidine; 4-(para-tolyl)-2-butanone; 4,6,11-trimethyl-5-oxatricyclo(6.2.2.0*4,9*)dodec-6-ene; 4,6-dimethyl-alpha-allyl-3-cyclohexene methanol; 4,8-dimethyl-7-nonen-2-ol; 4-cyclohexyl cyclohexanone; 4-cyclopentyl pentane nitrile; 4-dimethyl ionone; 4-ethoxy-3-anisaldehyde; 4-ethyl cyclohexane propanal; 4-hydroxyphenethyl alcohol; 4-isobutyl cyclohexane propanal; 4-mercapto-4-methyl-2-hexanone; 4-mercapto-4-methyl-2-pentanol; 4-methoxy-alpha-toluene thiol; 4-methyl biphenyl; 4-methyl cyclohexane propanal; 4-methyl-2-(2-methyl-1-propenyl)-1,3-dioxane; 4-methyl-3-pentyl-2(5H)-furanone; 4-methyl-4-phenyl pentanone; 4-methyl-4-phenyl-2-pentanol; 4-methyl-8-vinyl-4,7-nonadienal; 4-methyl-8-vinyl-4,8-nonadienal; 4-phenyl-2-butanol; 4-phenyl-3-buten-2-ol; 4-propyl cyclohexane propanal; 4-tert-butyl cyclohexane carboxaldehyde; 5-tricyclodecenyl acetate; 6,7,8-decen-1-ol; 6,8-dimethyl-2-nonanol; 6-methyl coumarin; 7-allyl benzo[b][1,4]dioxepin-3-one; 8,9-dehydrotheaspirone; 8-methylnonanal; 9-[9,12-epoxy-ethyl]-5-methyl-tricyclo[6.2.1.0.sup.2,7 ]undec-4-ene; 9-decenal; abrialis lavandin oil; acetal 318; acetaldehyde dibutyl acetal; acetaldehyde ethyl phenethyl acetal; acetyl tetralin; achillea millefolium herb oil; adoxal; alloocimene; allyl acetone; allyl anthranilate; allyl benzoate; alpha-acorenol; alpha-allyl-4,6-dimethyl-3-cyclohexene-1-methanyl acetate; alpha-allyl-4-methyl-3-cyclohexene-1-methanyl acetate; alpha-amyl cinnamaldehyde; alpha-amyl cinnamaldehyde / methyl anthranilate schiffs base; alpha-amyl cinnamaldehyde diethyl acetal; alpha-amyl cinnamyl acetate; alpha-amyl cinnamyl alcohol; alpha-bisabolene; alpha-bisabolol; alpha-butyl cinnamaldehyde; alpha-damascone; alpha-decalactone; alpha-hexyl cinnamaldehyde; alpha-ionol; alpha-ionone; alpha-ionone; alpha-ionyl acetate; alpha-irone; alpha-isomethyl ionone; alpha-methyl cinnamyl alcohol; alpha-methyl ionone; alpha-ocimene; alpha-terpineol; amber dioxane; amyl angelate; amyl benzoate; amyl cyclopentanone propanone; amyl isoeugenol; amyl nonanoate; amyl salicylate; aniba rosaeodora var. amazonica wood extract; anise indene; anisyl propanal / methyl anthranilate schiff's base; anthocephalus cadamba oil; atlas cedarwood oil fractions; autumn carboxylate; benzaldehyde / methyl anthranilate schiff's base; benzyl acetate; benzyl acetoacetate; benzyl acetone; benzyl alcohol; benzyl anthranilate; benzyl cinnamate; benzyl eugenol; benzyl formate; benzyl lactate; benzyl methyl ether; benzyl phenyl acetate; benzyl phenyl carbinol; benzyl pivalate; benzyl propionate; benzyl valerate; benzylidene acetone; bergamot acetoacetate; bergamot oil; berry hexanoate; beta-bisabolol; beta-bourbonene; beta-damascone; beta-ionol; beta-ionone; beta-isomethyl ionone; beta-methyl ionone; beta-naphthyl anthranilate; beta-naphthyl ethyl ether; beta-naphthyl methyl ether; beta-phenoxyethyl acetate; bitter orange peel oil ; bois de rose oil ; brachyleana hutchinsii wood oil; butyl 2-naphthyl ether; butyl anthranilate; butyl benzyl ether; butyl para-cresol ether; butyl tiglate; butyl undecylenate; cabreuva wood oil; calendula officinalis flower oil ; callitris intratropica wood oil ; callitropsis araucarioides wood oil; camellia oleifera leaf extract; cananga fruit oil; cananga leaf oil; cananga odorata flower extract; cananga oil; cardamom seed oil ; carrot seed oil ; cedarwood oil ; cedarwood oil terpenes; cerastium candidissimum corr. oil ; cestrum nocturnum flower oil; chamomile isobutyrate; chamomile propionate; chamomile valerate; chrysanthemum ketone; chrysanthemum oxide; cinnamyl acetate; cinnamyl cinnamate; cinnamyl formate; cis-muguet shiseol; cis-woody acetate; citral dimethyl acetal; citral ethylene glycol acetal; citronellal; citronellol; citronellone; citronellyl (R)-lactate; citronellyl acetate; citronellyl acetone; citronellyl butyrate; citronellyl ethyl ether; citronellyl hexanoate; citronellyl isobutyrate; citronellyl nitrile; citronellyl oxyacetaldehyde; citronellyl propionate; citronitrile; citrus ocimenol; citrus reticulata blanco var. mandarin oil ; clary propionate; clary propyl acetate; convolvulus scoparius wood oil; coranol; coriander oleoresin; coriander seed oil; corps popinal; cumin carbinol; cuminyl acetaldehyde; cyclamen aldehyde; cyclamen homoaldehyde; cyclamen propanal; cyclohexyl benzoate; cyclohexyl butyrate; cyclohexyl crotonate; cyclohexyl ethyl alcohol; cyclohexyl propanol; cyclohexyl salicylate; cymbopogon validus leaf oil; damascate; daniellia oliveri bark oil ; decanal ; decanal methyl enol ether; decanal propylene glycol acetal; decanol; decyl anthranilate; dehydrodihydroionone; dextro-linalool; dianthus ethone; diethyl acetonyl diisopropyl acetonyl sulfide; dihexyl (E)-fumarate; dihydro-alpha-ionone; dihydro-beta-ionol; dihydrocarvyl acetate; dihydrodamascone; dihydrofarnesol; dihydrogeranyl linalool; dihydroisojasmonate methyl ester; dihydromyrcenol; dihydroterpineol; diisobutyl carbinyl acetate; dimethyl alpha-ionone; dimethyl benzyl carbinol; dimethyl benzyl carbinyl acetate; dimethyl benzyl carbinyl butyrate; dimethyl glutarate; dimethyl succinate; diphenyl amine; dodecanal (aldehyde C-12 lauric); dryopteris filix-mas oleoresin; earthy acetal; earthy indane; epoxylinalyl acetate; eriocephalus punctulatus flower oil; eschweilera coriacea mori flower oil; ethyl (E)-cinnamate; ethyl 2-benzyl butyrate; ethyl 2-furoate; ethyl 3,5,5-trimethyl hexanoate; ethyl 3-hydroxyoctanoate; ethyl 4-methyl salicylate; ethyl anthranilate; ethyl cyclohexyl acetate; ethyl laurate; ethyl levulinate; ethyl linalyl acetal; ethyl linalyl acetate; ethyl linalyl ether; ethyl methyl-para-tolyl glycidate; ethyl ortho-anisate; ethyl para-methyl-beta-phenyl glycidate; ethyl phenoxyacetate; ethyl phenyl acetate; ethyl salicylate; ethylene brassylate; ethylene glycol diacetate; eucalyptus citriodora oil; eugenyl acetate; exo-isocitral; farnesal; farnesol; farnesyl acetate; farnesyl acetone; farnesyl palmitoleate; fokienol; freesia heptanol; fruity carboxylate; furfuryl isopropyl sulfide; gamma-damascone; gamma-ionone; gardenia concrete; georgywood; geraniol; geranium dihydropyran; geranium leaf oil; geranium oil; geranyl acetate; geranyl acetone; geranyl heptanoate; geranyl isobutyrate; geranyl linalool; geranyl phenyl acetate; geranyl propionate; geranyl undecylenate; ginsene; globulol; glycoacetal; grapefruit pentanol; guaiacyl phenyl acetate; hawthorn acetate; hawthorn carbinol; hawthorn ethanol; helichrysum bracteatum flower oil; heliotropyl acetate; heliotropyl diethyl acetal; heptyl butyrate; heptyl formate; heptyl isobutyrate; heptyl nonanoate; herbal pyran; herniaria incana lam. oil ; hexadecanol; hexanal diethyl acetal; hexen-1-yl oxypropane nitrile; hexenyl cyclopentanone; hexoxyacetaldehyde dimethyl acetal; hexyl (Z)-tiglate; hexyl 2-furoate; hexyl lactate; hexyl oxyacetonitrile; hexyl pivalate; hibiscus sabdariffa flower extract; ho leaf oil; ho wood oil; homalomena rubescens root oil; homoambrinol; homoanisaldehyde; homomenthyl acetate; homovanillin; honey extract; hyacinth acetals; hyacinth ether; hydroxycitronellal; hydroxycitronellal / methyl anthranilate schiffs base; hydroxycitronellal diethyl acetal; hydroxycitronellal propylene glycol acetal; hydroxycitronellal residue; hydroxycitronellol; ilex paraguariensis oleoresin; immortelle flower oil; immortelle flower resinoid; indocolore; indole; intreleven aldehyde; inula root oil; ionone mixed isomers; iris germanica florentina root extract; isoamyl 3-(2-furan) propionate; isoamyl angelate; isoamyl cinnamate; isoamyl nonanoate; isoamyl salicylate; isoamyl undecylenate; isobutyl anthranilate; isobutyl benzyl carbinol; isobutyl citral; isobutyl furyl propionate; isobutyl octanoate; isobutyl phenyl acetate; isobutyl salicylate; isobutyraldehyde; isocyclodimethyl octanol; isocyclogeraniol; isodecanal; isoeugenol; isoeugenyl acetate; isoeugenyl ethyl acetal; isoeugenyl phenyl acetate; isogreen methanoindene; isojasmone; isojasmone; isophytol; isopropyl anthranilate; isopropyl benzoate; ivy carbaldehyde / methyl anthranilate schiff's base; ivy dioxolane; jambu flower oil brazil; jasimia; jasmin acetate; jasmin cyclopentanol; jasmin lactone ; jasmin oil; jasmin pyranol; jasminum grandiflorum extract; jasminum grandiflorum flower extract; jasmopyrane ; ketaki flower oil; kunzea ericoides leaf oil; laevo-citronellol; laevo-linalool; laevo-menthyl acetate; laevo-menthyl propionate; laevo-rose oxide; lavandin concrete; lavandin flower water; lavandin oil; lavandula angustifolia flower oil; lavender concrete; lavender oil; lavender oil bulgaria; lavender oil france; lavender oil greece; lavender oil terpeneless; lavender stem oil lithuania; lawsonia inermis flower oil; lecythis usitata miers. var. paraensis (ducke) r. kunth. flower oil; leerall; leerall / methyl anthranilate schiffs base; lepidine; leptospermum scoparium branch/leaf oil; lily propanol; lilyall; lime octadienal; lime oil ;linaloe wood oil; linalool; linalool oxide; linalyl acetate; linalyl acetate terpenes; linalyl benzoate; lonicera japonica flower extract; mace oleoresin; magnolia cyclohexanol; magnolia decadienal; magnolia flower oil ; magnolia indene; malpighia glabra fruit oil; mandarin oil; mandarin oil; marigold pot flower oil; marine decadienal; matricaria chamomilla flower oil ; melaleuca ericifolia leaf oil; melozol acetate; menthadienyl formate; meta-cresyl acetate; meta-cresyl phenyl acetate; meta-cresyl phenyl ether; methoxycitronellal; methoxymelonal; methyl (Z)-3-hexenoate; methyl (Z)-jasmonate; methyl 10-undecenoate; methyl 2-methyl benzoate; methyl 2-undecynoate; methyl 4-methyl benzoate; methyl 4-phenyl butyrate; methyl benzoate; methyl benzyl acetate; methyl bicycloheptenyl methyloxirane carboxylate; methyl citronellate; methyl cyclocitrone; methyl cyclogeranate; methyl decanoate; methyl dihydrojasmonate; methyl heptanoate; methyl hydrocinnamate; methyl ionyl acetate; methyl isobutyrate; methyl nerate; methyl octine carbonate; methyl ortho-anisate; methyl phenoxyethanol; methyl phenyl acetate; methyl trans-3-oxo-2-pentyl cyclopentane carboxylate; methyl undecylenate; mimosa heptanal; mimosa tenuiflora leaf extract; muguet butanal; muguet butanol; muguet carbaldehyde; muguet carboxaldehyde; muguet dienal; muguet ethanol; muguet nitrile; muguet octadienol; muguet propanol; muguet shiseol; muguet undecadienal; musk acetate; musk dec-8-enone; myrascone; myrcenol; myrcenyl acetate; myristica fragrans fruit extract; myrtle oil; myrtle oil morocco; narcissus acetate; narcissus jonquilla extract; neroli extract; neroli ketone; neroli oil; neroli oil bigarde; nerolidol; nerolidol oxide; nerolidyl propionate; nerolione; neryl acetate; neryl benzoate; neryl isovalerate; neryl propionate; nonanal / methyl anthranilate schiff's base; nonanal diethyl acetal; nonanal dimethyl acetal; nonanol; nonisyl propionate; nonyl octanoate; ocean carboxaldehyde; ocean propanal / methyl anthranilate schiffs base; ocimen-1-yl acetate; octahydro-4,7-methano-1H-indene-5-acetaldehyde; octahydro-4,7-methano-1H-indene-5-acetaldehyde + 6-methyl-octahydro-4,7-methano-indene-5-carbaldehyde; octanal / methyl anthranilate schiffs base; octanal dimethyl acetal; octyl isovalerate; octyl oxyacetaldehyde; octyl propionate; O-ethyl S-(2-furyl methyl) thiocarbonate; origanum majorana leaf oil; orris butenone; orris pyridine; orris rhizome concrete butter (iris pallida); orris rhizome oil (iris germanica); orris rhizome oil; orris rhizome resinoid (iris pallida); ortho-acetyl-para-cresol; ortho-cresyl acetate; ortho-methoxybenzyl ethyl ether; ortho-methyl anisole; palmarosa oil; papaya isobutyrate; para-anisaldehyde; para-anisaldehyde dimethyl acetal; para-anisyl alcohol; para-anisyl butyrate; para-anisyl formate; para-anisyl nitrile; para-cresyl alcohol; para-cresyl isobutyrate; para-cresyl laurate; para-cresyl phenyl ether; para-ethyl acetophenone; para-jasmone; para-methoxy-alpha-methyl cinnamaldehyde; para-methoxycinnamaldehyde; para-methyl benzyl acetate; para-methyl hydratropaldehyde; para-methyl phenoxyacetaldehyde; para-tolualdehyde propylene glycol acetal; peach nitrile; peach pivalate; pentenyl cyclopentanone; peony acetonitrile; peony alcohol; perilla alcohol; perillyl acetate; persea americana fruit oil; petal pyranone; petitgrain bigarade oil; petitgrain cedrat oil; petitgrain lemon oil; petitgrain mandarin oil; petitgrain oil; petitgrain oil fractions; petitgrain oil ; petitgrain oil terpeneless; petitgrain oil terpenes; petitgrain tangerine oil; phenethyl acetate; phenethyl alcohol; phenethyl anthranilate; phenethyl benzoate; phenethyl butyrate; phenethyl heptanoate; phenethyl hexanoate; phenethyl isobutyrate; phenethyl isovalerate; phenethyl phenyl acetate; phenethyl propionate; phenethyl salicylate; phenoxyacetaldehyde ; phenoxyethyl isobutyrate; phenyl acetaldehyde; phenyl acetaldehyde / methyl anthranilate schiffs base; phenyl acetaldehyde 2,3-butylene glycol acetal; phenyl acetaldehyde dicitronellyl acetal; phenyl acetaldehyde digeranyl acetal; phenyl acetaldehyde diisoamyl acetal; phenyl acetaldehyde diisobutyl acetal; phenyl acetaldehyde dimethyl acetal; phenyl acetaldehyde; phenyl acetic acid; phenyl amyl alcohol; phenyl butyrate; phenyl ethylidene acetone; phenyl propyl valerate; phytol; phytyl acetate; piperidine; plum crotonate; plum damascone; prenyl salicylate; propiophenone; propyl phenyl acetate; propyl pyruvate; pseudoionone; raspberry ketone; rhodinol; rhodinyl acetate substitutes; rhodinyl benzoate; rhodinyl formate; rhodinyl phenyl acetate; rhubarb oxirane; rhubarb pyran; rosa alba flower oil; rosa damascena flower oil; rosa odorata flower oil; rose acetate; rose butanoate; rose oil (rosa centifolia) ; rose undecene; S-(2,5-dimethyl-3-furyl) ethane thioate; S-(methyl thio) hexanoate; safrole; salicynile; sambucus nigra flower extract; sambucus nigra flower oil ; sandal cyclopropane; sandal octanol; santalyl acetate; santalyl phenyl acetate; santolina oil; sec-butyl ethyl ether; siam wood oil; spicy acetoacetate; spicy carbonate; spike lavender oil; star fruit oil; styralyl butyrate; styralyl formate; styralyl propionate; styralyl valerate; sunflower oil; surfleurs d'oranger oil; sweet petitgrain oil; tagete oil madagascar; tangerine oil terpeneless; tarchonanthus camphoratus oil; terpineol acetate; terpineols; terpinyl benzoate; terpinyl butyrate; terpinyl formate; terpinyl isobutyrate; terpinyl valerate; tetrahydrofurfuryl phenyl acetate; tetrahydroionol; tetrahydroionyl acetate; tetrahydrolinalool; tetrahydrolinalyl acetate; tetrahydromyrcenol; tilia cordata flower oil; tonka ketone; tropical ionone; tuberose oil; undecanal; undecanal dimethyl acetal; undecanal propylene glycol acetal; vanilla tahitensis fruit oil; vassoura oil; verdyl acetate; vetiver pentanone; violet dienyne; violet methyl carbonate; violet nitrile; violet propanol; vitispirane; white frangipani concrete; white nelumbo nucifera flower concrete; white sassafras oil; witch hazel leaf oil; yarrow oil; ylang ylang flower concrete; ylang ylang flower oil; yuzu leaf oil; yuzunone and zdravetz oil, and
   - at least one surfactant.

It is preferred if the total quantity of the ingredients of list A is in the range from 0.0001 to 2.0% by weight, more preferably from 0.001 to 0.5% by weight and most preferably from 0.002 to 0.3% by weight, calculated to the total weight of the composition.

It is also preferred if the composition comprises at least (Z) alpha-Damascone, (Z) beta-Damascone, Aldehyd C14, Citronellol, Hydroxycitronellal, dextro-Limonene, Aldehyd C8, Aldehyd C-10, Linalool, Linalyl Acetate, Methyl Anthranilate, Benzyl Acetate, Benzyl Alcohol, Benzyl Cinnamate, Geraniol, Geranyl Acetate, Vanillin, Ethyl Vanillin, Vanillin Isobutyrate, Eugenyl Acetate, Isoeugenyl Acetate, Patchouli Ethanone, Phenethyl alcohol, Amyl Salicylate, alpha lonone, (E) beta lonone, Heliotropin, Dihydromyrcenol, alpha-Terpineol, Sandal Pentanol, Sandal Pentenol, Sandal Octanol, Melozol Acetate, Phenoxyethyl Isobutyrate, gamma-Decalactone, gamma-Nonalactone, (Aldehyde C18), gamma-Octalactone, delta Nonalactone, Dihydrocoumarin, Nerolidol, Isoeugenol, Cinnamyl Acetate, Farnesol, alpha-Amyl Cinnamaldehyde, alpha-Isomethyl lonone, Indole, Z-Jasmone, Dihydrojasmone, Dihydrojasmone Lactone, Methyldihydrojasmonate, Musk Tetralin, Ethylene Brassylate, Tetradecanal, Musk Amberol, Acetyl Cedrene, Sclareol, Ylang-Ylang Flower Oil, White Jasmin Flower Oil, Blood Orange Oil and/or Bitter Orange Peel Oil.

Further, it is preferred if the total quantity of (Z) alpha-Damascone, (Z) beta-Damascone, Aldehyd C14, Citronellol, Hydroxycitronellal, dextro-Limonene, Aldehyd C8, Aldehyd C-10, Linalool, Linalyl Acetate, Methyl Anthranilate, Benzyl Acetate, Benzyl Alcohol, Benzyl Cinnamate, Geraniol, Geranyl Acetate, Vanillin, Ethyl Vanillin, Vanillin Isobutyrate, Eugenyl Acetate, Isoeugenyl Acetate, Patchouli Ethanone, Phenethyl alcohol, Amyl Salicylate, alpha lonone, (E) beta lonone, Heliotropin, Dihydromyrcenol, alpha-Terpineol, Sandal Pentanol, Sandal Pentenol, Sandal Octanol, Melozol Acetate, Phenoxyethyl Isobutyrate, gamma-Decalactone, gamma-Nonalactone, (Aldehyde C18), gamma-Octalactone, delta Nonalactone, Dihydrocoumarin, Nerolidol, Isoeugenol, Cinnamyl Acetate, Farnesol, alpha-Amyl Cinnamaldehyde, alpha-Isomethyl lonone, Indole, Z-Jasmone, Dihydrojasmone, Dihydrojasmone Lactone, Methyldihydrojasmonate, Musk Tetralin, Ethylene Brassylate, Tetradecanal, Musk Amberol, Acetyl Cedrene, Sclareol, Ylang-Ylang Flower Oil, White Jasmin Flower Oil, Blood Orange Oil and/or Bitter Orange Peel Oil is in the range from 0.0001 to 2.0% by weight, more preferably from 0.001 to 0.5% by weight and most preferably from 0.002 to 0.3% by weight calculated to the total weight of the composition.

In another aspect of the present invention, it is preferred if the composition comprises a fragrance having a scent of coconut. Fragrances leading to a coconut scent can preferably be created by containing in the composition one or more ingredients select from the group consisting of (-)-alpha-bisabolol; (-)-cis-whiskey lactone; (R)-gamma-decalactone; (R)-gamma-hexalactone; (R)-gamma-nonalactone; (R)-gamma-octalactone; (R)-massoia lactone; (R)-tonka furanone; (S)-gamma-decalactone; (S)-gamma-heptalactone; (S)-gamma-hexalactone; (S)-gamma-nonalactone; (S)-gamma-octalactone; (Z)-gamma-jasmolactone; (Z+E)-2-methyl cyclohexanol; 1-dodecanol; 2-acetyl-5-methyl furan; 2-heptanone; 2-pentyl cyclopentanone; 2-pentyl-2-cyclohexen-1-one; 2-tridecanone; 3-butyl bicyclo[3.2.1]octan-2-one; 4-hydroxybenzyl alcohol; 4-octen-3-one; 5,5-dibutyl dihydrofuran-2(3H)-one; 6-amyl-alpha-pyrone; 6-methyl coumarin; 7-methyl coumarin; alpha-decalactone; amyl heptanoate; cis-oak lactone; coconut decanone methyl; coconut naphthalenone; cocos nucifera fruit oil ; costus valerolactone; delta-2-dodecenolactone; delta-decalactone; delta-dodecalactone; delta-heptalactone; delta-hexalactone; delta-nonalactone; delta-octalactone; delta-undecalactone; dihydrocoumarin; dihydrojasmone lactone; dimethyl benzofuranone; ethyl undecanoate; gamma-decalactone; gamma-heptalactone; gamma-hexalactone; gamma-nonalactone (aldehyde C-18 (so-called)); gamma-octalactone; gamma-undecalactone (aldehyde C-14 (so-called)); glyceryl 5-hydroxydecanoate; glyceryl 5-hydroxydodecanoate; heliotropin; hexahydrocoumarin; hexenyl cyclopentanone; isoamyl octanoate; isopropyl 2-methyl butyrate; isopropyl octanoate; massoia bark oil; massoia lactone; methyl heptadienone; methyl laurate; octyl octanoate; para-vanillyl alcohol; petal pyranone; phthalide; propyl octanoate; tetrahydrojasmone; tonka furanone; trans-oak lactone; undecanoic acid; whiskey lactone and wine lactone.

Thus, a preferred embodiment of the invention is a cosmetic product comprising
a) a container comprising at least 80% by weight post-consumer recycled plastic, based on the total weight of the container, whereby the container has at least one opening,
b) a cosmetic cleansing composition situated in the container characterized in that the composition comprises
   - at least one ingredient selected from the group consisting of (-)-alpha-bisabolol; (-)-cis-whiskey lactone; (R)-gamma-decalactone; (R)-gamma-hexalactone; (R)-gamma-nonalactone; (R)-gamma-octalactone; (R)-massoia lactone; (R)-tonka furanone; (S)-gamma-decalactone; (S)-gamma-heptalactone; (S)-gamma-hexalactone; (S)-gamma-nonalactone; (S)-gamma-octalactone; (Z)-gamma-jasmolactone; (Z+E)-2-methyl cyclohexanol; 1-dodecanol; 2-acetyl-5-methyl furan; 2-heptanone; 2-pentyl cyclopentanone; 2-pentyl-2-cyclohexen-1-one; 2-tridecanone; 3-butyl bicyclo[3.2.1]octan-2-one; 4-hydroxybenzyl alcohol; 4-octen-3-one; 5,5-dibutyl dihydrofuran-2(3H)-one; 6-amyl-alpha-pyrone; 6-methyl coumarin; 7-methyl coumarin; alpha-decalactone; amyl heptanoate; cis-oak lactone; coconut decanone methyl; coconut naphthalenone; cocos nucifera fruit oil ; costus valerolactone; delta-2-dodecenolactone; delta-decalactone; delta-dodecalactone; delta-heptalactone; delta-hexalactone; delta-nonalactone; delta-octalactone; delta-undecalactone; dihydrocoumarin; dihydrojasmone lactone; dimethyl benzofuranone; ethyl undecanoate; gamma-decalactone; gamma-heptalactone; gamma-hexalactone; gamma-nonalactone (aldehyde C-18 (so-called)); gamma-octalactone; gamma-undecalactone (aldehyde C-14 (so-called)); glyceryl 5-hydroxydecanoate; glyceryl 5-hydroxydodecanoate; heliotropin; hexahydrocoumarin; hexenyl cyclopentanone; isoamyl octanoate; isopropyl 2-methyl butyrate; isopropyl octanoate; massoia bark oil; massoia lactone; methyl heptadienone; methyl laurate; octyl octanoate; para-vanillyl alcohol; petal pyranone; phthalide; propyl octanoate; tetrahydrojasmone; tonka furanone; trans-oak lactone; undecanoic acid; whiskey lactone and wine lactone, and
   - at least one surfactant.

It is preferred if the total quantity of the ingredients of (-)-alpha-bisabolol; (-)-cis-whiskey lactone; (R)-gamma-decalactone; (R)-gamma-hexalactone; (R)-gamma-nonalactone; (R)-gamma-octalactone; (R)-massoia lactone; (R)-tonka furanone; (S)-gamma-decalactone; (S)-gamma-heptalactone; (S)-gamma-hexalactone; (S)-gamma-nonalactone; (S)-gamma-octalactone; (Z)-gamma-jasmolactone; (Z+E)-2-methyl cyclohexanol; 1-dodecanol; 2-acetyl-5-methyl furan; 2-heptanone; 2-pentyl cyclopentanone; 2-pentyl-2-cyclohexen-1-one; 2-tridecanone; 3-butyl bicyclo[3.2.1]octan-2-one; 4-hydroxybenzyl alcohol; 4-octen-3-one; 5,5-dibutyl dihydrofuran-2(3H)-one; 6-amyl-alpha-pyrone; 6-methyl coumarin; 7-methyl coumarin; alpha-decalactone; amyl heptanoate; cis-oak lactone; coconut decanone methyl; coconut naphthalenone; cocos nucifera fruit oil ; costus valerolactone; delta-2-dodecenolactone; delta-decalactone; delta-dodecalactone; delta-heptalactone; delta-hexalactone; delta-nonalactone; delta-octalactone; delta-undecalactone; dihydrocoumarin; dihydrojasmone lactone; dimethyl benzofuranone; ethyl undecanoate; gamma-decalactone; gamma-heptalactone; gamma-hexalactone; gamma-nonalactone (aldehyde C-18 (so-called)); gamma-octalactone; gamma-undecalactone (aldehyde C-14 (so-called)); glyceryl 5-hydroxydecanoate; glyceryl 5-hydroxydodecanoate; heliotropin; hexahydrocoumarin; hexenyl cyclopentanone; isoamyl octanoate; isopropyl 2-methyl butyrate; isopropyl octanoate; massoia bark oil; massoia lactone; methyl heptadienone; methyl laurate; octyl octanoate; para-vanillyl alcohol; petal pyranone; phthalide; propyl octanoate; tetrahydrojasmone; tonka furanone; trans-oak lactone; undecanoic acid; whiskey lactone and wine lactone is in the range from 0.0001 to 2.0% by weight, more preferably from 0.001 to 0.5% by weight and most preferably from 0.002 to 0.3% by weight, calculated to the total weight of the composition.

In another aspect of the present invention, it is preferred if the composition comprises a fragrance having a scent of orange. Fragrances leading to an orange scent can preferably be created by containing in the composition one or more ingredients select from the group consisting of (E)-2-dodecenal; (E)-2-pentenal; (E)-2-undecenal; (E)-4-decenal; (E,E)-2,4-dodecadienal; (E,Z)-2,6-dodecadienal; (Z)-3-hexen-1-yl anthranilate; (Z)-4-decen-1-yl acetate; (Z)-4-decenal; (Z)-4-decenal diethyl acetal; (Z)-8-tetradecenal; 1-formyl-3-isohexenyl-3-cyclohexene; 2,4-decadienal; 2,4-dimethyl-alpha-isopropyl-3-cyclohexene-1-methanol; 2-decanone; 2-decenal; 2-dodecanone; 2-dodecenal; 2-naphthyl phenyl ketone; 2-nonanol; 2-pentenal; 2-pentyl-2-cyclohexen-1-one; 2-undecenal; 3-(methallylthio)-2,6-dimethyl-4-heptanone; 3-decanol; 3-decanone; 3-dodecenal; 3-methyl-4-heptyl oxybutyraldehyde; 4-decenal; 6-methyl heptanal; 6-methyl octanal; abies alba cone oil; acetyl tetralin; alpha-naphthyl methyl ketone; alpha-sinensal; alpha-terpinyl anthranilate; alpha-terpinyl methyl ether; bergamot oil; beta-naphthyl ethyl ether; beta-naphthyl methyl ether; beta-naphthyl methyl ketone; beta-sinensal; bitter orange peel oil; blood orange oil; butyl nonanoate; carica papaya seed oil; citral / methyl anthranilate schiffs base; citral propylene glycol acetal; citrus aurantium amara flower cera; cyclamen aldehyde / methyl anthranilate schiffs base; cyclohexyl anthranilate; decanal (aldehyde C-10); decanal / methyl anthranilate schiffs base; decanal diethyl acetal; decanol; decyl formate; dextro-limonene; dimethyl nonanal; dodecane nitrile; ethyl (E)-2-hexenoate; ethyl anthranilate; grapefruit oil ;heptanal dimethyl acetal; heptyl nonanoate; hydroxycitronellal / methyl anthranilate schiff's base; intreleven aldehyde; isooctanol; ivy carbaldehyde / methyl anthranilate schiffs base; labdanum ethanone; leerall / methyl anthranilate schiff's base; lilyall / methyl anthranilate schiffs base; linalyl anthranilate; mandarin oil; methyl 2-methyl benzoate; methyl anthranilate; methyl anthranilate / hexyl cinnamaldehyde schiffs base; methyl anthranilate / isocyclocitral schiff's base; methyl dimethyl anthranilate; methyl indole-1-carboxylate; methyl octanoate; michelia champaca flower oil; mimusops elengi flower oil; neroli oil; nerolione; neryl butyrate; nonanal (aldehyde C-9); nonanal / methyl anthranilate schiff's base; nonanol; nonyl anthranilate; nonyl benzoate; nonyl heptanoate; nonyl nonanoate; nyctanthes arbor-tristis flower oil; ocean propanal / methyl anthranilate schiffs base; octanal (aldehyde C-8); octanal glycol acetal; octanol; octyl formate; octyl phenyl acetate; phenethyl anthranilate; propyl anthranilate; rue flower oil; satinaldehyde; surfleurs d'oranger oil; sweet orange peel oil; tangerine oil; terpinyl isovalerate; turmeric root oil and valencene.

Thus, a preferred embodiment of the invention is a cosmetic product comprising
a) a container comprising at least 80% by weight post-consumer recycled plastic, based on the total weight of the container, whereby the container has at least one opening,
b) a cosmetic cleansing composition situated in the container characterized in that the composition comprises
   - at least one ingredient selected from the group consisting of (E)-2-dodecenal; (E)-2-pentenal; (E)-2-undecenal; (E)-4-decenal; (E,E)-2,4-dodecadienal; (E,Z)-2,6-dodecadienal; (Z)-3-hexen-1-yl anthranilate; (Z)-4-decen-1-yl acetate; (Z)-4-decenal; (Z)-4-decenal diethyl acetal; (Z)-8-tetradecenal; 1-formyl-3-isohexenyl-3-cyclohexene; 2,4-decadienal; 2,4-dimethyl-alpha-isopropyl-3-cyclohexene-1-methanol; 2-decanone; 2-decenal; 2-dodecanone; 2-dodecenal; 2-naphthyl phenyl ketone; 2-nonanol; 2-pentenal; 2-pentyl-2-cyclohexen-1-one; 2-undecenal; 3-(methallylthio)-2,6-dimethyl-4-heptanone; 3-decanol; 3-decanone; 3-dodecenal; 3-methyl-4-heptyl oxybutyraldehyde; 4-decenal; 6-methyl heptanal; 6-methyl octanal; abies alba cone oil; acetyl tetralin; alpha-naphthyl methyl ketone; alpha-sinensal; alpha-terpinyl anthranilate; alpha-terpinyl methyl ether; bergamot oil; beta-naphthyl ethyl ether; beta-naphthyl methyl ether; beta-naphthyl methyl ketone; beta-sinensal; bitter orange peel oil; blood orange oil; butyl nonanoate; carica papaya seed oil; citral / methyl anthranilate schiff's base; citral propylene glycol acetal; citrus aurantium amara flower cera; cyclamen aldehyde / methyl anthranilate schiff's base; cyclohexyl anthranilate; decanal (aldehyde C-10); decanal / methyl anthranilate schiff's base; decanal diethyl acetal; decanol; decyl formate; dextro-limonene; dimethyl nonanal; dodecane nitrile; ethyl (E)-2-hexenoate; ethyl anthranilate; grapefruit oil ;heptanal dimethyl acetal; heptyl nonanoate; hydroxycitronellal / methyl anthranilate schiffs base; intreleven aldehyde; isooctanol; ivy carbaldehyde / methyl anthranilate schiff's base; labdanum ethanone; leerall / methyl anthranilate schiff's base; lilyall / methyl anthranilate schiffs base; linalyl anthranilate; mandarin oil; methyl 2-methyl benzoate; methyl anthranilate; methyl anthranilate / hexyl cinnamaldehyde schiff's base; methyl anthranilate / isocyclocitral schiff's base; methyl dimethyl anthranilate; methyl indole-1-carboxylate; methyl octanoate; michelia champaca flower oil; mimusops elengi flower oil; neroli oil; nerolione; neryl butyrate; nonanal (aldehyde C-9); nonanal / methyl anthranilate schiffs base; nonanol; nonyl anthranilate; nonyl benzoate; nonyl heptanoate; nonyl nonanoate; nyctanthes arbor-tristis flower oil; ocean propanal / methyl anthranilate schiff's base; octanal (aldehyde C-8); octanal glycol acetal; octanol; octyl formate; octyl phenyl acetate; phenethyl anthranilate; propyl anthranilate; rue flower oil; satinaldehyde; surfleurs d'oranger oil; sweet orange peel oil; tangerine oil; terpinyl isovalerate; turmeric root oil and valencene, and
   - at least one surfactant.

It is preferred if the total quantity of the ingredients of (E)-2-dodecenal; (E)-2-pentenal; (E)-2-undecenal; (E)-4-decenal; (E,E)-2,4-dodecadienal; (E,Z)-2,6-dodecadienal; (Z)-3-hexen-1-yl anthranilate; (Z)-4-decen-1-yl acetate; (Z)-4-decenal; (Z)-4-decenal diethyl acetal; (Z)-8-tetradecenal; 1-formyl-3-isohexenyl-3-cyclohexene; 2,4-decadienal; 2,4-dimethyl-alpha-isopropyl-3-cyclohexene-1-methanol; 2-decanone; 2-decenal; 2-dodecanone; 2-dodecenal; 2-naphthyl phenyl ketone; 2-nonanol; 2-pentenal; 2-pentyl-2-cyclohexen-1-one; 2-undecenal; 3-(methallylthio)-2,6-dimethyl-4-heptanone; 3-decanol; 3-decanone; 3-dodecenal; 3-methyl-4-heptyl oxybutyraldehyde; 4-decenal; 6-methyl heptanal; 6-methyl octanal; abies alba cone oil; acetyl tetralin; alpha-naphthyl methyl ketone; alpha-sinensal; alpha-terpinyl anthranilate; alpha-terpinyl methyl ether; bergamot oil; beta-naphthyl ethyl ether; beta-naphthyl methyl ether; beta-naphthyl methyl ketone; beta-sinensal; bitter orange peel oil; blood orange oil; butyl nonanoate; carica papaya seed oil; citral / methyl anthranilate schiff's base; citral propylene glycol acetal; citrus aurantium amara flower cera; cyclamen aldehyde / methyl anthranilate schiff's base; cyclohexyl anthranilate; decanal (aldehyde C-10); decanal / methyl anthranilate schiffs base; decanal diethyl acetal; decanol; decyl formate; dextro-limonene; dimethyl nonanal; dodecane nitrile; ethyl (E)-2-hexenoate; ethyl anthranilate; grapefruit oil ; heptanal dimethyl acetal; heptyl nonanoate; hydroxycitronellal / methyl anthranilate schiffs base; intreleven aldehyde; isooctanol; ivy carbaldehyde / methyl anthranilate schiff's base; labdanum ethanone; leerall / methyl anthranilate schiff's base; lilyall / methyl anthranilate schiffs base; linalyl anthranilate; mandarin oil; methyl 2-methyl benzoate; methyl anthranilate; methyl anthranilate / hexyl cinnamaldehyde schiffs base; methyl anthranilate / isocyclocitral schiff's base; methyl dimethyl anthranilate; methyl indole-1-carboxylate; methyl octanoate; michelia champaca flower oil; mimusops elengi flower oil; neroli oil; nerolione; neryl butyrate; nonanal (aldehyde C-9); nonanal / methyl anthranilate schiff's base; nonanol; nonyl anthranilate; nonyl benzoate; nonyl heptanoate; nonyl nonanoate; nyctanthes arbor-tristis flower oil; ocean propanal / methyl anthranilate schiffs base; octanal (aldehyde C-8); octanal glycol acetal; octanol; octyl formate; octyl phenyl acetate; phenethyl anthranilate; propyl anthranilate; rue flower oil; satinaldehyde; surfleurs d'oranger oil; sweet orange peel oil; tangerine oil; terpinyl isovalerate; turmeric root oil and valencene is in the range from 0.0001 to 1% by weight, more preferably from 0.001 to 0.5% by weight and most preferably from 0.002 to 0.1% by weight, calculated to the total weight of the composition.

In another aspect of the present invention, it is preferred if the composition comprises a fragrance having a scent of jasmine. Fragrances leading to a jasmine scent can preferably be created by containing in the composition one or more ingredients select from the group consisting of (+)-methyl dihydroepijasmonate; (4E,9Z)-13-methyloxacyclopentadeca-4,9-dien-2-one; (R)-styralyl acetate; (Z)-3-hexen-1-yl anthranilate; (Z)-7-tetradecen-2-one; (Z)-jasmone; (Z,E)-phytol; 1-phenyl propyl butyrate; 2-(4-methyl cyclohexylidene) heptanonitrile; 2-hexenyl cyclopentanyl acetate; 2-hexylidene cyclopentanone; 2-methyl-4-phenyl-1,3-dioxolane; 2-para-cresyl oxyethyl acetate; 2-pentadecanone; 2-pentyl cyclopentan-1-ol; 2-pentyl cyclopentanone; 2-pentyl-2-cyclohexen-1-one; 3-butyl bicyclo[3.2.1]octan-2-one; 3-decen-2-one; 3-nonanone; 4-methyl-3-pentyl oxazolidin-2-one; 6-(5(6)-decenoyl oxy) decanoic acid; alpha-amyl cinnamaldehyde; alpha-amyl cinnamaldehyde diethyl acetal; alpha-amyl cinnamaldehyde dimethyl acetal; alpha-amyl cinnamyl acetate; alpha-amyl cinnamyl alcohol; alpha-butyl cinnamaldehyde; alpha-hexyl cinnamaldehyde; amyl cyclopentanone propanone; amyl cyclopentenone; autumn carboxylate; benzyl acetate; benzyl butyrate; benzyl hexanoate; benzyl isobutyrate; benzyl phenyl acetate; benzyl propionate; berry hexanoate; chrysanthemum oxide; creamy lactone; dicyclopentadiene propionate; dihydroisojasmonate methyl ester; dihydrojasmone; dihydrojasmone lactone; dimethyl benzyl carbinyl acetate; dimethyl benzyl carbinyl formate; dimethyl benzyl carbinyl isobutyrate; dimethyl benzyl carbinyl propionate; fruity cyclopentanone; gardenia oxide; gelsone; hawthorn ethanol; herbal carbonate; herbal pyran; hexahydrofarnesyl acetone; hexenyl cyclopentanone; hexyl (Z)-tiglate; indoletal; isogreen methanoindene; isojasmone; jasmin acetate; jasmin cyclopentanol; jasmin cyclopentanone; jasmin lactone; jasmin oil; jasmin pyranol; jasminum grandiflorum flower extract; jasminum officinale flower extract; jasminum sambac flower extract; jasminum sambac flower oil ; jasmopyrane ; methyl (Z)-jasmonate; methyl bicycloheptenyl methyloxirane carboxylate; methyl dihydrojasmonate; methyl jasmonate; methyl phenoxyethanol; methyl trans-3-oxo-2-pentyl cyclopentane carboxylate; nyctanthes arbor-tristis flower oil; octen-1-yl cyclopentanone; octyl butyrate; para-anisaldehyde dimethyl acetal; para-cresyl caprylate; para-jasmone; peach nitrile; pear valerate; pentenyl cyclopentanone; petal pyranone; prenyl acetate; prenyl tiglate; privet dioxane; pseudojasmone; rosacyanthin; styralyl butyrate; styralyl isobutyrate; styralyl valerate; tetrahydrojasmone; tricyclodecenyl propionate; waxy lactone; white jasmin flower oil and ylang ylang flower oil.

Thus, a preferred embodiment of the invention is a cosmetic product comprising
a) a container comprising at least 80% by weight post-consumer recycled plastic, based on the total weight of the container, whereby the container has at least one opening,
b) a cosmetic cleansing composition situated in the container characterized in that the composition comprises
   - at least one ingredient selected from the group consisting of (+)-methyl dihydroepijasmonate; (4E,9Z)-13-methyloxacyclopentadeca-4,9-dien-2-one; (R)-styralyl acetate; (Z)-3-hexen-1-yl anthranilate; (Z)-7-tetradecen-2-one; (Z)-jasmone; (Z,E)-phytol; 1-phenyl propyl butyrate; 2-(4-methyl cyclohexylidene) heptanonitrile; 2-hexenyl cyclopentanyl acetate; 2-hexylidene cyclopentanone; 2-methyl-4-phenyl-1,3-dioxolane; 2-para-cresyl oxyethyl acetate; 2-pentadecanone; 2-pentyl cyclopentan-1-ol; 2-pentyl cyclopentanone; 2-pentyl-2-cyclohexen-1-one; 3-butyl bicyclo[3.2.1]octan-2-one; 3-decen-2-one; 3-nonanone; 4-methyl-3-pentyl oxazolidin-2-one; 6-(5(6)-decenoyl oxy) decanoic acid; alpha-amyl cinnamaldehyde; alpha-amyl cinnamaldehyde diethyl acetal; alpha-amyl cinnamaldehyde dimethyl acetal; alpha-amyl cinnamyl acetate; alpha-amyl cinnamyl alcohol; alpha-butyl cinnamaldehyde; alpha-hexyl cinnamaldehyde; amyl cyclopentanone propanone; amyl cyclopentenone; autumn carboxylate; benzyl acetate; benzyl butyrate; benzyl hexanoate; benzyl isobutyrate; benzyl phenyl acetate; benzyl propionate; berry hexanoate; chrysanthemum oxide; creamy lactone; dicyclopentadiene propionate; dihydroisojasmonate methyl ester; dihydrojasmone; dihydrojasmone lactone; dimethyl benzyl carbinyl acetate; dimethyl benzyl carbinyl formate; dimethyl benzyl carbinyl isobutyrate; dimethyl benzyl carbinyl propionate; fruity cyclopentanone; gardenia oxide; gelsone; hawthorn ethanol; herbal carbonate; herbal pyran; hexahydrofarnesyl acetone; hexenyl cyclopentanone; hexyl (Z)-tiglate; indoletal; isogreen methanoindene; isojasmone; jasmin acetate; jasmin cyclopentanol; jasmin cyclopentanone; jasmin lactone; jasmin oil; jasmin pyranol; jasminum grandiflorum flower extract; jasminum officinale flower extract; jasminum sambac flower extract; jasminum sambac flower oil ; jasmopyrane ; methyl (Z)-jasmonate; methyl bicycloheptenyl methyloxirane carboxylate; methyl dihydrojasmonate; methyl jasmonate; methyl phenoxyethanol; methyl trans-3-oxo-2-pentyl cyclopentane carboxylate; nyctanthes arbor-tristis flower oil; octen-1-yl cyclopentanone; octyl butyrate; para-anisaldehyde dimethyl acetal; para-cresyl caprylate; para-jasmone; peach nitrile; pear valerate; pentenyl cyclopentanone; petal pyranone; prenyl acetate; prenyl tiglate; privet dioxane; pseudojasmone; rosacyanthin; styralyl butyrate; styralyl isobutyrate; styralyl valerate; tetrahydrojasmone; tricyclodecenyl propionate; waxy lactone; white jasmin flower oil and ylang ylang flower oil, and
   - at least one surfactant.

It is preferred if the total quantity of the ingredients of (+)-methyl dihydroepijasmonate; (4E,9Z)-13-methyloxacyclopentadeca-4,9-dien-2-one; (R)-styralyl acetate; (Z)-3-hexen-1-yl anthranilate; (Z)-7-tetradecen-2-one; (Z)-jasmone; (Z,E)-phytol; 1-phenyl propyl butyrate; 2-(4-methyl cyclohexylidene) heptanonitrile; 2-hexenyl cyclopentanyl acetate; 2-hexylidene cyclopentanone; 2-methyl-4-phenyl-1,3-dioxolane; 2-para-cresyl oxyethyl acetate; 2-pentadecanone; 2-pentyl cyclopentan-1-ol; 2-pentyl cyclopentanone; 2-pentyl-2-cyclohexen-1-one; 3-butyl bicyclo[3.2.1]octan-2-one; 3-decen-2-one; 3-nonanone; 4-methyl-3-pentyl oxazolidin-2-one; 6-(5(6)-decenoyl oxy) decanoic acid; alpha-amyl cinnamaldehyde; alpha-amyl cinnamaldehyde diethyl acetal; alpha-amyl cinnamaldehyde dimethyl acetal; alpha-amyl cinnamyl acetate; alpha-amyl cinnamyl alcohol; alpha-butyl cinnamaldehyde; alpha-hexyl cinnamaldehyde; amyl cyclopentanone propanone; amyl cyclopentenone; autumn carboxylate; benzyl acetate; benzyl butyrate; benzyl hexanoate; benzyl isobutyrate; benzyl phenyl acetate; benzyl propionate; berry hexanoate; chrysanthemum oxide; creamy lactone; dicyclopentadiene propionate; dihydroisojasmonate methyl ester; dihydrojasmone; dihydrojasmone lactone; dimethyl benzyl carbinyl acetate; dimethyl benzyl carbinyl formate; dimethyl benzyl carbinyl isobutyrate; dimethyl benzyl carbinyl propionate; fruity cyclopentanone; gardenia oxide; gelsone; hawthorn ethanol; herbal carbonate; herbal pyran; hexahydrofarnesyl acetone; hexenyl cyclopentanone; hexyl (Z)-tiglate; indoletal; isogreen methanoindene; isojasmone; jasmin acetate; jasmin cyclopentanol; jasmin cyclopentanone; jasmin lactone; jasmin oil; jasmin pyranol; jasminum grandiflorum flower extract; jasminum officinale flower extract; jasminum sambac flower extract; jasminum sambac flower oil ; jasmopyrane ; methyl (Z)-jasmonate; methyl bicycloheptenyl methyloxirane carboxylate; methyl dihydrojasmonate; methyl jasmonate; methyl phenoxyethanol; methyl trans-3-oxo-2-pentyl cyclopentane carboxylate; nyctanthes arbor-tristis flower oil; octen-1-yl cyclopentanone; octyl butyrate; para-anisaldehyde dimethyl acetal; para-cresyl caprylate; para-jasmone; peach nitrile; pear valerate; pentenyl cyclopentanone; petal pyranone; prenyl acetate; prenyl tiglate; privet dioxane; pseudojasmone; rosacyanthin; styralyl butyrate; styralyl isobutyrate; styralyl valerate; tetrahydrojasmone; tricyclodecenyl propionate; waxy lactone; white jasmin flower oil and ylang ylang flower oil is in the range from 0.0001 to 1% by weight, more preferably from 0.0001 to 2.0% by weight, more preferably from 0.001 to 0.5% by weight and most preferably from 0.002 to 0.3% by weight, calculated to the total weight of the composition.

In another aspect of the present invention, it is preferred if the composition comprises a fragrance having a scent of sandalwood. Fragrances leading to a sandalwood scent can preferably be created by containing in the composition one or more ingredients select from the group consisting of (+)-alpha-santalyl acetate; (R)-sandal hexanol; 2-decalinyl formate; acetoxymethyl isolongifolene; agarwood oil; alpha-cedrene epoxide; alpha-ethyl-2,2,6-trimethyl cyclohexane propanol; alpha-santalol; amyris bark oil; beta,2,2,3-tetramethyl-delta-methylene-3-cyclopentene-1-butanol; brachyleana hutchinsii wood oil; cedarwood oil ; geranium nitrile; indisan; melozol acetate; methyl sandal; muscomer; sandal butenol; sandal cyclohexanol; sandal cyclopentane; sandal cyclopropane; sandal glycol acetal; sandal hexanol; sandal octanol; sandal pentanol; sandal pentenol; sandal pentenone; sandalrome; sandalwood oil acetylated; sandel; santall; santalol; santol pentenol; timber propanol; vetiverol; vetiveryl formate and woody epoxide.

Thus, a preferred embodiment of the invention is a cosmetic product comprising
a) a container comprising at least 80% by weight post-consumer recycled plastic, based on the total weight of the container, whereby the container has at least one opening,
b) a cosmetic cleansing composition situated in the container characterized in that the composition comprises
   - at least one ingredient selected from the group consisting of (+)-alpha-santalyl acetate; (R)-sandal hexanol; 2-decalinyl formate; acetoxymethyl isolongifolene; agarwood oil; alpha-cedrene epoxide; alpha-ethyl-2,2,6-trimethyl cyclohexane propanol; alpha-santalol; amyris bark oil; beta,2,2,3-tetramethyl-delta-methylene-3-cyclopentene-1-butanol; brachyleana hutchinsii wood oil; cedarwood oil ; geranium nitrile; indisan; melozol acetate; methyl sandal; muscomer; sandal butenol; sandal cyclohexanol; sandal cyclopentane; sandal cyclopropane; sandal glycol acetal; sandal hexanol; sandal octanol; sandal pentanol; sandal pentenol; sandal pentenone; sandalrome; sandalwood oil acetylated; sandel; santall; santalol; santol pentenol; timber propanol; vetiverol; vetiveryl formate and woody epoxide, and
   - at least one surfactant.

It is preferred if the total quantity of the ingredients of (+)-alpha-santalyl acetate; (R)-sandal hexanol; 2-decalinyl formate; acetoxymethyl isolongifolene; agarwood oil; alpha-cedrene epoxide; alpha-ethyl-2,2,6-trimethyl cyclohexane propanol; alpha-santalol; amyris bark oil; beta,2,2,3-tetramethyl-delta-methylene-3-cyclopentene-1-butanol; brachyleana hutchinsii wood oil; cedarwood oil ; geranium nitrile; indisan; melozol acetate; methyl sandal; muscomer; sandal butenol; sandal cyclohexanol; sandal cyclopentane; sandal cyclopropane; sandal glycol acetal; sandal hexanol; sandal octanol; sandal pentanol; sandal pentenol; sandal pentenone; sandalrome; sandalwood oil acetylated; sandel; santall; santalol; santol pentenol; timber propanol; vetiverol; vetiveryl formate and woody epoxide is in the range from 0.0001 to 2.0% by weight, more preferably from 0.001 to 0.5% by weight and most preferably from 0.002 to 0.3% by weight, calculated to the total weight of the composition.

In another aspect of the present invention, it is preferred if the composition comprises a fragrance having a scent of vanilla. Fragrances leading to a vanilla scent can preferably be created by containing in the composition one or more ingredients select from the group consisting of (E)-para-methoxycinnamaldehyde; 1-benzoyl acetone; 2,4,6-triisobutyl-1,3,5-trioxane; 2,4-dimethyl benzaldehyde; 3-(2-furyl) acrolein; 4-ethoxy-3-anisaldehyde; 4-methyl guaiacol; 6-methyl coumarin; alpha-ethoxy-ortho-cresol; benzoin; cis-oak lactone; coniferaldehyde; coumane; curcuma amada roxb. rhizome oil; dianthus ethone; divanillin; ethyl vanillin; ethyl vanillin acetate; ethyl vanillin diethyl acetal; ethyl vanillin hexylene glycol acetal; ethyl vanillin isobutyrate; ethyl vanillin propylene glycol acetal; eugenol isoamyl ether; furfural acetone; geranyl ethyl acetal; glucoethyl vanillin; glucovanillin; guaiacyl propionate; heliotropin; heliotropyl alcohol; homovanillin; isopropyl vanillin; methyl vanillate; ortho-anisaldehyde; ortho-dimethyl hydroquinone; ortho-guaiacol; para-anisyl acetate; para-anisyl formate; para-cresyl laurate; para-methoxycinnamaldehyde; para-vanillic acid; para-vanillyl alcohol; peru balsam; peru balsam oil; powdery ketone; propenyl guaethol; quercus robur oil; sumatra benzoin gum; vanilla; vanilla aromatica fruit extract; vanilla bean oil (vanilla planifolia); vanilla bean oil (vanilla tahitensis); vanilla bean planifolia; vanilla bean tahitensis; vanilla cresol; vanilla oleoresin bali; vanilla oleoresin bourbon; vanillin; vanillin 2,3-butylene glycol acetal; vanillin hexylene glycol acetal; vanillin menthoxypropane diol acetal; vanillin propylene glycol acetal; vanillyl acetate; vanillyl butyl ether; vanillyl ethyl ether; vanillyl isobutyrate; vanillylidene acetone; vanilmandelic acid; veratraldehyde and zingerone.

Thus, a preferred embodiment of the invention is a cosmetic product comprising
a) a container comprising at least 80% by weight post-consumer recycled plastic, based on the total weight of the container, whereby the container has at least one opening,
b) a cosmetic cleansing composition situated in the container characterized in that the composition comprises
   - at least one ingredient selected from the group consisting of (E)-para-methoxycinnamaldehyde; 1-benzoyl acetone; 2,4,6-triisobutyl-1,3,5-trioxane; 2,4-dimethyl benzaldehyde; 3-(2-furyl) acrolein; 4-ethoxy-3-anisaldehyde; 4-methyl guaiacol; 6-methyl coumarin; alpha-ethoxy-ortho-cresol; benzoin; cis-oak lactone; coniferaldehyde; coumane; curcuma amada roxb. rhizome oil; dianthus ethone; divanillin; ethyl vanillin; ethyl vanillin acetate; ethyl vanillin diethyl acetal; ethyl vanillin hexylene glycol acetal; ethyl vanillin isobutyrate; ethyl vanillin propylene glycol acetal; eugenol isoamyl ether; furfural acetone; geranyl ethyl acetal; glucoethyl vanillin; glucovanillin; guaiacyl propionate; heliotropin; heliotropyl alcohol; homovanillin; isopropyl vanillin; methyl vanillate; ortho-anisaldehyde; ortho-dimethyl hydroquinone; ortho-guaiacol; para-anisyl acetate; para-anisyl formate; para-cresyl laurate; para-methoxycinnamaldehyde; para-vanillic acid; para-vanillyl alcohol; peru balsam; peru balsam oil; powdery ketone; propenyl guaethol; quercus robur oil; sumatra benzoin gum; vanilla; vanilla aromatica fruit extract; vanilla bean oil (vanilla planifolia); vanilla bean oil (vanilla tahitensis); vanilla bean planifolia; vanilla bean tahitensis; vanilla cresol; vanilla oleoresin bali; vanilla oleoresin bourbon; vanillin; vanillin 2,3-butylene glycol acetal; vanillin hexylene glycol acetal; vanillin menthoxypropane diol acetal; vanillin propylene glycol acetal; vanillyl acetate; vanillyl butyl ether; vanillyl ethyl ether; vanillyl isobutyrate; vanillylidene acetone; vanilmandelic acid; veratraldehyde and zingerone, and
   - at least one surfactant.

It is preferred if the total quantity of the ingredients of (E)-para-methoxycinnamaldehyde; 1-benzoyl acetone; 2,4,6-triisobutyl-1,3,5-trioxane; 2,4-dimethyl benzaldehyde; 3-(2-furyl) acrolein; 4-ethoxy-3-anisaldehyde; 4-methyl guaiacol; 6-methyl coumarin; alpha-ethoxy-ortho-cresol; benzoin; cis-oak lactone; coniferaldehyde; coumane; curcuma amada roxb. rhizome oil; dianthus ethone; divanillin; ethyl vanillin; ethyl vanillin acetate; ethyl vanillin diethyl acetal; ethyl vanillin hexylene glycol acetal; ethyl vanillin isobutyrate; ethyl vanillin propylene glycol acetal; eugenol isoamyl ether; furfural acetone; geranyl ethyl acetal; glucoethyl vanillin; glucovanillin; guaiacyl propionate; heliotropin; heliotropyl alcohol; homovanillin; isopropyl vanillin; methyl vanillate; ortho-anisaldehyde; ortho-dimethyl hydroquinone; ortho-guaiacol; para-anisyl acetate; para-anisyl formate; para-cresyl laurate; para-methoxycinnamaldehyde; para-vanillic acid; para-vanillyl alcohol; peru balsam; peru balsam oil; powdery ketone; propenyl guaethol; quercus robur oil; sumatra benzoin gum; vanilla; vanilla aromatica fruit extract; vanilla bean oil (vanilla planifolia); vanilla bean oil (vanilla tahitensis); vanilla bean planifolia; vanilla bean tahitensis; vanilla cresol; vanilla oleoresin bali; vanilla oleoresin bourbon; vanillin; vanillin 2,3-butylene glycol acetal; vanillin hexylene glycol acetal; vanillin menthoxypropane diol acetal; vanillin propylene glycol acetal; vanillyl acetate; vanillyl butyl ether; vanillyl ethyl ether; vanillyl isobutyrate; vanillylidene acetone; vanilmandelic acid; veratraldehyde and zingerone is in the range from 00.0001 to 2.0% by weight, more preferably from 0.001 to 0.5% by weight and most preferably from 0.002 to 0.3% by weight, calculated to the total weight of the composition.

In another aspect of the present invention, it is preferred if the composition comprises a fragrance having a scent of amber. Fragrances leading to an amber scent can preferably be created by containing in the composition one or more ingredients select from the group consisting of (-)-isolongifolene; (+)-alpha-campholenic aldehyde; (1R,4S,5S,9R)-1-hydroxy-1,4,7,7,9-pentamethyl spiro(4.5)decan-2-one; (3R)-(+)-3-methyl-1,4-dioxacyclohexadecan-2-one; (3R)-(+)-3-methyl-1,4-dioxacyclopentadecan-2-one; (3S)-(-)-3-methyl-1,4-dioxacyclohexadecan-2-one; (3S)-(-)-3-methyl-1,4-dioxacyclopentadecan-2-one; (4aR,5R,7aS,9R)-octahydro-2,2,5,8,8,9a-hexamethyl-4h-4a,9-methanoazuleno(5,6-d)-1,3-dioxole; (cis+trans)-1,1,2,3,3-pentamethyl-2,3,3a,4,5,9b-hexahydro-1H-7,9-diazacyclopenta[a]naphthalene; (cis+trans)-1,1,3,3-pentamethyl-2,3,3a,4,5,9b-hexahydro-1H-7,9-diazacyclopenta[a]naphthalene; (cis+trans)-6,6a,7,8,9,9a-hexahydro-7,7,8,9,9-pentamethyl-5H-cyclopenta[H]quinazoline; (E)-3-methyl-5-cyclotetradecen-1-one; (E)-woody epoxide; (S)-dihydro-beta-ionol; (Z)-abienol; 2,6,10,10-tetramethyl-1-oxa-spiro[4.5]dec-3-en-6-yl acetate; 2-acetyl-4-isopropenyl pyridine; 2-methyl undecanal (aldehyde C-12 mna); 2-methyl undecanal dimethyl acetal; 2-methyl-4-(camphenyl-8) cyclohexanone; 3(or 2),4,5-trimethyl octahydro-4,7-methanoinden-5-yl acetate; 3a,3b,5,6,7,8a-hexahydro-2,2,3a,3b,7,7-hexamethyl-4H-indeno[1,2-D]-1,3-dioxole; 4-(3,3-dimethyl bicyclo(2.2.1)hept-2-yl)-1-methyl-2-oxabicyclo(2.2.2)octane; 4-(3,3-dimethyl bicyclo(2.2.1)hept-2-yl)-5-methyl-2-oxabicyclo(2.2.2)octane; 4-allyl-1,4-dimethyl bicyclo(3.2.1)octan-3-one; 4-isopropyl cinnamaldehyde; 5,5a,6,7,8,8a-hexahydro-6,6,7,8,8-pentamethyl-4H-indeno[5,4-D]isoxazole; 5,6,7,8-tetrahydro-6,6,7,8,8-pentamethyl-4H-indeno[5,4-D]isoxazole; 6,6,10,10-tetramethyl-5,7,8,9,10,10a-hexahydro-6H-6a,9-methanobenzo[H]quinazoline; 6,7,8,9-tetrahydro-7,7,8,9,9-pentamethyl-5H-cyclopenta[H]quinazoline; acetoxymethyl isolongifolene; acetyl cedrene; alpha-ambrinol; alpha-cedrene epoxide; alpha-ethyl-2,2,6-trimethyl cyclohexane propanol; alpha-selinene; amber acetate; amber butanol; amber carane; amber carbinol; amber cyclohexanol; amber decane; amber decatriene; amber dioxane; amber dioxepine; amber dodecane; amber formate; amber furan; amber naphthofuran; amber oil; amber oxepin; amber pentadecane; amber spirolene; ambergris naphthol; ambergris tincture; ambrain; ambrein; ambrene acetal; ambrette seed oil; ambrettolic acid; ambrettolide; ambrinol; ambroxan; ambroxide; amyl benzoate; amyl cinnamate; amyris acetate; angelica root oil; animal carbolactone; atlas cedarwood oil fractions; beta-caryophyllene alcohol acetate; beta-isomethyl ionone; black poplar bud oil; butyl benzoate; calarene epoxide; cedar cyclododecatriene; cedarwood acetate; cedarwood oil texas fractions; cedrol methyl ether; cedryl formate; cedryl methyl ether; cistus ladaniferus gum; cistus twig/leaf concrete; cistus twig/leaf oil; cistus twig/leaf oil molecular distilled; clearwood ; cyclohexanecarboxylic acid, 2,2,6-trimethyl-, ethyl ester, (1R,6S)-; dewy propionate; dihydro-beta-ionol; dihydro-beta-ionone; dihydro-gamma-ionone; elecampane root oil; eriocephalus punctulatus flower oil; formoxymethyl isolongifolene; georgywood; geranyl benzoate; geranyl cinnamate; homoambrinol; hydroxyambran; hydroxymethyl isolongifolene; isoamyl cinnamate; isobutyl phenyl acetate; juniper berry concrete; juniper lactone; labdanum concrete; labdanum gum extract ethyl ester; labdanum oil; linalyl cinnamate; mcp acetate; melozol acetate; methyl 2,6,10-trimethyl cyclododeca-2,5,9-trienyl ketone; methyl cedryl ketone; methyl hydrogenated rosinate; methyl methylene tricyclodecanol acetate; muscomer; musk amberol; musk dimethyl indane; musk indane; musk lactone; musk methyl ketone; musk tetralin; myrrh oil; para-methyl tetrahydroquinoline; patchouli ethanone; peru balsam oil; piper longum fruit oil; propyl cinnamate; romanal; rosa odorata flower oil; satinaldehyde; sclarene; sclareol; sclareolide; spruce needle oil canada; styrax gum (liquidambar styraciflua); symroxane (CAS: 676125-00-1 and 393517-29-8); tetradecanal; tetramethyl-4-methylene-2-heptanol; tetramethyl-5-oxatricyclododecane; timber propanol; tolu balsam; tricyclodecenyl isobutyrate; valeriana officinalis root extract; vetiver oil ;vetiver oil haiti; vetiveria zizanioides root oil; violet propanol; woody cyclohexanone; woody dioxolane; woody dodecane; woody epoxide; woody ether; woody furan; woody nonane (ethoxy) (equal to 4-ethoxy-4,8,8-trimethyl-9-methylidenebicyclo[3.3.1]nonane) and woody propanol.

Thus, a preferred embodiment of the invention is a cosmetic product comprising
a) a container comprising at least 80% by weight post-consumer recycled plastic, based on the total weight of the container, whereby the container has at least one opening,
b) a cosmetic cleansing composition situated in the container characterized in that the composition comprises
   - at least one ingredient selected from the group consisting of (-)-isolongifolene; (+)-alpha-campholenic aldehyde; (1R,4S,5S,9R)-1-hydroxy-1,4,7,7,9-pentamethyl spiro(4.5)decan-2-one; (3R)-(+)-3-methyl-1,4-dioxacyclohexadecan-2-one; (3R)-(+)-3-methyl-1,4-dioxacyclopentadecan-2-one; (3S)-(-)-3-methyl-1,4-dioxacyclohexadecan-2-one; (3S)-(-)-3-methyl-1,4-dioxacyclopentadecan-2-one; (4aR,5R,7aS,9R)-octahydro-2,2,5,8,8,9a-hexamethyl-4h-4a,9-methanoazuleno(5,6-d)-1,3-dioxole; (cis+trans)-1,1,2,3,3-pentamethyl-2,3,3a,4,5,9b-hexahydro-1H-7,9-diazacyclopenta[a]naphthalene; (cis+trans)-1,1,3,3-pentamethyl-2,3,3a,4,5,9b-hexahydro-1H-7,9-diazacyclopenta[a]naphthalene; (cis+trans)-6,6a,7,8,9,9a-hexahydro-7,7,8,9,9-pentamethyl-5H-cyclopenta[H]quinazoline; (E)-3-methyl-5-cyclotetradecen-1-one; (E)-woody epoxide; (S)-dihydro-beta-ionol; (Z)-abienol; 2,6,10,10-tetramethyl-1-oxa-spiro[4.5]dec-3-en-6-yl acetate; 2-acetyl-4-isopropenyl pyridine; 2-methyl undecanal (aldehyde C-12 mna); 2-methyl undecanal dimethyl acetal; 2-methyl-4-(camphenyl-8) cyclohexanone; 3(or 2),4,5-trimethyl octahydro-4,7-methanoinden-5-yl acetate; 3a,3b,5,6,7,8a-hexahydro-2,2,3a,3b,7,7-hexamethyl-4H-indeno[1,2-D]-1,3-dioxole; 4-(3,3-dimethyl bicyclo(2.2.1)hept-2-yl)-1-methyl-2-oxabicyclo(2.2.2)octane; 4-(3,3-dimethyl bicyclo(2.2.1)hept-2-yl)-5-methyl-2-oxabicyclo(2.2.2)octane; 4-allyl-1,4-dimethyl bicyclo(3.2.1)octan-3-one; 4-isopropyl cinnamaldehyde; 5,5a,6,7,8,8a-hexahydro-6,6,7,8,8-pentamethyl-4H-indeno[5,4-D]isoxazole; 5,6,7,8-tetrahydro-6,6,7,8,8-pentamethyl-4H-indeno[5,4-D]isoxazole; 6,6,10,10-tetramethyl-5,7,8,9,10,10a-hexahydro-6H-6a,9-methanobenzo[H]quinazoline; 6,7,8,9-tetrahydro-7,7,8,9,9-pentamethyl-5H-cyclopenta[H]quinazoline; acetoxymethyl isolongifolene; acetyl cedrene; alpha-ambrinol; alpha-cedrene epoxide; alpha-ethyl-2,2,6-trimethyl cyclohexane propanol; alpha-selinene; amber acetate; amber butanol; amber carane; amber carbinol; amber cyclohexanol; amber decane; amber decatriene; amber dioxane; amber dioxepine; amber dodecane; amber formate; amber furan; amber naphthofuran; amber oil; amber oxepin; amber pentadecane; amber spirolene; ambergris naphthol; ambergris tincture; ambrain; ambrein; ambrene acetal; ambrette seed oil; ambrettolic acid; ambrettolide; ambrinol; ambroxan; ambroxide; amyl benzoate; amyl cinnamate; amyris acetate; angelica root oil; animal carbolactone; atlas cedarwood oil fractions; beta-caryophyllene alcohol acetate; beta-isomethyl ionone; black poplar bud oil; butyl benzoate; calarene epoxide; cedar cyclododecatriene; cedarwood acetate; cedarwood oil texas fractions; cedrol methyl ether; cedryl formate; cedryl methyl ether; cistus ladaniferus gum; cistus twig/leaf concrete; cistus twig/leaf oil; cistus twig/leaf oil molecular distilled; clearwood ; cyclohexanecarboxylic acid, 2,2,6-trimethyl-, ethyl ester, (1R,6S)-; dewy propionate; dihydro-beta-ionol; dihydro-beta-ionone; dihydro-gamma-ionone; elecampane root oil; eriocephalus punctulatus flower oil; formoxymethyl isolongifolene; georgywood; geranyl benzoate; geranyl cinnamate; homoambrinol; hydroxyambran; hydroxymethyl isolongifolene; isoamyl cinnamate; isobutyl phenyl acetate; juniper berry concrete; juniper lactone; labdanum concrete; labdanum gum extract ethyl ester; labdanum oil; linalyl cinnamate; mcp acetate; melozol acetate; methyl 2,6,10-trimethyl cyclododeca-2,5,9-trienyl ketone; methyl cedryl ketone; methyl hydrogenated rosinate; methyl methylene tricyclodecanol acetate; muscomer; musk amberol; musk dimethyl indane; musk indane; musk lactone; musk methyl ketone; musk tetralin; myrrh oil; para-methyl tetrahydroquinoline; patchouli ethanone; peru balsam oil; piper longum fruit oil ;propyl cinnamate; romanal; rosa odorata flower oil; satinaldehyde; sclarene; sclareol; sclareolide; spruce needle oil canada; styrax gum (liquidambar styraciflua); symroxane (CAS: 676125-00-1 and 393517-29-8); tetradecanal; tetramethyl-4-methylene-2-heptanol; tetramethyl-5-oxatricyclododecane; timber propanol; tolu balsam; tricyclodecenyl isobutyrate; valeriana officinalis root extract; vetiver oil ; vetiver oil haiti; vetiveria zizanioides root oil; violet propanol; woody cyclohexanone; woody dioxolane; woody dodecane; woody epoxide; woody ether; woody furan; woody nonane (ethoxy) (equal to 4-ethoxy-4,8,8-trimethyl-9-methylidenebicyclo[3.3.1]nonane) and woody propanol, and
   - at least one surfactant.

It is preferred if the total quantity of the ingredients of (-)-isolongifolene; (+)-alpha-campholenic aldehyde; (1R,4S,5S,9R)-1-hydroxy-1,4,7,7,9-pentamethyl spiro(4.5)decan-2-one; (3R)-(+)-3-methyl-1,4-dioxacyclohexadecan-2-one; (3R)-(+)-3-methyl-1,4-dioxacyclopentadecan-2-one; (3S)-(-)-3-methyl-1,4-dioxacyclohexadecan-2-one; (3S)-(-)-3-methyl-1,4-dioxacyclopentadecan-2-one; (4aR,5R,7aS,9R)-octahydro-2,2,5,8,8,9a-hexamethyl-4h-4a,9-methanoazuleno(5,6-d)-1,3-dioxole; (cis+trans)-1,1,2,3,3-pentamethyl-2,3,3a,4,5,9b-hexahydro-1H-7,9-diazacyclopenta[a]naphthalene; (cis+trans)-1,1,3,3-pentamethyl-2,3,3a,4,5,9b-hexahydro-1H-7,9-diazacyclopenta[a]naphthalene; (cis+trans)-6,6a,7,8,9,9a-hexahydro-7,7,8,9,9-pentamethyl-5H-cyclopenta[H]quinazoline; (E)-3-methyl-5-cyclotetradecen-1-one; (E)-woody epoxide; (S)-dihydro-beta-ionol; (Z)-abienol; 2,6,10,10-tetramethyl-1-oxa-spiro[4.5]dec-3-en-6-yl acetate; 2-acetyl-4-isopropenyl pyridine; 2-methyl undecanal (aldehyde C-12 mna); 2-methyl undecanal dimethyl acetal; 2-methyl-4-(camphenyl-8) cyclohexanone; 3(or 2),4,5-trimethyl octahydro-4,7-methanoinden-5-yl acetate; 3a,3b,5,6,7,8a-hexahydro-2,2,3a,3b,7,7-hexamethyl-4H-indeno[1,2-D]-1,3-dioxole; 4-(3,3-dimethyl bicyclo(2.2.1)hept-2-yl)-1-methyl-2-oxabicyclo(2.2.2)octane; 4-(3,3-dimethyl bicyclo(2.2.1)hept-2-yl)-5-methyl-2-oxabicyclo(2.2.2)octane; 4-allyl-1,4-dimethyl bicyclo(3.2.1)octan-3-one; 4-isopropyl cinnamaldehyde; 5,5a,6,7,8,8a-hexahydro-6,6,7,8,8-pentamethyl-4H-indeno[5,4-D]isoxazole; 5,6,7,8-tetrahydro-6,6,7,8,8-pentamethyl-4H-indeno[5,4-D]isoxazole; 6,6,10,10-tetramethyl-5,7,8,9,10,10a-hexahydro-6H-6a,9-methanobenzo[H]quinazoline; 6,7,8,9-tetrahydro-7,7,8,9,9-pentamethyl-5H-cyclopenta[H]quinazoline; acetoxymethyl isolongifolene; acetyl cedrene; alpha-ambrinol; alpha-cedrene epoxide; alpha-ethyl-2,2,6-trimethyl cyclohexane propanol; alpha-selinene; amber acetate; amber butanol; amber carane; amber carbinol; amber cyclohexanol; amber decane; amber decatriene; amber dioxane; amber dioxepine; amber dodecane; amber formate; amber furan; amber naphthofuran; amber oil; amber oxepin; amber pentadecane; amber spirolene; ambergris naphthol; ambergris tincture; ambrain; ambrein; ambrene acetal; ambrette seed oil; ambrettolic acid; ambrettolide; ambrinol; ambroxan; ambroxide; amyl benzoate; amyl cinnamate; amyris acetate; angelica root oil; animal carbolactone; atlas cedarwood oil fractions; beta-caryophyllene alcohol acetate; beta-isomethyl ionone; black poplar bud oil; butyl benzoate; calarene epoxide; cedar cyclododecatriene; cedarwood acetate; cedarwood oil texas fractions; cedrol methyl ether; cedryl formate; cedryl methyl ether; cistus ladaniferus gum; cistus twig/leaf concrete; cistus twig/leaf oil; cistus twig/leaf oil molecular distilled; clearwood ; cyclohexanecarboxylic acid, 2,2,6-trimethyl-, ethyl ester, (1R,6S)-; dewy propionate; dihydro-beta-ionol; dihydro-beta-ionone; dihydro-gamma-ionone; elecampane root oil; eriocephalus punctulatus flower oil; formoxymethyl isolongifolene; georgywood; geranyl benzoate; geranyl cinnamate; homoambrinol; hydroxyambran; hydroxymethyl isolongifolene; isoamyl cinnamate; isobutyl phenyl acetate; juniper berry concrete; juniper lactone; labdanum concrete; labdanum gum extract ethyl ester; labdanum oil; linalyl cinnamate; mcp acetate; melozol acetate; methyl 2,6,10-trimethyl cyclododeca-2,5,9-trienyl ketone; methyl cedryl ketone; methyl hydrogenated rosinate; methyl methylene tricyclodecanol acetate; muscomer; musk amberol; musk dimethyl indane; musk indane; musk lactone; musk methyl ketone; musk tetralin; myrrh oil; para-methyl tetrahydroquinoline; patchouli ethanone; peru balsam oil; piper longum fruit oil ; propyl cinnamate; romanal; rosa odorata flower oil; satinaldehyde; sclarene; sclareol; sclareolide; spruce needle oil canada; styrax gum (liquidambar styraciflua); symroxane (CAS: 676125-00-1 and 393517-29-8); tetradecanal; tetramethyl-4-methylene-2-heptanol; tetramethyl-5-oxatricyclododecane; timber propanol; tolu balsam; tricyclodecenyl isobutyrate; valeriana officinalis root extract; vetiver oil ; vetiver oil haiti; vetiveria zizanioides root oil; violet propanol; woody cyclohexanone; woody dioxolane; woody dodecane; woody epoxide; woody ether; woody furan; woody nonane (ethoxy) (equal to 4-ethoxy-4,8,8-trimethyl-9-methylidenebicyclo[3.3.1]nonane) and woody propanol is in the range from 0.0001 to 2.0% by weight, more preferably from 0.001 to 0.5% by weight and most preferably from 0.002 to 0.3% by weight, calculated to the total weight of the composition.

A further preferred embodiment is a cosmetic product comprising
a) a container comprising at least 80% by weight post-consumer recycled plastic, based on the total weight of the container, whereby the container has at least one opening,
b) a cosmetic cleansing composition situated in the container characterized in that the composition comprises
   - at least one ingredient selected from the group consisting of Alpha-Isomethyl lonone, Anise Alcohol, Benzyl Alcohol, Benzyl Benzoate, Benzyl Cinnamate, Benzyl Salicylate, Butylphenyl Methylpropional, Cinnamal, Cinnamyl Alcohol, Citral, Citronellol, Coumarin, D-Limonene, Eugenol, Linalool, Amyl Cinnamal, Amylcinnamyl Alcohol, Evernia Furfuracea (Treemoss) Extract, Evernia Prunastri (Oakmoss) Extract, Farnesol, Geraniol, Hexyl Cinnamal, Hydroxycitronellal, Isoeugenol, Methyl 2-Octynoate, Acetyl Cedrene, Alpha-Pinene, Alpha-Santalol and Betasantalol, Alpha-Terpinene, Anethole, Benzaldehyde, Beta-Caryophyllene, Beta-Pinene, Camphor, Cedrus Atlantica Extract, Cinnamomum Zeylanicum Extract, Citrus Sinensis/Aurantium Dulcis Extract, Cymbopogon Citratus / Schoenanthus Extract, Damascenone, Damascone Delta, Damascone-Cis-Alpha, Damascone-Cis-Beta, Dimethyl Benzyl Carbinyl Acetate (Dmbca), Eucalyptus Extract, Eucalyptus Extract, Eugenia Caryophyllus Extract, Eugenyl Acetate, Laurus Nobilis Extract, Lavandula Hybrida Extract, Lavandula Officinalis Extract, Linalyl Acetate, Mentha Piperita Extract, Menthol, Methyl Salicylate, Narcissus Extract, Otne, Pelargonium Graveolens Extract, Peru Balsam Extracts and Distillates (Myroxylon Balsamum Var Pereirae), Pinus Mugo Extract, Pinus Pumila Extract, Propylidene Phthalide, Terpinolene, Trimethyl-Benzenepropanol, Turpentine, Verbena Absolute (Lippia Citriodora Kunth.), 3-Methyl-5-(2,2,3-Trimethyl-3-Cyclopentenyl)Pent-4-en-2-ol, Alpha-Terpineol, Amyl Salicylate, Carvone, Cinnamomum Cassia Extract, Citrus Aurantium Amara Extract, Citrus Bergamia Extract, Citrus Limonum Extract, Geranyl Acetate, Hexadecanolactone, Hexamethylindanopyran, Isoeugenyl Acetate, Jasmine Absolute (Grandiflorum), Juniperus Virginiana Extract, Pogostemon Cablin Extract, Rose Extract, Salicylaldehyde, Santalum Album Extract, Sclareol, Terpineol, Vanillin und Ylang Ylang Extracts, and
   - at least one surfactant.

The composition of the invention is preferably a cleansing composition, preferably a skin cleanser, body wash or shampoo.

According to the invention it is preferred if the composition comprises surfactants in a total quantity ranging from 0.5 to 15.0% by weight, more preferably from 6.0 to 13.0% by weight and most preferably from 9.5 to 12.0% by weight, calculated to the total weight of the composition.

Firstly, it is preferred if the composition comprises at least one anionic surfactant.

Anionic surfactants may be chosen from:
- linear and branched fatty acids having 8 to 30 C atoms (soaps),
- ether carboxylic acids of the formula R-O-(CH₂-CH₂O)ₓCH₂-COOH, in which R is a linear or branched, saturated or unsaturated alkyl group having 8 to 30 C atoms and x=0 or 1 to 16,
- acyl sarcosides having 8 to 24 C atoms in the acyl group,
- acyl taurides having 8 to 24 C atoms in the acyl group,
- acyl isethionates having 8 to 24 C atoms in the acyl group,
- sulfosuccinic acid mono- and/or dialkyl esters having 8 to 24 C atoms in the alkyl group and sulfosuccinic acid monoalkylpolyoxyethyl esters having 8 to 24 C atoms in the alkyl group and 1 to 6 oxyethyl groups,
- alpha-olefinsulfonates having 8 to 24 C atoms,
- alkyl sulfate and/or alkyl polyglycol ether sulfate salts of the formula R-(OCH₂-CH₂)ₓ-OSO₃ ⁻M⁺, in which R is a preferably linear or branched, saturated or unsaturated alkyl group having 8 to 30 C atoms, x=0 or 1 to 12, and M is an alkali or ammonium ion,
- sulfonates of unsaturated fatty acids having 8 to 24 C atoms and 1 to 6 double bonds,
- esters of tartaric acid and citric acid with alcohols, which are adducts of about 2-15 molecules of ethylene oxide and/or propylene oxide to fatty alcohols having 8 to 22 C atoms,
- alkyl and/or alkenyl ether phosphates of the formula, 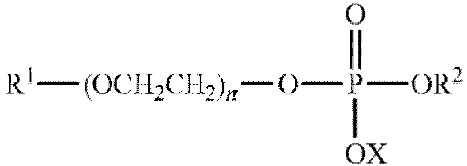 in which R¹ preferably stands for an aliphatic hydrocarbon group having 8 to 30 carbon atoms, R² for hydrogen, a group (CH₂CH₂O)ₙR¹ or X, n for numbers from 0 to 10, and X for hydrogen, an alkali metal or alkaline earth metal, or NR³R⁴R⁵R⁶, where R³ to R⁶ independently of one another stand for a C₁ to C₄ hydrocarbon group.

Preferred anionic surfactants are ether carboxylic acids of the aforementioned formula, acyl sarcosides having 8 to 24C atoms in the acyl group, sulfosuccinic acid mono and/or -dialkyl esters having 8 to 24 C atoms in the alkyl group and sulfosuccinic acid monoalkylpolyoxyethyl esters having 8 to 24C atoms in the alkyl group and 1 to 6 oxyethyl groups, alpha-olefinsulfonates having 8 to 24C atoms, and/or alkylsulfate salts and/or alkyl polyglycol ether sulfate salts of the aforementioned formula.

Especially preferred anionic surfactants are straight-chain or branched alkyl ether sulfates, which include an alkyl group having 8 to 18 and especially having 10 to 16 C atoms, and 1 to 6 and particularly 2 to 4 ethylene oxide units. Furthermore, especially preferred anionic surfactants are straight-chain or branched alkyl sulfonates, which include an alkyl group having 8 to 18 and especially having 10 to 16 C atoms. Preferred in particular are the sodium, magnesium, and/or triethanolamine salts of linear or branched lauryl, tridecyl, and/or myristyl sulfates, which have a degree of ethoxylation of 2 to 4.

Preferred compositions according to the invention are characterized in that they include at least one anionic surfactant from the group of alkyl sulfates and alkyl polyglycol ether sulfates of the formula R-(OCH₂-CH₂)ₓ-OSO₃M, in which R preferably stands for a straight-chain or branched, saturated or mono- or polyunsaturated alkyl or alkenyl group having 8 to 24 carbon atoms, x for 0 or 1 to 12, and M for an alkali metal or alkaline earth metal or for triethanolamine, whereby anionic surfactants with the INCI name Sodium Lauryl Sulfate, Sodium Laureth Sulfate and/or Sodium Myreth Sulfate are especially preferred.

According to the invention it is preferred if the total quantity of anionic surfactants is in the range from 3.0 to 9.0% by weight, more preferably, 3.8 to 8.7% by weight and most preferably from 4.8 to 7.3% by weight, calculated to the total weight of the composition.

Further it is preferred if the composition comprises at least one amphoteric surfactant. Amphoteric surfactants are added to the agents according to the invention, based on their total weight, preferably in amounts from 1.0 to 12% by weight, more preferably from 2.0 to 10% by weight, and especially from 3.0 to 6.0% by weight. Suitable amphoteric surfactants are selected from compounds of the following formulas (i) to (v), in which the group R in each case stands for a straight-chain or branched, saturated or mono- or polyunsaturated alkyl or alkenyl group having 8 to 24 carbon atoms,

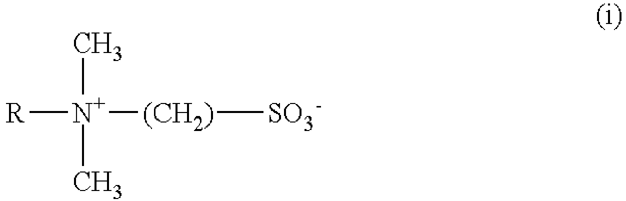

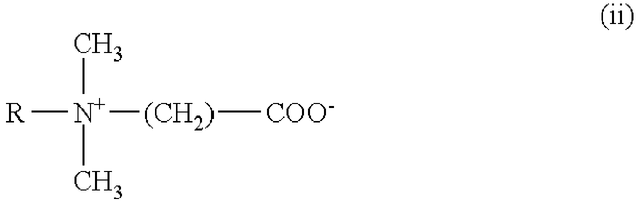

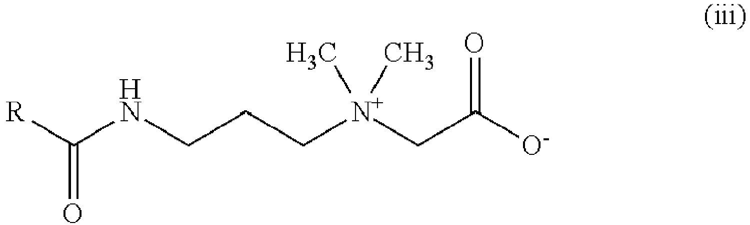

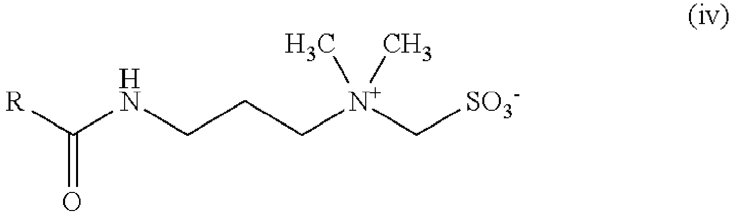

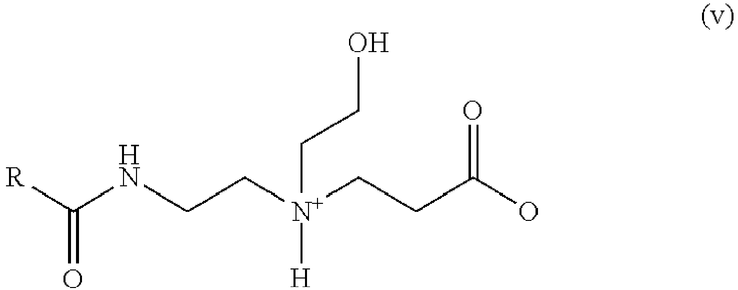

Especially preferred amphoteric surfactants are alkyl amidoalkyl betaines and/or alkyl ampho(di)acetates of the aforementioned formulas (i) to (v). Especially suitable amphoteric surfactants include the surfactants known under the INCI names Cocamidopropyl Betaine and Disodium Cocoamphodiacetate. Compositions especially preferred according to the invention are characterized in that they include at least one amphoteric surfactant (B) from the group of the general formula (iii), whereby amphoteric surfactants with the INCI name Cocamidopropyl Betaine are especially preferred.

According to the invention it is preferred if cocamidopropyl betaine is contained in the composition in a total quantity ranging from 2.0 to 10% by weight, more preferably 2.5 to 8.0% by weight and most preferably 3.0 to 6.0% by weight, calculated to the total weight of the composition.

Further, it is preferred if the composition comprises at least one nonionic surfactant. The total quantity of nonionic surfactants is preferably in the range from 0.1 to 7.5% by weight, preferably 0.25 to 5% by weight, and especially 0.5 to 3% by weight, calculated to the total weight of the compositions.

Especially suitable nonionic surfactants are alkyl oligoglucosides, especially alkyl oligoglucosides based on hydrogenated C12/14 coconut alcohol with a DP of 1 to 3, as they are commercially available, for example, under the INCI name "Coco-Glucosides," and fatty acid alkanolamides, especially C8-C24 fatty acid (di)ethanolamides. According to the invention it is preferred if the alkyl glycosides are chosen from lauryl glucoside, decyl glucoside and coco glucoside.

It is also preferred if alkyl glycosides are contained in the composition in a total quantity ranging from 0.1 to 7.0% by weight, more preferably 0.5 to 3.0% by weight and most preferably 1.0 to 2.5% by weight, calculated to the total weight of the composition. For the case that lauryl glucoside, decyl glucoside and/or coco glucoside are contained, it is preferred if the total quantity of lauryl glucoside, decyl glucoside and/or coco glucoside is in the range from 0.1 to 7.0% by weight, more preferably 0.5 to 3.0% by weight and most preferably 1.0 to 2.5% by weight, calculated to the total weight of the composition.

Further compositions according to the invention may preferably comprise further oils to provide an extra caring effect. Those oils can preferably be selected from mineral, natural, and synthetic oil components. However, natural and/or synthetic oils are more preferred than mineral oils.

Triglycerides and mixtures of triglycerides are usually employed as natural (plant) oils. Preferred natural oils are coconut oil, (sweet) almond oil, walnut oil, peach kernel oil, apricot kernel oil, avocado oil, tea tree oil, soybean oil, glycine soja oil, sesame oil, sunflower oil, tsubaki oil, evening primrose oil, rice bran oil, palm kernel oil, mango kernel oil, cuckoo flower oil, thistle oil, macadamia nut oil, grape seed oil, amaranth seed oil, argan oil, bamboo oil, olive oil, wheat germ oil, pumpkin seed oil, mallow oil, hazelnut oil, safflower oil, canola oil, sasanqua oil, jojoba oil, rambutan oil, cocoa butter, and shea butter. Glycine soja oil is preferred.

Mineral oils, paraffin oils, and isoparaffin oils, and synthetic hydrocarbons in particular are used as mineral oils.

A dialkyl ether can serve furthermore as an oil. Dialkyl ethers which can be used are, in particular, di-n-alkyl ethers having a total of between 12 and 36 carbon atoms, particularly 12 to 24 carbon atoms, such as, for example, di-n-octyl ether, di-n-decyl ether, di-n-nonyl ether, di-n-undecyl ether, di-n-dodecyl ether, n-hexyl n-octyl ether, n-octyl n-decyl ether, n-decyl n-undecyl ether, n-undecyl n-dodecyl ether, and n-hexyl n-undecyl ether, as well as di-tert-butyl ether, di-isopentyl ether, di-3-ethyldecyl ether, tert-butyl n-octyl ether, isopentyl n-octyl ether, and 2-methylpentyl n-octyl ether.

Further oils preferred according to the invention are ester oils. Ester oils are understood to be the esters of C6-C30 fatty acids with C2-C30 fatty alcohols. The monoesters of the fatty acids with alcohols having 2 to 24 C atoms are preferred. Examples of employed fatty acid components in the esters are caproic acid, caprylic acid, 2-ethylhexanoic acid, capric acid, lauric acid, isotridecanoic acid, myristic acid, palmitic acid, palmitoleic acid, stearic acid, isostearic acid, oleic acid, elaidic acid, petroselic acid, linoleic acid, linolenic acid, elaeostearic acid, arachidic acid, gadoleic acid, behenic acid, and erucic acid, and technical mixtures thereof. Examples of the fatty alcohol components in the ester oils are isopropyl alcohol, caproic alcohol, caprylic alcohol, 2-ethylhexyl alcohol, capric alcohol, lauryl alcohol, isotridecyl alcohol, myristyl alcohol, cetyl alcohol, palmoleyl alcohol, stearyl alcohol, isostearyl alcohol, oleyl alcohol, elaidyl alcohol, petroselinyl alcohol, linolyl alcohol, linolenyl alcohol, elaeostearyl alcohol, arachyl alcohol, gadoleyl alcohol, behenyl alcohol, erucyl alcohol, and brassidyl alcohol, and technical mixtures thereof. Isopropyl myristate (Rilanit^{®} IPM), isononanoic acid C16-18 alkyl ester (Cetiol^{®} SN), 2-ethylhexyl palmitate (Cegesoft^{®} 24), stearic acid 2-ethylhexyl ester (Cetiol^{®} 868), cetyl oleate, glycerol tricaprylate, coconut fatty alcohol caprinate/caprylate (Cetiol^{®} LC), n-butyl stearate, oleyl erucate (Cetiol^{®} J 600), isopropyl palmitate (Rilanit^{®} IPP), oleyl oleate (Cetiol^{®}), lauric acid hexyl ester (Cetiol^{®} A), di-n-butyl adipate (Cetiol^{®} B), myristyl myristate (Cetiol^{®} MM), cetearyl isononanoate (Cetiol^{®} SN), and oleic acid decyl ester (Cetiol^{®} V) are especially preferred.

Further, it is preferred if the oil contained in the composition is a PPG-alkyl ether. Preferred PPG-alkyl ethers are selected from the group defined by the formula 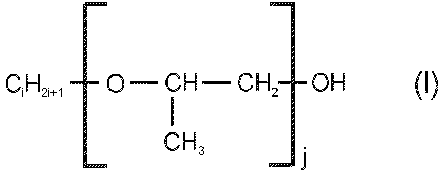 wherein i is an integer in the range from 4 to 20, and *j* is an integer in the range from 2 to 20.

Preferred PPG-alkyl ethers are defined by i being an integer in the range from 10 to 18 and j being an integer in the range from 10 to 16. More preferred is the PPG-15 stearyl ether.

Most preferred is the use of PPG-alkyl ethers as defined above and/or natural oils as described above.

Further, it is preferred if the composition comprises one or more further polymeric compounds. Polymeric compounds are preferably polysaccharides. It is preferred if the polymeric compound is a non-ionic polysaccharide. According to the invention it is preferred if the nonionic Polysaccharide is selected from natural gums. Natural gums are for example sclerotium gum and xanthan gum. It is particularly preferred if xanthan gum is included as nonionic polysaccharide.

Accordingly, it is preferred if a natural gum is included in the range from 0.02 to 1.2% by weight, more preferably 0.03 to 1.0% by weight, more preferably 0.04 to 0.8% and most preferably 0.05 to 0.7% by weight, calculated to the total weight of the composition.

In one embodiment of the invention, it is preferred if one or more nonionic emulsifiers are contained. Although basically all nonionic emulsifiers can be contained in the composition of the invention, some emulsifiers were found to be particularly beneficial for the effect described. Preferred nonionic emulsifier are selected from oleth-20, glyceryl stearate, glyceryl stearate SE and PEG-40 hydrogenated castor oil.

Further, it was noted that it is beneficial according to the invention if the composition further comprises at least one fatty alcohol. Fatty alcohols are straight-chain primary alcohols containing 8 to 26 carbon atoms. For the case that at least one fatty alcohol is contained, it is preferred if the total quantity of the fatty alcohols is in the range from 0.05 to 4.0% by weight, more preferably 0.2 to 3% by weight and most preferably 0.5 to 2.5% by weight, calculated to the total weight of the composition.

In one embodiment of the invention tt is preferred if the composition is an oil in water emulsion. If the two liquids of an emulsion are water and oil and if oil droplets are finely distributed in water, then this is an oil in water emulsion (O/W emulsion, e.g., milk). The basic character of an O/W emulsion is determined by the water. In the case of a water-in-oil emulsion (W/O emulsion, e.g., butter), the principle is reversed, the basic character here being determined by the oil.

Further it is preferred if the composition is free from polymers polymerized from a mixture comprising acrylic acid and/or methacrylic acid. Such polymers were often used in the past. However, due to not known biodegradability these polymers should not be included in state-of-the-art formulations anymore. Accordingly, it is preferred if no polymers polymerized from a mixture comprising acrylic acid and/or methacrylic acid are contained.

Furthermore, it is advantageous if the cosmetic composition according to the invention contains water as cosmetic carrier in a proportion of 50% by weight to 98% by weight, preferably 60% by weight to 94% by weight and most preferably from 65% by weight to 95% by weight, based on the total weight of the composition.

Further it is preferred if the composition comprises sodium benzoate and/or sodium salicylate to ensure antimicrobial stability. For that purpose, it is preferred to have sodium benzoate and/or sodium salicylate contained in a total quantity ranging from 0.1 to 2% by weight, calculated to the total weight of the composition.

Further it is preferred if the composition comprises phenoxyethanol to ensure antimicrobial stability. For that purpose, it is preferred to have phenoxyethanol contained in a total quantity ranging from 0.1 to 1% by weight, calculated to the total weight of the composition.

The composition may further comprise sodium chloride in order to thicken the composition. Sodium chloride is preferably contained in quantities ranging from 0.1 to 1.5% by weight, calculated to the total weight of the composition.

Further, it is preferred if the composition comprises glycol distearate. Glycol distearate may be used as opacifier. For the case that glycol distearate is contained, it is preferred if the total quantity of glycol distearate is in the range from 0.1 to 0.9% by weight, calculated to the total weight of the composition.

According to the invention it is further preferred if the composition has a pH value in the range from 4.5 to 8. The pH may be adjusted using citric acid or sodium hydroxide.

### Examples:

The following examples should illustrate the compositions and the use of this invention, without intending to limit the invention to these examples. The numerical values in the examples are percentages by weight, based on the total weight of the preparations.

The following formulations were prepared:

| **INCI Name** | **Ex. 1** | **Ex. 2** | **Ex. 3** | **Ex. 4** | **Ex. 5** | **Ex. 6** | **Ex. 7** | **Ex. 8** | **Ex. 9** | **Ex. 10** |
|---|---|---|---|---|---|---|---|---|---|---|
| Sodium Laureth Sulfate | 7 | 6.5 | 6 | 5.55 | 5 | 2 | 3 | 3 | 3 | 3 |
| Sodium Myreth Sulfate | | | | | | 3 | 3 | 2.5 | 1.5 | 3.5 |
| Cocamidopropyl Betaine | 3.2 | 3.53 | 3.7 | 4.53 | 5 | 3.55 | 3.53 | 3.53 | 2.55 | 2.55 |
| Coco Glucoside | 0.8 | 1.2 | 1 | 1.1 | 1.2 | 1 | 1.2 | 1 | 1.2 | 0.8 |
| Sodium Chloride | 0.2 | 0.1 | 0.15 | 0.09 | 0.2 | 0.4 | 0.4 | 0.4 | 0.5 | 0.45 |
| Citric Acid | 0.45 | 0.45 | 0.45 | 0.45 | 0.45 | 0.45 | 0.45 | 0.45 | 0.45 | 0.45 |
| Sodium Benzoate | 0.45 | 0.45 | 0.45 | 0.45 | 0.45 | 0.45 | 0.45 | 0.45 | 0.45 | 0.45 |
| Glycol Distearate | 0.6 | 0.6 | 0.6 | 0.6 | 0.6 | 0.6 | 0.6 | 0.6 | 0.6 | 0.6 |
| One of the perfume ingredient X1 to X28 | 0.7 | 1.1 | 0.9 | 0.3 | 0.8 | 1.5 | 1.0 | 0.6 | 0.9 | 1.2 |
| Aqua | add 100 | add 100 | Add 100 | add 100 | add 100 | add 100 | add 100 | add 100 | add 100 | add 100 |

The following parfum ingredients X1 to X28 were contained in the formulations (all % values in weight %):

| Perfume ingredient | Ingredient |
|---|---|
| X1 (Floral) | Mixture containing Phenetyl Alcohol, Dihydromyrcenol, alpha Terpineol, Damascone cis alpha, Linalyl Acetate, Beta lonone, Geranyl Acetate and Ethylene Brassylate |
| X2 (coconut) | Mixture containing Aldehyd C18, Aldehyd C14 and gamma decalactone |
| X3 (orange) | Mixture containing dextro-Limonene, Methyl Anthranilate, Aldehyd C8, Blood Orange Oil and Bitter Orange Peel Oil |
| X4 (jasmine) | Mixture containing Dihydrojasmone, Dihydrojasmone Lactone, Methyldihydrojasmonate and Ylang Ylang Flower Oil |
| X5 (sandalwood) | Mixture containing Sandal Pentanol, Sandal Pentenol and Sandal Octanol. |
| X6 (vanilla) | Mixture containing Vanillin, Heliotropin and Ethylvanillin. |
| X7 (amber) | Mixture containing Musk Amberol, Patchouli Ethanone, Acetyl Cedrene, Sclareol and Musk Tetralin |
| X8 (Floral) | Phenetyl Alcohol |
| X9 (coconut) | Aldehyd C18 |
| X10 (orange) | Bitter Orange Peel Oil |
| X11 (jasmine) | Ylang Ylang Flower Oil, |
| X12 (sandalwood) | Sandal Pentanol |
| X13 (vanilla) | Ethylvanillin |
| X14 (amber) | Musk Amberol |
| X15 (Floral) | 25% Phenetyl Alcohol, 20% Dihydromyrcenol 10% alpha Terpineol, 5% Linanyl Acetate, 5% Geranyl Acetate, 5% Beta lone, 30% Solventmixture I |
| X16 (coconut) | 28% gamma decalactone, 32% Aldehyd C18, 40% Solventmixture I |
| X17 (orange) | 30% dextro-Limolnene, 20% Methyl Anthranilate, 20% Aldehyde C8, 6% Blood Orange Oil, 24% Solventmixture I |
| X18 (jasmine) | 20% Dihydrojasmone, 18% Dihydrojasmone Lactone, 15% Methyldihydrojasmonate and 20% Ylang Ylang Flower Oil 27% Solventmixture I |
| X19 (sandalwood) | 32% Sandal Pentanol, 28% Sandal Pentenol, 10% Sandal Octanol, 30% Solventmixture I |
| X20 (vanilla) | 40% Vanillin, 25% Ethylvanillin, 35% Solventmixture I |
| X21 (amber) | 30% Musk Amberol, 25% Musk Tetralin, 20% Patchouli Ethanone, 25% Solventmixture I |
| X22 (Floral) | 25% Phenetyl Alcohol, 20% Dihydromyrcenol 10% alpha Terpineol, 5% Linanyl Acetate, 5% Geranyl Acetate, 5% Beta lone, 30% Solventmixture II |
| X23 (coconut) | 28% gamma decalactone, 32% Aldehyd C18, 40% Solventmixture I |
| X24 (orange) | 30% dextro-Limolnene, 20% Methyl Anthranilate, 20% Aldehyde C8, 6% Blood Orange Oil, 24% Solventmixture II |
| X25 (jasmine) | 20% Dihydrojasmone, 18% Dihydrojasmone Lactone, 15% Methyldihydrojasmonate and 20% Ylang Ylang Flower Oil 27% Solventmixture II |
| X26 (sandalwood) | 32% Sandal Pentanol, 28% Sandal Pentenol, 10% Sandal Octanol, 30% Solventmixture II |
| X27 (vanilla) | 40% Vanillin, 25% Ethylvanillin, 35% Solventmixture II |
| X28 (amber) | 30% Musk Amberol, 25% Musk Tetralin, 20% Patchouli Ethanone, 25% Solventmixture II |

Solventmixture I comprises 95% Dipropylene Glycol, 5% Triethyl Citrate (all values by weight). Solventmixture II comprises 85% Dipropylene Glycol, 10% Hexylene Glycol, 3% Isopropylmyristate, 2% Triacetin (all values by weight).

Instead of one or more of the perfume ingredients X1 to X28 comparative formulations were prepared containing either no perfume or a perfume having an aquatic ozonic sent comprising 7-Methyl-2H-benzo-1,5-dioxepin-3(4H)-one (Calone), Aldehyde C12, Cascalone and Helional. Further formulations as those of Ex.1 to Ex.21 were prepared without a perfume ingredient.

All formulations were packed in a bottle (container) made of post-consumer recycled polyethylene. Afterwards the bottle was closed using a cap and stored for at least 4 weeks at a temperature of 40 C.

After storing the cap was opened and the smell of the headspace was analyzed by an expert panel. It was found that the smell with the bottle stored with no perfume ingredient had significant malodors. These malodors can be covered by using formulations according to the invention.

Further formulations, which can be included in the bottle (container) of the invention are the following:
Further formulations are indicated in the table below:

| **Ingredients** | **Ex.11** | **Ex.12** | **Ex.13** | **Ex.14** |
|---|---|---|---|---|
| Glycine Soja Oil | 5 | 5 | | 0.01 |
| Prunus Amygdalus Dulcis Oil | | | | 0.01 |
| Helianthus Annuus Seed Oil | | | | 0.01 |
| PPG-15 Stearyl Ether | | | | |
| Paraffinum Liquidum | | | | |
| Glycerin | | | 1.7 | 0.6 |
| Sodium Hydroxide | | | 0.2 | |
| Glyceryl Stearate SE | 2 | 2 | | |
| Glyceryl Stearate | 1 | 1 | | |
| Mixture containing Arachidic Acid (1.5%); Myristic Acid (2.5%); Oleic Acid (0.5%); Palmitic Acid (48.5%); Stearic Acid (47%) | 5 | 5 | | 0.01 |
| Citric Acid | 0.5 | 0.5 | | |
| Glycol Distearate | | | | 1.2 |
| Laureth-4 | | | | 0.6 |
| Ethylparaben | | | 0.35 | |
| Sodium Chloride | | | | 0.65 |
| Sodium Benzoate | 0.4 | 0.4 | | 0.45 |
| Phenoxyethanol | | | 0.9 | |
| Methylparaben | | | 0.35 | |
| Cetearyl Alcohol | 2 | 2 | | |
| Xanthan Gum | 0.5 | 0.5 | | |
| One of the Perfume ingredients X1 to X28 | 0.2 | 0.5 | 0.3 | 1 |
| PEG-200 Hydrogenated Glyceryl Palmate | | | 0.5 | |
| Acrylates Copolymer | | | 2.1 | |
| Aqua | Ad 100 | Ad 100 | Ad 100 | Ad 100 |
| Sodium Laureth Sulfate | 7.58 | 3 | 0.01 | 6.5 |
| Sodium Myreth Sulfate | | | 0.9 | |
| Lauryl Glucoside or Cocoyl Glucoside or Decyl Glucoside | | | 1.1 | 1.02 |
| Cocamidopropyl Betaine | 3.33 | 3.2 | 4.1 | 3.5 |

## Claims

1. Cosmetic product comprising
a) a container comprising at least 80% by weight post-consumer recycled plastic, based on the total weight of the container, whereby the container has at least one opening,
b) a cosmetic cleansing composition situated in the container **characterized in that** the composition comprises
- a fragrance having a floral family scent and
- at least one surfactant.

2. Cosmetic product comprising
a) a container comprising at least 80% by weight post-consumer recycled plastic, based on the total weight of the container, whereby the container has at least one opening,
b) a cosmetic cleansing composition situated in the container **characterized in that** the composition comprises
- a fragrance having a scent of coconut, orange, jasmine, sandalwood, vanilla and/or amber and
- at least one surfactant.

3. Cosmetic product according to claim 1 or 2 **characterized in that** the container comprises at least 85% by weight, more preferably 90% by weight, more preferably 95% by weight, more preferably 98% by weight, more preferably 99% by weight and most preferably 100% by weight post-consumer recycled plastic, calculated to the total weight of the container.

4. Cosmetic product according to any of the proceeding claims **characterized in that** virgin petroleum-based materials are not contained in the container.

5. Cosmetic product according to any of the proceeding claims **characterized in that** the container is composed of at least about 30% by weight, preferably at least about 50% by weight, more preferably at least about 65% by weight, even more preferably at least about 85% by weight, more preferably at least about 95% by weight or about 100% by weight of polyethylene (PE), based on the total weight of the container.

6. Cosmetic product according to any of the proceeding claims **characterized in that** the container is composed of at least about 25% by weight, preferably at least about 35% by weight, more preferably at least about 50% by weight, even more preferably at least about 75% by weight, preferably at least about 95% by weight or preferably about 100% by weight of polypropylene (PP), based on the total weight of the container.

7. Cosmetic product according to any of the proceeding claims **characterized in that** the ratio by weight between polyethylene and polypropylene is in the range from 1000:1 to 1:1000, preferably from 500:1 to 1:500 and most preferably from 100:1 to 1:100.

8. Cosmetic product according to any of the proceeding claims **characterized in that** the fragrance having a floral family scent is created by containing one or more of the ingredients selected from the group consisting of (-)-alpha-terpineol; (+)-alpha-terpineol; (+)-nerolidol; (±)-2,3-dihydrofarnesol; (±)-2,4,8-trimethyl-7-nonen-2-ol; (±)-3-((2-methyl-3-furyl)thio)-2-butanone; (±)-3-methyl-gamma-decalactone; (±)-4-ethyl octanal; (±)-8,9-dehydrotheaspirone; (±)-lilac aldehyde; (2-methoxy-1-methyl butyl) benzene; (2-methoxy-1-methyl propyl) benzene; (2S)-1-isopropoxy-1-oxopropan-2-yl pivalate; (3-methoxy-2-methyl propyl) benzene; (3S,6S)-(Z)-linalool oxide (pyranoid); (4E,9Z)-13-methyloxacyclopentadeca-4,9-dien-2-one; (E)-2,5,9-trimethyl-4,9-decadien-1-al; (E)-2-hexen-1-yl salicylate; (E)-2-undecen-1-ol; (E)-3-hexen-1-ol; (E)-4-undecenal; (E)-5-decen-1-ol; (E)-7-methyl-3-octen-2-one; (E)-alpha-amyl cinnamaldehyde; (E)-alpha-damascone; (E)-alpha-methyl ionone; (E)-beta-damascone; (E)-beta-ionone; (E)-beta-methyl ionone; (E)-cinnamophenone; (E)-cinnamyl acetate; (E)-citronellyl tiglate; (E)-ethyl tiglate; (E)-geranyl linalool; (E)-isoeugenol; (E)-linalool oxide (furanoid); (E)-nerolidol; (E)-para-methoxycinnamaldehyde; (E)-squalene; (E)-trimethyl phenyl butenone; (E,E)-2,4-hexadienal; (E,E)-2,6-alloocimene; (E,E)-digeranyl ether; (E,E)-farnesol; (E+Z)-4,8-dimethyl-3,7-nonadien-2-one; (R)-(+)-dihydro-alpha-ionone; (R)-1-phenyl propyl alcohol; (R)-2-phenyl propionaldehyde; (R)-2-phenyl propyl alcohol; (R)-citronellyl nitrile; (R)-dihydro-beta-ionol; (R)-dihydro-gamma-ionone; (R)-hydroxycitronellal; (R)-linden ether; (R)-styralyl acetate; (R)-styralyl alcohol; (S)-(-)-dihydro-alpha-ionone; (S)-1-phenyl propyl alcohol; (S)-2,5,6-trimethyl-2-heptanol; (S)-2-phenyl propionaldehyde; (S)-2-phenyl propyl alcohol; (S)-campholene acetate; (S)-dihydro-beta-ionol; (S)-dihydro-gamma-ionone; (S)-hydroxycitronellal; (S)-ocean propanal; (S)-rhodinol; (Z)-2-octen-1-ol; (Z)-3-hexen-1-yl acetoacetate; (Z)-3-hexen-1-yl angelate; (Z)-3-hexen-1-yl benzoate; (Z)-3-hexen-1-yl methyl carbonate; (Z)-3-hexen-1-yl salicylate; (Z)-4-decen-1-yl acetate; (Z)-4-hepten-2-yl salicylate; (Z)-8-tetradecenal; (Z)-8-undecenal; (Z)-9-undecene nitrile; (Z)-alpha-amyl cinnamaldehyde; (Z)-alpha-damascone; (Z)-beta-damascone; (Z)-beta-ocimene; (Z)-cinnamyl acetate; (Z)-jasmone; (Z)-linalool oxide (furanoid); (Z)-methyl epi-jasmonate; (Z)-nerolidol; (Z)-woody amylene; (Z,E)-phytol; [(4E,4Z)-5-methoxy-3-methyl-4-penten-1-yl] benzene; 1-(2-methyl allyl oxy)-2-methyl butane; 1-(2-methyl allyl oxy)-3-methoxy-3-methyl-1-butane; 1-(octahydro-4,7-methanoinden-5-yl)-propan-2-ol; 1-(octahydro-4,7-methanoinden-5-yl)-propan-2-one + 1-(6-methyloctahydro-4,7-methanoinden-5-yl)-ethanone; 1,3,3,5-tetramethyl-7 and 8-cyanobicyclo(2.2.2)oct-5-ene; 1,4-diisopropyl-6,8-dioxabicyclo(3.2.1)octane; 1,8-octane dinitrile; 1-[(3aS,7aR)-3a,4,7,7a-tetrahydro-1H-inden-6-yl]ethanone; 10-epi-gamma-eudesmol; 10-undecen-1-ol; 1-acetyl cyclohexyl acetate; 1-acetyl indole; 1-isobutyl-2-methyl butyl 2-butenoate; 1-octen-3-yl butyrate; 1-phenethyl mercaptan; 1-phenyl propyl alcohol; 1-phenyl propyl butyrate; 1-undecanol; 2-(2-cyanoethylidene)-3-methyl bicyclo(2.2.1)hept-5-ene; 2,2'-oxamidobis[ethyl-3-(3,5-di-tert-butyl-4-hydroxyphenyl)-propionate; 2,4,6-trimethyl-6-(3-methyl-2-buten-1-yl)-2-cyclohexen-1-one; 2,4-difurfuryl furan; 2',4'-dimethyl acetophenone; 2,4-dimethyl cyclohexyl methyl acetate; 2,4-dimethyl-3-cyclohexene-1-methanol; 2,4-dimethyl-3-cyclohexene-1-methanyl acetate; 2,4-dimethyl-alpha-allyl-3-cyclohexene methanol; 2,4-ivy carbaldehyde; 2,5,6-trimethyl-2-heptanol; 2,5-dimethyl-2-indan methanol; 2,6-dimethyl-2,18-nonadecadien-8-one; 2-acetyl-3-methyl thiophene; 2-butyl thiophene; 2-decalinyl acetate; 2-decalinyl formate; 2-decanone; 2-decenal; 2-dodecanone; 2-ethyl butyl 2-butenoate; 2-ethyl octine carbonate; 2-ethyl-1-hexanol; 2-ethyl-4-methyl-1,3-oxathiane; 2-ethylidene-6-methyl-cis-3-heptenal; 2-heptanol; 2-heptyl cyclopropane carboxylic acid; 2-heptyl pyridine; 2-heptyl tetrahydrofuran; 2-hexyl thiophene; 2-hexyl-4-acetoxytetrahydrofuran; 2-isopropoxyethyl salicylate; 2-methyl butyl angelate; 2-methyl butyl salicylate; 2-methyl naphthalene; 2-methyl octanal; 2-methyl-4-phenyl butanal; 2-methyl-4-phenyl-1,3-dioxolane; 2-naphthyl phenyl ketone; 2-nonanone oxime; 2-nonanone propylene glycol acetal; 2-octanone oxime; 2-para-cresyl ethanol; 2-para-cresyl oxyethyl acetate; 2-pentyl cyclopentan-1-ol; 2-phenyl propionaldehyde dimethyl acetal; 2-phenyl propionaldehyde ethylene glycol acetal; 2-phenyl propyl isobutyrate; 2-phenyl-2-pentenal; 2-phenyl-4-pentenal; 2-tetradecenal; 2-undecanone; 2-undecen-1-ol; 3-(3-propen-2-yl phenyl) butanal; 3-(3-tert-butyl phenyl) propanal; 3-(4-hydroxy-4-methyl cyclohexyl) butanal; 3-(4-isobutyl-2-methylphenyl)propanal; 3-(4-propyl phenyl) propionaldehyde; 3-(5-methyl-2-furyl) butanal; 3-(benzyl oxy)-2,2-dimethyl propanal; 3',4'-dimethoxyacetophenone; 3,4-dimethoxystyrene; 3,5,5-trimethyl hexanol; 3,6-dimethyl-3-octyl acetate; 3,6-dimethylheptan-2-ol; 3,7-dimethyl-6-octenoic acid; 3-[(4R)-4-(propan-2-yl)cyclohex-1-en-1-yl]propanal; 3-allylthio-2,6-dimethyl-4-heptanone; 3-crotylthio-2,6-dimethyl-4-heptanone; 3-cyclohexyl propyl acetate; 3-decanol; 3-decanone; 3-heptyl acetate; 3-isopropenyl-6-oxoheptanoic acid; 3-mercaptohexyl acetate; 3-methyl pentyl angelate; 3-methyl-2-hexen-1-ol 1-acetate; 3-methyl-4-(2-methyl butyl oxy) butyraldehyde; 3-methyl-4-hexyl oxybutyraldehyde; 3-methyl-6-ethyl-5-octen-1-ol; 3-nonanon-1-yl acetate; 3-pentyl bicyclo[3.2.1]octan-2-one; 3-phenyl propyl formate; 3-phenyl propyl propionate; 3-propyl bicyclo(3.2.1)-6-octen-2-one; 4-(1,1,2,3,3-pentamethyl indan-5-yl) pyrimidine; 4-(2,3-dihydro-1,1,2,3,3-pentamethyl-1H-inden-5-yl)pyrimidine; 4-(para-tolyl)-2-butanone; 4,6,11-trimethyl-5-oxatricyclo(6.2.2.0*4,9*)dodec-6-ene; 4,6-dimethyl-alpha-allyl-3-cyclohexene methanol; 4,8-dimethyl-7-nonen-2-ol; 4-cyclohexyl cyclohexanone; 4-cyclopentyl pentane nitrile; 4-dimethyl ionone; 4-ethoxy-3-anisaldehyde; 4-ethyl cyclohexane propanal; 4-hydroxyphenethyl alcohol; 4-isobutyl cyclohexane propanal; 4-mercapto-4-methyl-2-hexanone; 4-mercapto-4-methyl-2-pentanol; 4-methoxy-alpha-toluene thiol; 4-methyl biphenyl; 4-methyl cyclohexane propanal; 4-methyl-2-(2-methyl-1-propenyl)-1,3-dioxane; 4-methyl-3-pentyl-2(5H)-furanone; 4-methyl-4-phenyl pentanone; 4-methyl-4-phenyl-2-pentanol; 4-methyl-8-vinyl-4,7-nonadienal; 4-methyl-8-vinyl-4,8-nonadienal; 4-phenyl-2-butanol; 4-phenyl-3-buten-2-ol; 4-propyl cyclohexane propanal; 4-tert-butyl cyclohexane carboxaldehyde; 5-tricyclodecenyl acetate; 6,7,8-decen-1-ol; 6,8-dimethyl-2-nonanol; 6-methyl coumarin; 7-allyl benzo[b][1,4]dioxepin-3-one; 8,9-dehydrotheaspirone; 8-methylnonanal; 9-[9,12-epoxy-ethyl]-5-methyl-tricyclo[6.2.1.0.sup.2,7 ]undec-4-ene; 9-decenal; abrialis lavandin oil; acetal 318; acetaldehyde dibutyl acetal; acetaldehyde ethyl phenethyl acetal; acetyl tetralin; achillea millefolium herb oil CO2 extract; adoxal; alloocimene; allyl acetone; allyl anthranilate; allyl benzoate; alpha-acorenol; alpha-allyl-4,6-dimethyl-3-cyclohexene-1-methanyl acetate; alpha-allyl-4-methyl-3-cyclohexene-1-methanyl acetate; alpha-amyl cinnamaldehyde; alpha-amyl cinnamaldehyde / methyl anthranilate schiffs base; alpha-amyl cinnamaldehyde diethyl acetal; alpha-amyl cinnamyl acetate; alpha-amyl cinnamyl alcohol; alpha-bisabolene; alpha-bisabolol; alpha-butyl cinnamaldehyde; alpha-damascone; alpha-decalactone; alpha-hexyl cinnamaldehyde; alpha-ionol; alpha-ionone; alpha-ionone; alpha-ionyl acetate; alpha-irone; alpha-isomethyl ionone; alpha-methyl cinnamyl alcohol; alpha-methyl ionone; alpha-ocimene; alpha-terpineol; amber dioxane; amyl angelate; amyl benzoate; amyl cyclopentanone propanone; amyl isoeugenol; amyl nonanoate; amyl salicylate; aniba rosaeodora var. amazonica wood extract; anise indene; anisyl propanal / methyl anthranilate schiff's base; anthocephalus cadamba oil; atlas cedarwood oil fractions; autumn carboxylate; benzaldehyde / methyl anthranilate schiff's base; benzyl acetate; benzyl acetoacetate; benzyl acetone; benzyl alcohol; benzyl anthranilate; benzyl cinnamate; benzyl eugenol; benzyl formate; benzyl lactate; benzyl methyl ether; benzyl phenyl acetate; benzyl phenyl carbinol; benzyl pivalate; benzyl propionate; benzyl valerate; benzylidene acetone; bergamot acetoacetate; bergamot oil; berry hexanoate; beta-bisabolol; beta-bourbonene; beta-damascone; beta-ionol; beta-ionone; beta-isomethyl ionone; beta-methyl ionone; beta-naphthyl anthranilate; beta-naphthyl ethyl ether; beta-naphthyl methyl ether; beta-phenoxyethyl acetate; bitter orange peel oil ; bois de rose oil ; brachyleana hutchinsii wood oil; butyl 2-naphthyl ether; butyl anthranilate; butyl benzyl ether; butyl para-cresol ether; butyl tiglate; butyl undecylenate; cabreuva wood oil; calendula officinalis flower oil ; callitris intratropica wood oil ; callitropsis araucarioides wood oil; camellia oleifera leaf extract; cananga fruit oil; cananga leaf oil; cananga odorata flower extract; cananga oil; cardamom seed oil ; carrot seed oil ; cedarwood oil ; cedarwood oil terpenes; cerastium candidissimum corr. oil ; cestrum nocturnum flower oil; chamomile isobutyrate; chamomile propionate; chamomile valerate; chrysanthemum ketone; chrysanthemum oxide; cinnamyl acetate; cinnamyl cinnamate; cinnamyl formate; cis-muguet shiseol; cis-woody acetate; citral dimethyl acetal; citral ethylene glycol acetal; citronellal; citronellol; citronellone; citronellyl (R)-lactate; citronellyl acetate; citronellyl acetone; citronellyl butyrate; citronellyl ethyl ether; citronellyl hexanoate; citronellyl isobutyrate; citronellyl nitrile; citronellyl oxyacetaldehyde; citronellyl propionate; citronitrile; citrus ocimenol; citrus reticulata blanco var. mandarin oil ; clary propionate; clary propyl acetate; convolvulus scoparius wood oil; coranol; coriander oleoresin; coriander seed oil; corps popinal; cumin carbinol; cuminyl acetaldehyde; cyclamen aldehyde; cyclamen homoaldehyde; cyclamen propanal; cyclohexyl benzoate; cyclohexyl butyrate; cyclohexyl crotonate; cyclohexyl ethyl alcohol; cyclohexyl propanol; cyclohexyl salicylate; cymbopogon validus leaf oil; damascate; daniellia oliveri bark oil ; decanal ; decanal methyl enol ether; decanal propylene glycol acetal; decanol; decyl anthranilate; dehydrodihydroionone; dextro-linalool; dianthus ethone; diethyl acetonyl diisopropyl acetonyl sulfide; dihexyl (E)-fumarate; dihydro-alpha-ionone; dihydro-beta-ionol; dihydrocarvyl acetate; dihydrodamascone; dihydrofarnesol; dihydrogeranyl linalool; dihydroisojasmonate methyl ester; dihydromyrcenol; dihydroterpineol; diisobutyl carbinyl acetate; dimethyl alpha-ionone; dimethyl benzyl carbinol; dimethyl benzyl carbinyl acetate; dimethyl benzyl carbinyl butyrate; dimethyl glutarate; dimethyl succinate; diphenyl amine; dodecanal (aldehyde C-12 lauric); dryopteris filix-mas oleoresin; earthy acetal; earthy indane; epoxylinalyl acetate; eriocephalus punctulatus flower oil; eschweilera coriacea mori flower oil ; ethyl (E)-cinnamate; ethyl 2-benzyl butyrate; ethyl 2-furoate; ethyl 3,5,5-trimethyl hexanoate; ethyl 3-hydroxyoctanoate; ethyl 4-methyl salicylate; ethyl anthranilate; ethyl cyclohexyl acetate; ethyl laurate; ethyl levulinate; ethyl linalyl acetal; ethyl linalyl acetate; ethyl linalyl ether; ethyl methyl-para-tolyl glycidate; ethyl ortho-anisate; ethyl para-methyl-beta-phenyl glycidate; ethyl phenoxyacetate; ethyl phenyl acetate; ethyl salicylate; ethylene brassylate; ethylene glycol diacetate; eucalyptus citriodora oil; eugenyl acetate; exo-isocitral; farnesal; farnesol; farnesyl acetate; farnesyl acetone; farnesyl palmitoleate; fokienol; freesia heptanol; fruity carboxylate; furfuryl isopropyl sulfide; gamma-damascone; gamma-ionone; gardenia concrete; georgywood; geraniol; geranium dihydropyran; geranium leaf oil; geranium oil; geranyl acetate; geranyl acetone; geranyl heptanoate; geranyl isobutyrate; geranyl linalool; geranyl phenyl acetate; geranyl propionate; geranyl undecylenate; ginsene; globulol; glycoacetal; grapefruit pentanol; guaiacyl phenyl acetate; hawthorn acetate; hawthorn carbinol; hawthorn ethanol; helichrysum bracteatum flower oil; heliotropyl acetate; heliotropyl diethyl acetal; heptyl butyrate; heptyl formate; heptyl isobutyrate; heptyl nonanoate; herbal pyran; herniaria incana lam. oil ; hexadecanol; hexanal diethyl acetal; hexen-1-yl oxypropane nitrile; hexenyl cyclopentanone; hexoxyacetaldehyde dimethyl acetal; hexyl (Z)-tiglate; hexyl 2-furoate; hexyl lactate; hexyl oxyacetonitrile; hexyl pivalate; hibiscus sabdariffa flower extract; ho leaf oil; ho wood oil; homalomena rubescens root oil; homoambrinol; homoanisaldehyde; homomenthyl acetate; homovanillin; honey extract; hyacinth acetals; hyacinth ether; hydroxycitronellal; hydroxycitronellal / methyl anthranilate schiffs base; hydroxycitronellal diethyl acetal; hydroxycitronellal propylene glycol acetal; hydroxycitronellal residue; hydroxycitronellol; ilex paraguariensis oleoresin; immortelle flower oil; immortelle flower resinoid; indocolore ; indole; intreleven aldehyde; inula root oil; ionone mixed isomers; iris germanica florentina root extract; isoamyl 3-(2-furan) propionate; isoamyl angelate; isoamyl cinnamate; isoamyl nonanoate; isoamyl salicylate; isoamyl undecylenate; isobutyl anthranilate; isobutyl benzyl carbinol; isobutyl citral; isobutyl furyl propionate; isobutyl octanoate; isobutyl phenyl acetate; isobutyl salicylate; isobutyraldehyde; isocyclodimethyl octanol; isocyclogeraniol ; isodecanal; isoeugenol; isoeugenyl acetate; isoeugenyl ethyl acetal; isoeugenyl phenyl acetate; isogreen methanoindene; isojasmone; isojasmone; isophytol; isopropyl anthranilate; isopropyl benzoate; ivy carbaldehyde / methyl anthranilate schiff'sbase; ivy dioxolane; jambu flower oil brazil; jasimia; jasmin acetate; jasmin cyclopentanol; jasmin lactone ; jasmin oil; jasmin pyranol; jasminum grandiflorum extract; jasminum grandiflorum flower extract; jasmopyrane ; ketaki flower oil; kunzea ericoides leaf oil; laevo-citronellol; laevo-linalool; laevo-menthyl acetate; laevo-menthyl propionate; laevo-rose oxide; lavandin concrete; lavandin flower water; lavandin oil; lavandula angustifolia flower oil; lavender concrete; lavender oil; lavender oil bulgaria; lavender oil france; lavender oil greece; lavender oil terpeneless; lavender stem oil lithuania; lawsonia inermis flower oil; lecythis usitata miers. var. paraensis (ducke) r. kunth. flower oil; leerall; leerall / methyl anthranilate schiff's base; lepidine; leptospermum scoparium branch/leaf oil; lily propanol; lilyall; lime octadienal; lime oil ; linaloe wood oil; linalool; linalool oxide; linalyl acetate; linalyl acetate terpenes; linalyl benzoate; lonicera japonica flower extract; mace oleoresin; magnolia cyclohexanol; magnolia decadienal; magnolia flower oil ; magnolia indene; malpighia glabra fruit oil; mandarin oil; mandarin oil; marigold pot flower oil; marine decadienal; matricaria chamomilla flower oil ; melaleuca ericifolia leaf oil; melozol acetate; menthadienyl formate; meta-cresyl acetate; meta-cresyl phenyl acetate; meta-cresyl phenyl ether; methoxycitronellal; methoxymelonal; methyl (Z)-3-hexenoate; methyl (Z)-jasmonate; methyl 10-undecenoate; methyl 2-methyl benzoate; methyl 2-undecynoate; methyl 4-methyl benzoate; methyl 4-phenyl butyrate; methyl benzoate; methyl benzyl acetate; methyl bicycloheptenyl methyloxirane carboxylate; methyl citronellate; methyl cyclocitrone; methyl cyclogeranate; methyl decanoate; methyl dihydrojasmonate; methyl heptanoate; methyl hydrocinnamate; methyl ionyl acetate; methyl isobutyrate; methyl nerate; methyl octine carbonate; methyl ortho-anisate; methyl phenoxyethanol; methyl phenyl acetate; methyl trans-3-oxo-2-pentyl cyclopentane carboxylate; methyl undecylenate; mimosa heptanal; mimosa tenuiflora leaf extract; muguet butanal; muguet butanol; muguet carbaldehyde; muguet carboxaldehyde; muguet dienal; muguet ethanol; muguet nitrile; muguet octadienol; muguet propanol; muguet shiseol; muguet undecadienal; musk acetate; musk dec-8-enone; myrascone; myrcenol; myrcenyl acetate; myristica fragrans fruit extract; myrtle oil; myrtle oil morocco; narcissus acetate; narcissus jonquilla extract; neroli extract; neroli ketone; neroli oil; neroli oil bigarde; nerolidol; nerolidol oxide; nerolidyl propionate; nerolione; neryl acetate; neryl benzoate; neryl isovalerate; neryl propionate; nonanal / methyl anthranilate schiffs base; nonanal diethyl acetal; nonanal dimethyl acetal; nonanol; nonisyl propionate; nonyl octanoate; ocean carboxaldehyde; ocean propanal / methyl anthranilate schiffs base; ocimen-1-yl acetate; octahydro-4,7-methano-1H-indene-5-acetaldehyde; octahydro-4,7-methano-1H-indene-5-acetaldehyde + 6-methyl-octahydro-4,7-methano-indene-5-carbaldehyde; octanal / methyl anthranilate schiff's base; octanal dimethyl acetal; octyl isovalerate; octyl oxyacetaldehyde; octyl propionate; O-ethyl S-(2-furyl methyl) thiocarbonate; origanum majorana leaf oil; orris butenone; orris pyridine; orris rhizome concrete butter (iris pallida); orris rhizome oil (iris germanica); orris rhizome oil; orris rhizome resinoid (iris pallida); ortho-acetyl-para-cresol; ortho-cresyl acetate; ortho-methoxybenzyl ethyl ether; ortho-methyl anisole; palmarosa oil; papaya isobutyrate; para-anisaldehyde; para-anisaldehyde dimethyl acetal; para-anisyl alcohol; para-anisyl butyrate; para-anisyl formate; para-anisyl nitrile; para-cresyl alcohol; para-cresyl isobutyrate; para-cresyl laurate; para-cresyl phenyl ether; para-ethyl acetophenone; para-jasmone; para-methoxy-alpha-methyl cinnamaldehyde; para-methoxycinnamaldehyde; para-methyl benzyl acetate; para-methyl hydratropaldehyde; para-methyl phenoxyacetaldehyde; para-tolualdehyde propylene glycol acetal; peach nitrile; peach pivalate; pentenyl cyclopentanone; peony acetonitrile; peony alcohol; perilla alcohol; perillyl acetate; persea americana fruit oil; petal pyranone; petitgrain bigarade oil; petitgrain cedrat oil; petitgrain lemon oil; petitgrain mandarin oil; petitgrain oil; petitgrain oil fractions; petitgrain oil ;petitgrain oil terpeneless; petitgrain oil terpenes; petitgrain tangerine oil; phenethyl acetate; phenethyl alcohol; phenethyl anthranilate; phenethyl benzoate; phenethyl butyrate; phenethyl heptanoate; phenethyl hexanoate; phenethyl isobutyrate; phenethyl isovalerate; phenethyl phenyl acetate; phenethyl propionate; phenethyl salicylate; phenoxyacetaldehyde ; phenoxyethyl isobutyrate; phenyl acetaldehyde; phenyl acetaldehyde / methyl anthranilate schiff's base; phenyl acetaldehyde 2,3-butylene glycol acetal; phenyl acetaldehyde dicitronellyl acetal; phenyl acetaldehyde digeranyl acetal; phenyl acetaldehyde diisoamyl acetal; phenyl acetaldehyde diisobutyl acetal; phenyl acetaldehyde dimethyl acetal; phenyl acetaldehyde; phenyl acetic acid; phenyl amyl alcohol; phenyl butyrate; phenyl ethylidene acetone; phenyl propyl valerate; phytol; phytyl acetate; piperidine; plum crotonate; plum damascone; prenyl salicylate; propiophenone; propyl phenyl acetate; propyl pyruvate; pseudoionone; raspberry ketone; rhodinol; rhodinyl acetate substitutes; rhodinyl benzoate; rhodinyl formate; rhodinyl phenyl acetate; rhubarb oxirane; rhubarb pyran; rosa alba flower oil; rosa damascena flower oil; rosa odorata flower oil; rose acetate; rose butanoate; rose oil (rosa centifolia) ; rose undecene; S-(2,5-dimethyl-3-furyl) ethane thioate; S-(methyl thio) hexanoate; safrole; salicynile; sambucus nigra flower extract; sambucus nigra flower oil ; sandal cyclopropane; sandal octanol; santalyl acetate; santalyl phenyl acetate; santolina oil; sec-butyl ethyl ether; siam wood oil; spicy acetoacetate; spicy carbonate; spike lavender oil; star fruit oil; styralyl butyrate; styralyl formate; styralyl propionate; styralyl valerate; sunflower oil; surfleurs d'oranger oil; sweet petitgrain oil; tagete oil madagascar; tangerine oil terpeneless; tarchonanthus camphoratus oil; terpineol acetate; terpineols; terpinyl benzoate; terpinyl butyrate; terpinyl formate; terpinyl isobutyrate; terpinyl valerate; tetrahydrofurfuryl phenyl acetate; tetrahydroionol; tetrahydroionyl acetate; tetrahydrolinalool; tetrahydrolinalyl acetate; tetrahydromyrcenol; tilia cordata flower oil; tonka ketone; tropical ionone; tuberose oil; undecanal; undecanal dimethyl acetal; undecanal propylene glycol acetal; vanilla tahitensis fruit oil; vassoura oil; verdyl acetate; vetiver pentanone; violet dienyne; violet methyl carbonate; violet nitrile; violet propanol; vitispirane; white frangipani concrete; white nelumbo nucifera flower concrete; white sassafras oil; witch hazel leaf oil; yarrow oil; ylang ylang flower concrete; ylang ylang flower oil; yuzu leaf oil; yuzunone and zdravetz oil.

9. Cosmetic product according to any of the proceeding claims **characterized in that** the composition comprises at least (Z) alpha-Damascone, (Z) beta-Damascone, Aldehyd C14, Citronellol, Hydroxycitronellal, dextro-Limonene, Aldehyd C8, Aldehyd C-10, Linalool, Linalyl Acetate, Methyl Anthranilate, Benzyl Acetate, Benzyl Alcohol, Benzyl Cinnamate, Geraniol, Geranyl Acetate, Vanillin, Ethyl Vanillin, Vanillin Isobutyrate, Eugenyl Acetate, Isoeugenyl Acetate, Patchouli Ethanone, Phenethyl alcohol, Amyl Salicylate, alpha lonone, (E) beta lonone, Heliotropin, Dihydromyrcenol, alpha-Terpineol, Sandal Pentanol, Sandal Pentenol, Sandal Octanol, Melozol Acetate, Phenoxyethyl Isobutyrate, gamma-Decalactone, gamma-Nonalactone, (Aldehyde C18), gamma-Octalactone, delta Nonalactone, Dihydrocoumarin, Nerolidol, Isoeugenol, Cinnamyl Acetate, Farnesol, alpha-Amyl Cinnamaldehyde, alpha-Isomethyl lonone, Indole, Z-Jasmone, Dihydrojasmone, Dihydrojasmone Lactone, Methyldihydrojasmonate, Musk Tetralin, Ethylene Brassylate, Tetradecanal, Musk Amberol, Acetyl Cedrene, Sclareol, Ylang-Ylang Flower Oil, White Jasmin Flower Oil, Blood Orange Oil and/or Bitter Orange Peel Oil.

10. Further, it is preferred if the total quantity of (Z) alpha-Damascone, (Z) beta-Damascone, Aldehyd C14, Citronellol, Hydroxycitronellal, dextro-Limonene, Aldehyd C8, Aldehyd C-10, Linalool, Linalyl Acetate, Methyl Anthranilate, Benzyl Acetate, Benzyl Alcohol, Benzyl Cinnamate, Geraniol, Geranyl Acetate, Vanillin, Ethyl Vanillin, Vanillin Isobutyrate, Eugenyl Acetate, Isoeugenyl Acetate, Patchouli Ethanone, Phenethyl alcohol, Amyl Salicylate, alpha lonone, (E) beta lonone, Heliotropin, Dihydromyrcenol, alpha-Terpineol, Sandal Pentanol, Sandal Pentenol, Sandal Octanol, Melozol Acetate, Phenoxyethyl Isobutyrate, gamma-Decalactone, gamma-Nonalactone, (Aldehyde C18), gamma-Octalactone, delta Nonalactone, Dihydrocoumarin, Nerolidol, Isoeugenol, Cinnamyl Acetate, Farnesol, alpha-Amyl Cinnamaldehyde, alpha-Isomethyl lonone, Indole, Z-Jasmone, Dihydrojasmone, Dihydrojasmone Lactone, Methyldihydrojasmonate, Musk Tetralin, Ethylene Brassylate, Tetradecanal, Musk Amberol, Acetyl Cedrene, Sclareol, Ylang-Ylang Flower Oil, White Jasmin Flower Oil, Blood Orange Oil and/or Bitter Orange Peel Oil is in the range from 0.0001 to 2.0% by weight, more preferably from 0.001 to 0.5% by weight and most preferably from 0.002 to 0.3% by weight calculated to the total weight of the composition.

11. Cosmetic product according to any of the claims 2 to 8 **characterized in** the composition comprises at least one ingredient selected from the group consisting of (-)-alpha-bisabolol; (-)-cis-whiskey lactone; (R)-gamma-decalactone; (R)-gamma-hexalactone; (R)-gamma-nonalactone; (R)-gamma-octalactone; (R)-massoia lactone; (R)-tonka furanone; (S)-gamma-decalactone; (S)-gamma-heptalactone; (S)-gamma-hexalactone; (S)-gamma-nonalactone; (S)-gamma-octalactone; (Z)-gamma-jasmolactone; (Z+E)-2-methyl cyclohexanol; 1-dodecanol; 2-acetyl-5-methyl furan; 2-heptanone; 2-pentyl cyclopentanone; 2-pentyl-2-cyclohexen-1-one; 2-tridecanone; 3-butyl bicyclo[3.2.1]octan-2-one; 4-hydroxybenzyl alcohol; 4-octen-3-one; 5,5-dibutyl dihydrofuran-2(3H)-one; 6-amyl-alpha-pyrone; 6-methyl coumarin; 7-methyl coumarin; alpha-decalactone; amyl heptanoate; cis-oak lactone; coconut decanone methyl; coconut naphthalenone; cocos nucifera fruit oil ; costus valerolactone; delta-2-dodecenolactone; delta-decalactone; delta-dodecalactone; delta-heptalactone; delta-hexalactone; delta-nonalactone; delta-octalactone; delta-undecalactone; dihydrocoumarin; dihydrojasmone lactone; dimethyl benzofuranone; ethyl undecanoate; gamma-decalactone; gamma-heptalactone; gamma-hexalactone; gamma-nonalactone (aldehyde C-18 (so-called)); gamma-octalactone; gamma-undecalactone (aldehyde C-14 (so-called)); glyceryl 5-hydroxydecanoate; glyceryl 5-hydroxydodecanoate; heliotropin; hexahydrocoumarin; hexenyl cyclopentanone; isoamyl octanoate; isopropyl 2-methyl butyrate; isopropyl octanoate; massoia bark oil; massoia lactone; methyl heptadienone; methyl laurate; octyl octanoate; para-vanillyl alcohol; petal pyranone; phthalide; propyl octanoate; tetrahydrojasmone; tonka furanone; trans-oak lactone; undecanoic acid; whiskey lactone and wine lactone.

12. Cosmetic product according to any of the claims 2 to 8 **characterized in** the composition comprises at least one ingredient selected from the group consisting of (E)-2-dodecenal; (E)-2-pentenal; (E)-2-undecenal; (E)-4-decenal; (E,E)-2,4-dodecadienal; (E,Z)-2,6-dodecadienal; (Z)-3-hexen-1-yl anthranilate; (Z)-4-decen-1-yl acetate; (Z)-4-decenal; (Z)-4-decenal diethyl acetal; (Z)-8-tetradecenal; 1-formyl-3-isohexenyl-3-cyclohexene; 2,4-decadienal; 2,4-dimethyl-alpha-isopropyl-3-cyclohexene-1-methanol; 2-decanone; 2-decenal; 2-dodecanone; 2-dodecenal; 2-naphthyl phenyl ketone; 2-nonanol; 2-pentenal; 2-pentyl-2-cyclohexen-1-one; 2-undecenal; 3-(methallylthio)-2,6-dimethyl-4-heptanone; 3-decanol; 3-decanone; 3-dodecenal; 3-methyl-4-heptyl oxybutyraldehyde; 4-decenal; 6-methyl heptanal; 6-methyl octanal; abies alba cone oil; acetyl tetralin; alpha-naphthyl methyl ketone; alpha-sinensal; alpha-terpinyl anthranilate; alpha-terpinyl methyl ether; bergamot oil; beta-naphthyl ethyl ether; beta-naphthyl methyl ether; beta-naphthyl methyl ketone; beta-sinensal; bitter orange peel oil; blood orange oil; butyl nonanoate; carica papaya seed oil; citral / methyl anthranilate schiff's base; citral propylene glycol acetal; citrus aurantium amara flower cera; cyclamen aldehyde / methyl anthranilate schiff's base; cyclohexyl anthranilate; decanal (aldehyde C-10); decanal / methyl anthranilate schiffs base; decanal diethyl acetal; decanol; decyl formate; dextro-limonene; dimethyl nonanal; dodecane nitrile; ethyl (E)-2-hexenoate; ethyl anthranilate; grapefruit oil ; heptanal dimethyl acetal; heptyl nonanoate; hydroxycitronellal / methyl anthranilate schiff's base; intreleven aldehyde; isooctanol; ivy carbaldehyde / methyl anthranilate schiffs base; labdanum ethanone; leerall / methyl anthranilate schiffs base; lilyall / methyl anthranilate schiff's base; linalyl anthranilate; mandarin oil; methyl 2-methyl benzoate; methyl anthranilate; methyl anthranilate / hexyl cinnamaldehyde schiffs base; methyl anthranilate / isocyclocitral schiffs base; methyl dimethyl anthranilate; methyl indole-1-carboxylate; methyl octanoate; michelia champaca flower oil; mimusops elengi flower oil; neroli oil; nerolione; neryl butyrate; nonanal (aldehyde C-9); nonanal / methyl anthranilate schiff's base; nonanol; nonyl anthranilate; nonyl benzoate; nonyl heptanoate; nonyl nonanoate; nyctanthes arbor-tristis flower oil; ocean propanal / methyl anthranilate schiff's base; octanal (aldehyde C-8); octanal glycol acetal; octanol; octyl formate; octyl phenyl acetate; phenethyl anthranilate; propyl anthranilate; rue flower oil; satinaldehyde; surfleurs d'oranger oil; sweet orange peel oil; tangerine oil; terpinyl isovalerate; turmeric root oil and valencene.

13. Cosmetic product according to any of the claims 2 to 8 **characterized in** the composition comprises at least one ingredient selected from the group consisting of (+)-methyl dihydroepijasmonate; (4E,9Z)-13-methyloxacyclopentadeca-4,9-dien-2-one; (R)-styralyl acetate; (Z)-3-hexen-1-yl anthranilate; (Z)-7-tetradecen-2-one; (Z)-jasmone; (Z,E)-phytol; 1-phenyl propyl butyrate; 2-(4-methyl cyclohexylidene) heptanonitrile; 2-hexenyl cyclopentanyl acetate; 2-hexylidene cyclopentanone; 2-methyl-4-phenyl-1,3-dioxolane; 2-para-cresyl oxyethyl acetate; 2-pentadecanone; 2-pentyl cyclopentan-1-ol; 2-pentyl cyclopentanone; 2-pentyl-2-cyclohexen-1-one; 3-butyl bicyclo[3.2.1]octan-2-one; 3-decen-2-one; 3-nonanone; 4-methyl-3-pentyl oxazolidin-2-one; 6-(5(6)-decenoyl oxy) decanoic acid; alpha-amyl cinnamaldehyde; alpha-amyl cinnamaldehyde diethyl acetal; alpha-amyl cinnamaldehyde dimethyl acetal; alpha-amyl cinnamyl acetate; alpha-amyl cinnamyl alcohol; alpha-butyl cinnamaldehyde; alpha-hexyl cinnamaldehyde; amyl cyclopentanone propanone; amyl cyclopentenone; autumn carboxylate; benzyl acetate; benzyl butyrate; benzyl hexanoate; benzyl isobutyrate; benzyl phenyl acetate; benzyl propionate; berry hexanoate; chrysanthemum oxide; creamy lactone; dicyclopentadiene propionate; dihydroisojasmonate methyl ester; dihydrojasmone; dihydrojasmone lactone; dimethyl benzyl carbinyl acetate; dimethyl benzyl carbinyl formate; dimethyl benzyl carbinyl isobutyrate; dimethyl benzyl carbinyl propionate; fruity cyclopentanone; gardenia oxide; gelsone; hawthorn ethanol; herbal carbonate; herbal pyran; hexahydrofarnesyl acetone; hexenyl cyclopentanone; hexyl (Z)-tiglate; indoletal; isogreen methanoindene; isojasmone; jasmin acetate; jasmin cyclopentanol; jasmin cyclopentanone; jasmin lactone; jasmin oil; jasmin pyranol; jasminum grandiflorum flower extract; jasminum officinale flower extract; jasminum sambac flower extract; jasminum sambac flower oil ; jasmopyrane ; methyl (Z)-jasmonate; methyl bicycloheptenyl methyloxirane carboxylate; methyl dihydrojasmonate; methyl jasmonate; methyl phenoxyethanol; methyl trans-3-oxo-2-pentyl cyclopentane carboxylate; nyctanthes arbor-tristis flower oil; octen-1-yl cyclopentanone; octyl butyrate; para-anisaldehyde dimethyl acetal; para-cresyl caprylate; para-jasmone; peach nitrile; pear valerate; pentenyl cyclopentanone; petal pyranone; prenyl acetate; prenyl tiglate; privet dioxane; pseudojasmone; rosacyanthin; styralyl butyrate; styralyl isobutyrate; styralyl valerate; tetrahydrojasmone; tricyclodecenyl propionate; waxy lactone; white jasmin flower oil and ylang ylang flower oil.

14. Cosmetic product according to any of the claims 2 to 8 **characterized in** the composition comprises at least one ingredient selected from the group consisting of (+)-alpha-santalyl acetate; (R)-sandal hexanol; 2-decalinyl formate; acetoxymethyl isolongifolene; agarwood oil; alpha-cedrene epoxide; alpha-ethyl-2,2,6-trimethyl cyclohexane propanol; alpha-santalol; amyris bark oil; beta,2,2,3-tetramethyl-delta-methylene-3-cyclopentene-1-butanol; brachyleana hutchinsii wood oil; cedarwood oil; geranium nitrile; indisan; melozol acetate; methyl sandal; muscomer; sandal butenol; sandal cyclohexanol; sandal cyclopentane; sandal cyclopropane; sandal glycol acetal; sandal hexanol; sandal octanol; sandal pentanol; sandal pentenol; sandal pentenone; sandalrome; sandalwood oil acetylated; sandel; santall; santalol; santol pentenol; timber propanol; vetiverol; vetiveryl formate and woody epoxide.

15. Cosmetic product according to any of the claims 2 to 8 **characterized in** the composition comprises at least one ingredient selected from the group consisting of (E)-para-methoxycinnamaldehyde; 1-benzoyl acetone; 2,4,6-triisobutyl-1,3,5-trioxane; 2,4-dimethyl benzaldehyde; 3-(2-furyl) acrolein; 4-ethoxy-3-anisaldehyde; 4-methyl guaiacol; 6-methyl coumarin; alpha-ethoxy-ortho-cresol; benzoin; cis-oak lactone; coniferaldehyde; coumane; curcuma amada roxb. rhizome oil; dianthus ethone; divanillin; ethyl vanillin; ethyl vanillin acetate; ethyl vanillin diethyl acetal; ethyl vanillin hexylene glycol acetal; ethyl vanillin isobutyrate; ethyl vanillin propylene glycol acetal; eugenol isoamyl ether; furfural acetone; geranyl ethyl acetal; glucoethyl vanillin; glucovanillin; guaiacyl propionate; heliotropin; heliotropyl alcohol; homovanillin; isopropyl vanillin; methyl vanillate; ortho-anisaldehyde; ortho-dimethyl hydroquinone; ortho-guaiacol; para-anisyl acetate; para-anisyl formate; para-cresyl laurate; paramethoxycinnamaldehyde; para-vanillic acid; para-vanillyl alcohol; peru balsam; peru balsam oil; powdery ketone; propenyl guaethol; quercus robur oil; sumatra benzoin gum; vanilla; vanilla aromatica fruit extract; vanilla bean oil (vanilla planifolia); vanilla bean oil (vanilla tahitensis); vanilla bean planifolia; vanilla bean tahitensis; vanilla cresol; vanilla oleoresin bali; vanilla oleoresin bourbon; vanillin; vanillin 2,3-butylene glycol acetal; vanillin hexylene glycol acetal; vanillin menthoxypropane diol acetal; vanillin propylene glycol acetal; vanillyl acetate; vanillyl butyl ether; vanillyl ethyl ether; vanillyl isobutyrate; vanillylidene acetone; vanilmandelic acid; veratraldehyde and zingerone.

16. Cosmetic product according to any of the claims 2 to 8 **characterized in** the composition comprises at least one ingredient selected from the group consisting of (- )-isolongifolene; (+)-alpha-campholenic aldehyde; (1R,4S,5S,9R)-1-hydroxy-1,4,7,7,9-pentamethyl spiro(4.5)decan-2-one; (3R)-(+)-3-methyl-1,4-dioxacyclohexadecan-2-one; (3R)-(+)-3-methyl-1,4-dioxacyclopentadecan-2-one; (3S)-(-)-3-methyl-1,4-dioxacyclohexadecan-2-one; (3S)-(-)-3-methyl-1,4-dioxacyclopentadecan-2-one; (4aR,5R,7aS,9R)-octahydro-2,2,5,8,8,9a-hexamethyl-4h-4a,9-methanoazuleno(5,6-d)-1,3-dioxole; (cis+trans)-1,1,2,3,3-pentamethyl-2,3,3a,4,5,9b-hexahydro-1H-7,9-diazacyclopenta[a]naphthalene; (cis+trans)-1,1,3,3-pentamethyl-2,3,3a,4,5,9b-hexahydro-1H-7,9-diazacyclopenta[a]naphthalene; (cis+trans)-6,6a,7,8,9,9a-hexahydro-7,7,8,9,9-pentamethyl-5H-cyclopenta[H]quinazoline; (E)-3-methyl-5-cyclotetradecen-1-one; (E)-woody epoxide; (S)-dihydro-beta-ionol; (Z)-abienol; 2,6,10,10-tetramethyl-1-oxa-spiro[4.5]dec-3-en-6-yl acetate; 2-acetyl-4-isopropenyl pyridine; 2-methyl undecanal (aldehyde C-12 mna); 2-methyl undecanal dimethyl acetal; 2-methyl-4-(camphenyl-8) cyclohexanone; 3(or 2),4,5-trimethyl octahydro-4,7-methanoinden-5-yl acetate; 3a,3b,5,6,7,8a-hexahydro-2,2,3a,3b,7,7-hexamethyl-4H-indeno[1,2-D]-1,3-dioxole; 4-(3,3-dimethyl bicyclo(2.2.1)hept-2-yl)-1-methyl-2-oxabicyclo(2.2.2)octane; 4-(3,3-dimethyl bicyclo(2.2.1)hept-2-yl)-5-methyl-2-oxabicyclo(2.2.2)octane; 4-allyl-1,4-dimethyl bicyclo(3.2.1)octan-3-one; 4-isopropyl cinnamaldehyde; 5,5a,6,7,8,8a-hexahydro-6,6,7,8,8-pentamethyl-4H-indeno[5,4-D]isoxazole; 5,6,7,8-tetrahydro-6,6,7,8,8-pentamethyl-4H-indeno[5,4-D]isoxazole; 6,6,10,10-tetramethyl-5,7,8,9,10,10a-hexahydro-6H-6a,9-methanobenzo[H]quinazoline; 6,7,8,9-tetrahydro-7,7,8,9,9-pentamethyl-5H-cyclopenta[H]quinazoline; acetoxymethyl isolongifolene; acetyl cedrene; alpha-ambrinol; alpha-cedrene epoxide; alpha-ethyl-2,2,6-trimethyl cyclohexane propanol; alpha-selinene; amber acetate; amber butanol; amber carane; amber carbinol; amber cyclohexanol; amber decane; amber decatriene; amber dioxane; amber dioxepine; amber dodecane; amber formate; amber furan; amber naphthofuran; amber oil; amber oxepin; amber pentadecane; amber spirolene; ambergris naphthol; ambergris tincture; ambrain; ambrein; ambrene acetal; ambrette seed oil; ambrettolic acid; ambrettolide; ambrinol; ambroxan; ambroxide; amyl benzoate; amyl cinnamate; amyris acetate; angelica root oil; animal carbolactone; atlas cedarwood oil fractions; beta-caryophyllene alcohol acetate; beta-isomethyl ionone; black poplar bud oil; butyl benzoate; calarene epoxide; cedar cyclododecatriene; cedarwood acetate; cedarwood oil texas fractions; cedrol methyl ether; cedryl formate; cedryl methyl ether; cistus ladaniferus gum; cistus twig/leaf concrete; cistus twig/leaf oil; cistus twig/leaf oil molecular distilled; clearwood ; cyclohexanecarboxylic acid, 2,2,6-trimethyl-, ethyl ester, (1R,6S)-; dewy propionate; dihydro-beta-ionol; dihydro-beta-ionone; dihydro-gamma-ionone; elecampane root oil; eriocephalus punctulatus flower oil; formoxymethyl isolongifolene; georgywood; geranyl benzoate; geranyl cinnamate; homoambrinol; hydroxyambran; hydroxymethyl isolongifolene; isoamyl cinnamate; isobutyl phenyl acetate; juniper berry concrete; juniper lactone; labdanum concrete; labdanum gum extract ethyl ester; labdanum oil; linalyl cinnamate; mcp acetate; melozol acetate; methyl 2,6,10-trimethyl cyclododeca-2,5,9-trienyl ketone; methyl cedryl ketone; methyl hydrogenated rosinate; methyl methylene tricyclodecanol acetate; muscomer; musk amberol; musk dimethyl indane; musk indane; musk lactone; musk methyl ketone; musk tetralin; myrrh oil; paramethyl tetrahydroquinoline; patchouli ethanone; peru balsam oil; piper longum fruit oil ; propyl cinnamate; romanal; rosa odorata flower oil; satinaldehyde; sclarene; sclareol; sclareolide; spruce needle oil canada; styrax gum (liquidambar styraciflua); symroxane (CAS: 676125-00-1 and 393517-29-8); tetradecanal; tetramethyl-4-methylene-2-heptanol; tetramethyl-5-oxatricyclododecane; timber propanol; tolu balsam; tricyclodecenyl isobutyrate; valeriana officinalis root extract; vetiver oil ; vetiver oil haiti; vetiveria zizanioides root oil; violet propanol; woody cyclohexanone; woody dioxolane; woody dodecane; woody epoxide; woody ether; woody furan; woody nonane (ethoxy) (equal to 4-ethoxy-4,8,8-trimethyl-9-methylidenebicyclo[3.3.1]nonane) and woody propanol.

17. Cosmetic product according to any of the proceeding claims **characterized in that** the composition comprises surfactants in a total quantity ranging from 0.5 to 15.0% by weight, more preferably from 6.0 to 13.0% by weight and most preferably from 9.5 to 12.0% by weight, calculated to the total weight of the composition.

18. Cosmetic product according to any of the proceeding claims **characterized in that** composition comprises Sodium Lauryl Sulfate, Sodium Laureth Sulfate and/or Sodium Myreth Sulfate as surfactant.
